# EUROPEAN PATENT SPECIFICATION

(11) **EP 4 093 738 B1**
(45) Date of publication and mention of the grant of the patent: **06.03.2024**
(21) Application number: 21705391.7
(22) Date of filing: 23.01.2021
(51) Int. Cl.: C07D 471/04, A01N 43/90, A01P 7/04

(54) **MESOIONIC INSECTICIDES**
MESOIONISCHE INSEKTIZIDE
INSECTICIDES MÉSOIONIQUES

(30) Priority: 24.01.2020 US 202062965183 P
(43) Date of publication of application: 30.11.2022
(73) Proprietor: FMC CORPORATION, Philadelphia, PA 19104 (US)
(72) Inventor: HOLMES, Michael, Philadelphia, PA 19104 (US); HOLYOKE, Caleb, William, Jr., Philadelphia, PA 19104 (US); KAR, Moumita, Philadelphia, PA 19104 (US); LAHM, George, Philip, Philadelphia, PA 19104 (US)
(74) Representative: Dehns
(86) International application number: PCT/US2021/014805
(87) International publication number: WO 2021/151034

(56) References cited:
- EP-A1- 3 287 457
- WO-A1-2009/099929
- WO-A2-2011/017342
- CN-A- 111 153 900
- CORDOVA DANIEL ET AL: "Mode of action of triflumezopyrim: A novel mesoionic insecticide which inhibits the nicotinic acetylcholine receptor", INSECTS BIOCHEMISTRY AND MOLECULAR BIOLOGY, ELSEVIER LTD, AMSTERDAM, NL, vol. 74, 26 April 2016 (2016-04-26), pages 32-41, XP029562636, ISSN: 0965-1748, DOI: 10.1016/J.IBMB.2016.04.008
- ZHANG WENMING ET AL: "Mesoionic pyrido[1,2-a]pyrimidinones: Discovery of dicloromezotiaz as a lepidoptera insecticide acting on nicotinic acetylcholine receptors1,2", BIORGANIC & MEDICINAL CHEMISTRY LETTERS, ELSEVIER, AMSTERDAM , NL, vol. 27, no. 4, 5 January 2017 (2017-01-05), pages 911-917, XP029906421, ISSN: 0960-894X, DOI: 10.1016/J.BMCL.2017.01.002
- WENMING ZHANG: "Mesoionic Pyrido[1,2- a ]pyrimidinone Insecticides: From Discovery to Triflumezopyrim and Dicloromezotiaz", ACCOUNTS OF CHEMICAL RESEARCH, vol. 50, no. 9, 21 August 2017 (2017-08-21), pages 2381-2388, XP055631174, US ISSN: 0001-4842, DOI: 10.1021/acs.accounts.7b00311

## Description

### CROSS-REFERENCE TO RELATED APPLICATIONS

This application claims the benefit of U.S. Provisional Application No. 62/965,183 filed January 24, 2020.

### FIELD

This disclosure relates to certain substituted mesoionic compounds, their N-oxides, salts and compositions suitable for agronomic and nonagronomic uses, and methods of their use for controlling invertebrate pests such as arthropods in both agronomic and nonagronomic environments.

### BACKGROUND

The control of invertebrate pests is extremely important in achieving high crop efficiency. Damage by invertebrate pests to growing and stored agronomic crops can cause significant reduction in productivity and thereby result in increased costs to the consumer. The control of invertebrate pests in forestry, greenhouse crops, ornamentals, nursery crops, stored food and fiber products, livestock, household, turf, wood products, and public and animal health is also important. Many products are commercially available for these purposes, but the need continues for new compounds that are more effective, less costly, less toxic, environmentally safer or have different sites of action.

PCT Patent Publications WO 2011/017342 and WO 2011/017334 disclose mesoionic compounds and their use as insecticides in agricultural environments.

### SUMMARY

The scope of the invention and thus of protection is defined by the appended claims. Subjectmatter of the present disclosure not encompassed by the claims is not to be understood as forming part of the invention. In particular, the invention shall not include any methods of therapeutic or prophylactic treatment of the human or animal body, even if described in the following disclosure.

This disclosure is directed to compounds of Formula 1 (including all stereoisomers), *N*-oxides, and salts thereof, compositions containing them and their use as insecticides: 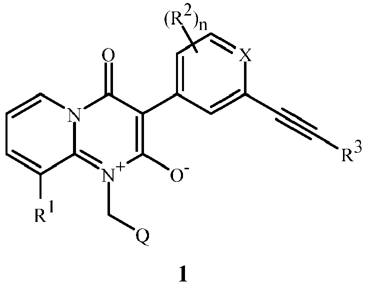 wherein
R¹ is H, halogen, C₁-C₄ alkyl, C₁-C₄ haloalkyl, C₁-C₄ alkoxy or C₁-C₄ haloalkoxy;
X is CR⁴ or N;
Q is CH₂CN, 6-chloro-3-pyridinyl or 2-chloro-5-thiazolyl;
each R² is independently halogen, cyano, nitro, C₁-C₄ alkyl, C₁-C₄ haloalkyl, C₃-C₆ cycloalkyl, C₁-C₆ alkoxy, C₁-C₆ haloalkoxy, C₃-C₆ cycloalkoxy, C₁-C₆ alkoxyalkoxy, C₁-C₆ alkylthio, C₁-C₆ alkylsulfinyl, C₁-C₆ alkylsulfonyl, C₁-C₆ haloalkylthio, C₁-C₆ haloalkylsulfinyl, or C₁-C₆ haloalkylsulfonyl;
n is 0, 1, 2 or 3;
R⁴ is H, halogen, cyano, nitro, C₁-C₄ alkyl, C₁-C₄ haloalkyl, C₃-C₆ cycloalkyl, C₁-C₆ alkoxy, C₁-C₆ haloalkoxy, C₁-C₆ alkylthio, C₁-C₆ alkylsulfinyl, C₁-C₆ alkylsulfonyl, C₁-C₆ haloalkylthio, C₁-C₆ haloalkylsulfinyl, or C₁-C₆ haloalkylsulfonyl; and
R³ is C₁-C₆ alkyl, C₁-C₆ haloalkyl, C₁-C₆ alkoxyalkyl, C₁-C₆ hydroxyalkyl, C₃-C₆ cycloalkyl, C₃-C₆ halocycloalkyl, C₄-C₇ alkylcycloalkyl or phenyl optionally substituted with halogen, cyano, nitro, C₁-C₄ alkyl, C₁-C₄ haloalkyl, C₃-C₆ cycloalkyl, C₁-C₆ alkoxy, C₁-C₆ haloalkoxy, C₁-C₆ alkylthio, C₁-C₆ alkylsulfinyl, C₁-C₆ alkylsulfonyl, C₁-C₆ haloalkylthio, C₁-C₆ haloalkylsulfinyl, or C₁-C₆ haloalkylsulfonyl;
provided that when Q is CH₂CN, then R³ is other than H and CH₃.

This disclosure also provides a composition comprising a compound of Formula **1,** an *N*-oxide or a salt thereof, and at least one additional component selected from the group consisting of surfactants, solid diluents and liquid diluents. In one embodiment, this disclosure also provides a composition for controlling an invertebrate pest comprising a compound of Formula **1,** an *N*-oxide or a salt thereof, and at least one additional component selected from the group consisting of surfactants, solid diluents and liquid diluents, said composition optionally further comprising at least one additional biologically active compound or agent (e.g., fungicide).

This disclosure further provides a spray composition for controlling an invertebrate pest comprising a compound of Formula **1,** an *N*-oxide or a salt thereof, or the compositions described above, and a propellant. This disclosure also provides a bait composition for controlling an invertebrate pest comprising a compound of Formula **1,** an *N*-oxide or a salt thereof, or the compositions described in the embodiments above, one or more food materials, optionally an attractant, and optionally a humectant.

This disclosure further provides a trap device for controlling an invertebrate pest comprising said bait composition and a housing adapted to receive said bait composition, wherein the housing has at least one opening sized to permit the invertebrate pest to pass through the opening so the invertebrate pest can gain access to said bait composition from a location outside the housing, and wherein the housing is further adapted to be placed in or near a locus of potential or known activity for the invertebrate pest.

This disclosure provides a method for controlling an invertebrate pest comprising contacting the invertebrate pest or its environment with a biologically effective amount of a compound of Formula **1,** an *N*-oxide or a salt thereof, (e.g., as a composition described herein). This disclosure also relates to such method wherein the invertebrate pest or its environment is contacted with a composition comprising a biologically effective amount of a compound of Formula **1,** an *N*-oxide or a salt thereof, and at least one additional component selected from the group consisting of surfactants, solid diluents and liquid diluents, said composition optionally further comprising a biologically effective amount of at least one additional biologically active compound or agent.

This disclosure also provides a method for protecting a seed from an invertebrate pest comprising contacting the seed with a biologically effective amount of a compound of Formula **1,** an *N*-oxide or a salt thereof, (e.g., as a composition described herein). This disclosure also relates to the treated seed. This disclosure further provides a method for protecting an animal from an invertebrate parasitic pest comprising administering to the animal a parasiticidally effective amount of a compound of Formula **1,** an *N*-oxide or a salt thereof, (e.g., as a composition described herein). This disclosure also provides for the use of a compound of Formula **1,** an *N*-oxide or a salt thereof, (e.g., as a composition described herein) in protecting an animal from an invertebrate pest.

This disclosure also provides a method for increasing vigor of a crop plant comprising contacting the crop plant, the seed from which the crop plant is grown or the locus (e.g., growth medium) of the crop plant with a biologically effective amount of a compound of Formula **1** (e.g., as a composition described herein).

In one embodiment, the compositions as disclosed herein further comprise liquid fertilizer. In some embodiments, the liquid fertilizer is aqueous-based.

In one embodiment, this disclosure provides a soil drench formulation comprising the compositions disclosed herein.

In one embodiment, this disclosure provides a spray composition comprising the compositions disclosed herein. In some embodiments the spray composition further comprises a propellant.

In one embodiment, this disclosure provides a bait composition comprising the composition disclosed herein. In one embodiment, the bait composition further comprises one or more food materials. In one embodiment, the bait composition further comprises an attractant. In one embodiment, the bait composition further comprises a humectant.

In one embodiment, the compositions disclosed herein are solid compositions, such as dusts, powders, granules, pellets, prills, pastilles, tablets, or filled films. In some embodiments, the compositions disclosed herein are solid compositions and are water-dispersible or water - soluble.

In one embodiment, a liquid or dry formulation comprising the compositions as disclosed herein for use in a drip irrigation system, furrow during planting, handheld sprayer, backpack sprayer, boom sprayer, ground sprayer, aerial application, unmanned aerial vehicle, or a seed treatment.

In one embodiment, the compositions as disclosed herein for use in a drip irrigation system, furrow during planting, handheld sprayer, backpack sprayer, boom sprayer, ground sprayer, aerial application, unmanned aerial vehicle, or a seed treatment wherein said formulation is sprayed at an ultra-low volume.

In one embodiment, this disclosure also relates to the treated seed.

### DETAILED DESCRIPTION

As used herein, the terms "comprises," "comprising," "includes," "including," "has," "having," "contains," "containing," "characterized by" or any other variation thereof, are intended to cover a non-exclusive inclusion, subject to any limitation explicitly indicated. For example, a composition, mixture, process, method, article, or apparatus that comprises a list of elements is not necessarily limited to only those elements but may include other elements not expressly listed or inherent to such composition, mixture, process, method, article, or apparatus.

The transitional phrase "consisting of" excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition, method or apparatus that includes materials, steps, features, components, or elements, in addition to those literally disclosed, provided that these additional materials, steps, features, components, or elements do not materially affect the basic and novel characteristic(s) of the claims. The term "consisting essentially of" occupies a middle ground between "comprising" and "consisting of".

Where applicants have defined an embodiment or a portion thereof with an open-ended term such as "comprising," it should be readily understood that (unless otherwise stated) the description should be interpreted to also describe such an embodiment using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of this disclosure are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As referred to in this disclosure, the term "invertebrate pest" includes arthropods, gastropods, nematodes and helminths of economic importance as pests. The term "arthropod" includes insects, mites, spiders, scorpions, centipedes, millipedes, pill bugs and symphylans. The term "gastropod" includes snails, slugs and other Stylommatophora. The term "nematode" includes members of the phylum Nematoda, such as phytophagous nematodes and helminth nematodes parasitizing animals. The term "helminth" includes all of the parasitic worms, such as roundworms (phylum Nematoda), heartworms (phylum Nematoda, class Secementea), flukes (phylum Platyhelminthes, class Tematoda), acanthocephalans (phylum Acanthocephala), and tapeworms (phylum Platyhelminthes, class Cestoda).

In the context of this disclosure "invertebrate pest control" means inhibition of invertebrate pest development (including mortality, feeding reduction, and/or mating disruption), and related expressions are defined analogously.

The term "agronomic" refers to the production of field crops such as for food and fiber and includes the growth of maize or corn, soybeans and other legumes, rice, cereal (e.g., wheat, oats, barley, rye and rice), leafy vegetables (e.g., lettuce, cabbage, and other cole crops), fruiting vegetables (e.g., tomatoes, pepper, eggplant, crucifers and cucurbits), potatoes, sweet potatoes, grapes, cotton, tree fruits (e.g., pome, stone and citrus), small fruit (e.g., berries and cherries) and other specialty crops (e.g., canola, sunflower and olives).

The term "nonagronomic" refers to other than field crops, such as horticultural crops (e.g., greenhouse, nursery or ornamental plants not grown in a field), residential, agricultural, commercial and industrial structures, turf (e.g., sod farm, pasture, golf course, lawn, sports field, etc.), wood products, stored product, agro-forestry and vegetation management, public health (i.e. human) and animal health (e.g., domesticated animals such as pets, livestock and poultry, undomesticated animals such as wildlife) applications.

The term "crop vigor" refers to rate of growth or biomass accumulation of a crop plant. An "increase in vigor" refers to an increase in growth or biomass accumulation in a crop plant relative to an untreated control crop plant. The term "crop yield" refers to the return on crop material, in terms of both quantity and quality, obtained after harvesting a crop plant. An "increase in crop yield" refers to an increase in crop yield relative to an untreated control crop plant.

The term "biologically effective amount" refers to the amount of a biologically active compound (e.g., a compound of Formula **1**) sufficient to produce the desired biological effect when applied to (i.e. contacted with) an invertebrate pest to be controlled or its environment, or to a plant, the seed from which the plant is grown, or the locus of the plant (e.g., growth medium) to protect the plant from injury by the invertebrate pest or for other desired effect (e.g., increasing plant vigor).

Generally when a molecular fragment (i.e. radical) is denoted by a series of atom symbols (e.g., C, H, N, O and S) the implicit point or points of attachment will be easily recognized by those skilled in the art. In some instances herein, particularly when alternative points of attachment are possible, the point or points of attachment may be explicitly indicated by a hyphen ("-"). For example, "-NCS" indicates that the point of attachment is the nitrogen atom (i.e. isothiocyanato, not thiocyanato).

As used herein, the term "alkylating agent" refers to a chemical compound in which a carbon-containing radical is bound through a carbon atom to a leaving group such as halide or sulfonate, which is displaceable by bonding of a nucleophile to said carbon atom. Unless otherwise indicated, the term "alkylating" does not limit the carbon-containing radical to alkyl; the carbon-containing radicals in alkylating agents include the variety of carbon-bound substituent radicals specified, for example, for R².

In the above recitations, the term "alkyl", used either alone or in compound words such as "alkylthio" or "haloalkyl" includes straight-chain or branched alkyl, such as, methyl, ethyl, n-propyl, i-propyl, or the different butyl, pentyl or hexyl isomers. "Alkoxy" includes, for example, methoxy, ethoxy, n-propyloxy, i-propyloxy, and the different butoxy, pentoxy and hexyloxy isomers. "Alkoxyalkyl" denotes alkoxy substitution on alkyl. Examples of "alkoxyalkyl" include CH₃OCH₂, CH₃OCH₂CH₂, CH₃CH₂OCH₂, CH₃CH₂CH₂OCH₂ and CH₃CH₂OCH₂CH₂. "Alkoxyalkoxy" denotes alkoxy substitution on another alkoxy moiety.

The term "alkylthio" includes straight-chain and branched alkylthio moieties such as methylthio, ethylthio, and the different propylthio, butylthio, pentylthio and hexylthio isomers. "Alkylsulfinyl" includes both enantiomers of an alkylsulfinyl group. Examples of "alkylsulfinyl" include CH₃S(=O), CH₃CH₂S(=O), CH₃CH₂CH₂S(=O), (CH₃)₂CHS(=O), and the different butylsulfinyl, pentylsulfinyl and hexylsulfinyl isomers. Examples of "alkylsulfonyl" include CH₃S(=O)₂, CH₃CH₂S(=O)₂, CH₃CH₂CH₂S(=O)₂, (CH₃)₂CHS(=O)₂, and the different butylsulfonyl, pentylsulfonyl and hexylsulfonyl isomers.

"Cycloalkyl" includes, for example, cyclopropyl, cyclobutyl, cyclopentyl and cyclohexyl. The term "alkylcycloalkyl" denotes alkyl substitution on a cycloalkyl moiety and includes, for example, ethylcyclopropyl, i-propylcyclobutyl, methylcyclopentyl and methylcyclohexyl. The term "cycloalkoxy" denotes cycloalkyl attached to and linked through an oxygen atom including, for example, cyclopentyloxy and cyclohexyloxy.

The term "halogen", either alone or in compound words such as "haloalkyl", or when used in descriptions such as "alkyl substituted with halogen" includes fluorine, chlorine, bromine or iodine. Further, when used in compound words such as "haloalkyl", or when used in descriptions such as "alkyl substituted with halogen" said alkyl may be partially or fully substituted with halogen atoms which may be the same or different. Examples of "haloalkyl" or "alkyl substituted with halogen" include F₃C-, C1CH₂-, CF₃CH₂- and CF₃CCl₂-. The terms "halocycloalkyl", "haloalkoxy", "haloalkylthio", "haloalkylsulfinyl", "haloalkylsulfonyl", and the like, are defined analogously to the term "haloalkyl". Examples of "haloalkoxy" include CF₃O-, CCl₃CH₂O-, HCF₂CH₂CH₂O- and CF₃CH₂O-. Examples of "haloalkylthio" include CCl₃S-, CF₃S-, CCl₃CH₂S- and ClCH₂CH₂CH₂S-.

"Hydroxyalkyl" denotes an alkyl group substituted with one hydroxy group. Examples of "hydroxyalkyl" include HOCH₂CH₂, CH₃CH₂(OH)CH and HOCH₂CH₂CH₂CH₂.

The total number of carbon atoms in a substituent group is indicated by the "Cᵢ-Cⱼ" prefix where i and j are numbers from 1 to 10. For example, C₁-C₆ alkylsulfonyl designates methylsulfonyl through hexylsulfonyl; C₂ alkoxyalkyl designates CH₃OCH₂-; C₃ alkoxyalkyl designates, for example, CH₃CH(OCH₃)-, CH₃OCH₂CH₂- or CH₃CH₂OCH₂-; and C₄ alkoxyalkyl designates the various isomers of an alkyl group substituted with an alkoxy group containing a total of four carbon atoms, examples including CH₃CH₂CH₂OCH₂- and CH₃CH₂OCH₂CH₂-.

The term "unsubstituted" in connection with a group such as a ring means the group does not have any substituents other than its one or more attachments to the remainder of Formula **1.** The term "optionally substituted" means that the number of substituents can be zero. Unless otherwise indicated, optionally substituted groups may be substituted with as many optional substituents as can be accommodated by replacing a hydrogen atom with a non-hydrogen substituent on any available carbon or nitrogen atom. Commonly, the number of optional substituents (when present) range from 1 to 3. As used herein, the term "optionally substituted" is used interchangeably with the phrase "substituted or unsubstituted" or with the term "(un)substituted."

When a compound is substituted with a substituent bearing a subscript that indicates the number of said substituents can vary (e.g., (R²)ₙ in Formula **1** wherein n is 1 to 3), then said substituents are independently selected from the group of defined substituents, unless otherwise indicated. Further, when the subscript indicates a range, e.g. (R)ᵢ₋ⱼ, then the number of substituents may be selected from the integers between i and j inclusive. When a group contains a substituent which can be hydrogen, for example R⁴, then when this substituent is taken as hydrogen, it is recognized that this is equivalent to said group being unsubstituted. When a variable group is shown to be optionally attached to a position, for example (R²)ₙ in Formula 1 wherein n may be 0, then hydrogen may be at the position even if not recited in the definition of the variable group. When one or more positions on a group are said to be "not substituted" or "unsubstituted", then hydrogen atoms are attached to take up any free valency.

Naming of substituents in the present disclosure uses recognized terminology providing conciseness in precisely conveying to those skilled in the art the chemical structure. For sake of conciseness, locant descriptors may be omitted. In some instances herein the point or points of attachment of substituents (e.g., R²) are indicated by locant numbers which may be different from the Chemical Abstracts naming system if the difference does not affect the meaning.

Unless otherwise indicated, a "ring" or "ring system" as a component of Formula **1** is carbocyclic or heterocyclic. The term "ring system" denotes two or more fused rings. The terms "bicyclic ring system" and "fused bicyclic ring system" denote a ring system consisting of two fused rings, which can be "ortho-fused", "bridged bicyclic" or "spirocyclic". An "ortho-fused bicyclic ring system" denotes a ring system wherein the two constituent rings have two adjacent atoms in common. A "bridged bicyclic ring system" is formed by bonding a segment of one or more atoms to nonadjacent ring members of a ring. A "spirocyclic ring system" is formed by bonding a segment of two or more atoms to the same ring member of a ring. The term "ring member" refers to an atom or other moiety (e.g., C(=O), C(=S), S(O) or S(O)₂) forming the backbone of a ring or ring system.

The terms "carbocyclic ring", "carbocycle" or "carbocyclic ring system" denote a ring or ring system wherein the atoms forming the ring backbone are selected only from carbon. The terms "heterocyclic ring", "heterocycle" or "heterocyclic ring system" denote a ring or ring system in which at least one atom forming the ring backbone is not carbon, e.g., nitrogen, oxygen or sulfur. Typically a heterocyclic ring contains no more than 4 nitrogen atoms, no more than 2 oxygens and no more than 2 sulfurs. Unless otherwise indicated, a carbocyclic ring or heterocyclic ring can be a saturated or unsaturated ring. "Saturated" refers to a ring having a backbone consisting of atoms linked to one another by single bonds; unless otherwise specified, the remaining atom valences are occupied by hydrogen atoms. Unless otherwise stated, an "unsaturated ring" may be partially unsaturated or fully unsaturated. The expression "fully unsaturated ring" means a ring of atoms in which the bonds between atoms in the ring are single or double bonds according to valence bond theory and furthermore the bonds between atoms in the ring include as many double bonds as possible without double bonds being cumulative (i.e. no C=C=C or C=C=N). The term "partially unsaturated ring" denotes a ring comprising at least one ring member bonded to an adjacent ring member through a double bond and which conceptually potentially accommodates a number of non-cumulated double bonds between adjacent ring members (i.e. in its fully unsaturated counterpart form) greater than the number of double bonds present (i.e. in its partially unsaturated form).

Unless otherwise indicated, heterocyclic rings and ring systems can be attached through any available carbon or nitrogen by replacement of a hydrogen on said carbon or nitrogen.

"Aromatic" indicates that each of the ring atoms is essentially in the same plane and has a p-orbital perpendicular to the ring plane, and in which (4n + 2) π electrons, where n is a positive integer, are associated with the ring to comply with Hückel's rule. The term "aromatic ring system" denotes a carbocyclic or heterocyclic ring system in which at least one ring of the ring system is aromatic. When a fully unsaturated carbocyclic ring satisfies Hückel's rule, then said ring is also called an "aromatic ring" or "aromatic carbocyclic ring".

The term "aromatic carbocyclic ring system" denotes a carbocyclic ring system in which at least one ring of the ring system is aromatic. When a fully unsaturated heterocyclic ring satisfies Hückel's rule, then said ring is also called a "heteroaromatic ring", "aromatic heterocyclic ring" or "heterocyclic aromatic ring". The term "aromatic heterocyclic ring system" denotes a heterocyclic ring system in which at least one ring of the ring system is aromatic. The term "nonaromatic ring system" denotes a carbocyclic or heterocyclic ring system that may be fully saturated, as well as partially or fully unsaturated, provided that none of the rings in the ring system are aromatic. The term "nonaromatic carbocyclic ring system" denotes a carbocyclic ring in which no ring in the ring system is aromatic. The term "nonaromatic heterocyclic ring system" denotes a heterocyclic ring system in which no ring in the ring system is aromatic.

The term "optionally substituted" in connection with the heterocyclic rings refers to groups which are unsubstituted or have at least one non-hydrogen substituent that does not extinguish the biological activity possessed by the unsubstituted analog. As used herein, the following definitions shall apply unless otherwise indicated. The term "optionally substituted" is used interchangeably with the phrase "substituted or unsubstituted" or with the term "(un)substituted." Unless otherwise indicated, an optionally substituted group may have a substituent at each substitutable position of the group, and each substitution is independent of the other.

As depicted in Formula **1,** each R² is bonded to a ring specified to be a 6-membered aromatic ring, wherein X is either CR⁴ or N. In one embodiment of this disclosure R² is bonded to a ring member distal relative to the ring member connecting the ring to the remainder of Formula **1.** As depicted in Exhibit 1, in the six-membered aromatic ring, a ring member distal relative to the ring member connecting the ring to the remainder of Formula **1** is linked through two ring bonds to the connecting ring member.

### Exhibit 1

### Optional R² distal substitution on Formula 1

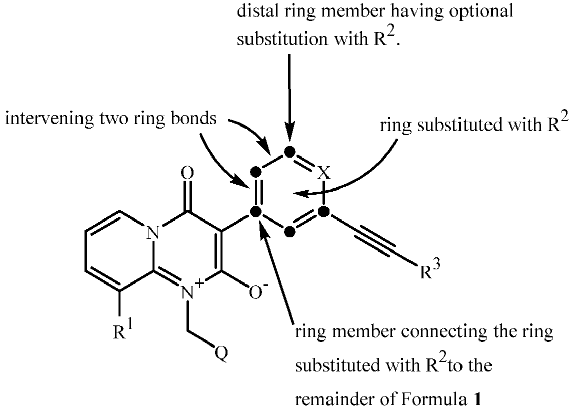

One skilled in the are art would recognize that in compounds of Formula 1 there is only one distal ring member available for substitution with R². The other distal ring member is bonded to the alkyne.

A wide variety of synthetic methods are known in the art to enable preparation of aromatic and nonaromatic heterocyclic rings and ring systems; for extensive reviews see the eight volume set of Comprehensive Heterocyclic Chemistry, A. R. Katritzky and C. W. Rees editors-in-chief, Pergamon Press, Oxford, 1984 and the twelve volume set of Comprehensive Heterocyclic Chemistry II, A. R. Katritzky, C. W. Rees and E. F. V. Scriven editors-in-chief, Pergamon Press, Oxford, 1996.

The compounds of Formula **1** are mesoionic inner salts. "Inner salts", also known in the art as "zwitterions", are electronically neutral molecules but carry formal positive and negative charges on different atoms in each valence bond structure according to valence bond theory. Furthermore the molecular structure of the compounds of Formula **1** can be represented by the six valence bond structures shown below, each placing the formal positive and negative charges on different atoms. Because of this resonance, the compounds of Formula **1** are also described as "mesoionic". Although for sake of simplicity, the molecular structure of Formula **1** is depicted as a single valence bond structure herein, this particular valence bond structure is to be understood as representative of all six valence bond structures relevant to bonding in molecules of compounds of Formula **1.** Therefore reference to Formula **1** herein relates to all six applicable valence bond structures as depicted in Exhibit 2 and other (e.g., molecular orbital theory) structures unless otherwise specified.

### Exhibit 2

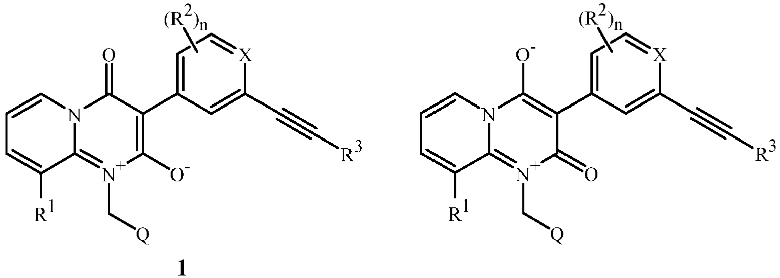

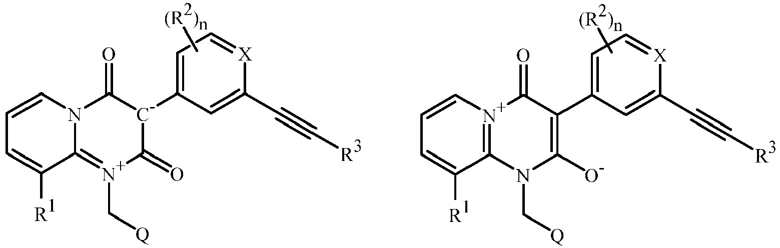

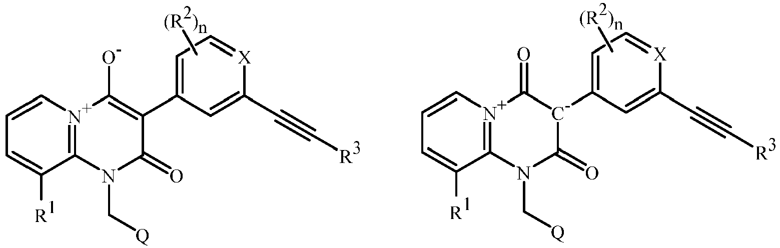

Compounds of this disclosure can exist as one or more stereoisomers. Stereoisomers are isomers of identical constitution but differing in the arrangement of their atoms in space and include enantiomers, diastereomers, *cis-* and trans-isomers (also known as geometric isomers) and atropisomers. Atropisomers result from restricted rotation about single bonds where the rotational barrier is high enough to permit isolation of the isomeric species. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. For a comprehensive discussion of all aspects of stereoisomerism, see Ernest L. Eliel and Samuel H. Wilen, Stereochemistry of Organic Compounds, John Wiley & Sons, 1994.

Compounds of this disclosure may be present as a mixture of stereoisomers, individual stereoisomers, or as an optically active form. Unless the structural formula or the language of this application specifically designate a particular *cis-* or *trans-isomer,* or a configuration of a chiral center, the scope of the present disclosure is intended to cover all such isomers per se, as well as mixtures of *cis-* and trans-isomers, mixtures of diastereomers and racemic mixtures of enantiomers (optical isomers) as well. Molecular depictions drawn herein follow standard conventions for depicting stereochemistry. To indicate stereoconfiguration, bonds rising from the plane of the drawing and towards the viewer are denoted by solid wedges wherein the broad end of the wedge is attached to the atom rising from the plane of the drawing towards the viewer. Bonds going below the plane of the drawing and away from the viewer are denoted by dashed wedges wherein the narrow end of the wedge is attached to the atom further away from the viewer. Constant width lines indicate bonds with a direction opposite or neutral relative to bonds shown with solid or dashed wedges; constant width lines also depict bonds in molecules or parts of molecules in which no particular stereoconfiguration is intended to be specified.

One skilled in the art will appreciate that not all nitrogen-containing heterocycles can form *N*-oxides since the nitrogen requires an available lone pair for oxidation to the oxide; one skilled in the art will recognize those nitrogen-containing heterocycles which can form N-oxides. One skilled in the art will also recognize that tertiary amines can form N-oxides. Synthetic methods for the preparation of N-oxides of heterocycles and tertiary amines are very well-known by one skilled in the art including the oxidation of heterocycles and tertiary amines with peroxy acids such as peracetic and 3-chloroperbenzoic acid (MCPBA), hydrogen peroxide, alkyl hydroperoxides such as t-butyl hydroperoxide, sodium perborate, and dioxiranes such as dimethyldioxirane. These methods for the preparation of N-oxides have been extensively described and reviewed in the literature, see for example: T. L. Gilchrist in Comprehensive Organic Synthesis, vol. 7, pp 748-750, S. V. Ley, Ed., Pergamon Press; M. Tisler and B. Stanovnik in Comprehensive Heterocyclic Chemistry, vol. 3, pp 18-20, A. J. Boulton and A. McKillop, Eds., Pergamon Press; M. R. Grimmett and B. R. T. Keene in Advances in Heterocyclic Chemistry, vol. 43, pp 149-161, A. R. Katritzky, Ed., Academic Press; M. Tisler and B. Stanovnik in Advances in Heterocyclic Chemistry, vol. 9, pp 285-291, A. R. Katritzky and A. J. Boulton, Eds., Academic Press; and G. W. H. Cheeseman and E. S. G. Werstiuk in Advances in Heterocyclic Chemistry, vol. 22, pp 390-392, A. R. Katritzky and A. J. Boulton, Eds., Academic Press.

One skilled in the art recognizes that because in the environment and under physiological conditions salts of chemical compounds are in equilibrium with their corresponding nonsalt forms, salts share the biological utility of the nonsalt forms. Thus a wide variety of salts of the compounds of Formula 1 are useful for control of invertebrate pests. The salts of the compounds of Formula 1 include acid-addition salts with inorganic or organic acids such as hydrobromic, hydrochloric, nitric, phosphoric, sulfuric, acetic, butyric, fumaric, lactic, maleic, malonic, oxalic, propionic, salicylic, tartaric, 4-toluenesulfonic or valeric acids. When a compound of Formula 1 contains an acidic moiety such as a carboxylic acid or phenol, salts also include those formed with organic or inorganic bases such as pyridine, triethylamine or ammonia, or amides, hydrides, hydroxides or carbonates of sodium, potassium, lithium, calcium, magnesium or barium. Accordingly, the present disclosure comprises compounds selected from Formula **1,** *N*-oxides and suitable salts thereof.

Compounds selected from Formula **1,** stereoisomers, tautomers, *N*-oxides, and salts thereof, typically exist in more than one form, and Formula **1** thus includes all crystalline and non-crystalline forms of the compounds that Formula **1** represents. Non-crystalline forms include embodiments which are solids such as waxes and gums as well as embodiments which are liquids such as solutions and melts. Crystalline forms include embodiments which represent essentially a single crystal type and embodiments which represent a mixture of polymorphs (i.e. different crystalline types). The term "polymorph" refers to a particular crystalline form of a chemical compound that can crystallize in different crystalline forms, these forms having different arrangements and/or conformations of the molecules in the crystal lattice. Although polymorphs can have the same chemical composition, they can also differ in composition due to the presence or absence of co-crystallized water or other molecules, which can be weakly or strongly bound in the lattice. Polymorphs can differ in such chemical, physical and biological properties as crystal shape, density, hardness, color, chemical stability, melting point, hygroscopicity, suspensibility, dissolution rate and biological availability. One skilled in the art will appreciate that a polymorph of a compound represented by Formula **1** can exhibit beneficial effects (e.g., suitability for preparation of useful formulations, improved biological performance) relative to another polymorph or a mixture of polymorphs of the same compound represented by Formula **1.** Preparation and isolation of a particular polymorph of a compound represented by Formula **1** can be achieved by methods known to those skilled in the art including, for example, crystallization using selected solvents and temperatures. For a comprehensive discussion of polymorphism see R. Hilfiker, Ed., Polymorphism in the Pharmaceutical Industry, Wiley-VCH, Weinheim, 2006.

Embodiments of the present disclosure as described in the Summary include those described below. In the following Embodiments, Formula **1** includes stereoisomers, N-oxides and salts thereof, and reference to "a compound of Formula **1"** includes the definitions of substituents specified in the Summary unless further defined in the Embodiments.
Embodiment 1. A compound of Formula **1** wherein R¹ is H, halogen, C₁-C₄ alkyl or C₁-C₄ alkoxy.
Embodiment 2. A compound of Embodiment 1 wherein R¹ is H, halogen, C₁-C₂ alkyl or C₁-C₂ alkoxy.
Embodiment 3. A compound of Embodiment 2 wherein R¹ is H, halogen, CH₃ or OCH₃.
Embodiment 4. A compound of Embodiment 3 wherein R¹ is H, Cl, CH₃ or OCH₃.
Embodiment 5. A compound of Embodiment 4 wherein R¹ is H.
Embodiment 6. A compound of Embodiment 4 wherein R¹ is Cl.
Embodiment 7. A compound of Embodiments 4 wherein R¹ is CH₃.
Embodiment 8. A compound of Formula **1** or any one of Embodiments 1 through 7 wherein X is N.
Embodiment 9. A compound of Formula **1** or any one of Embodiments 1 through 7 wherein X is CR⁴.
Embodiment 9A. A compound of Embodiment 9 wherein R⁴ is H, halogen, cyano, C₁-C₄ alkyl, C₁-C₄ haloalkyl, C₁-C₆ alkoxy or C₁-C₆ haloalkoxy.
Embodiment 9B. A compound of Embodiment 9A wherein R⁴ is H, halogen, C₁-C₄ alkyl or C₁-C₆ alkoxy.
Embodiment 9C. A compound of Embodiment 9B wherein R⁴ is H, halogen, C₁-C₂ alkyl or C₁-C₂ alkoxy.
Embodiment 9D. A compound of Embodiment 9C wherein R⁴ is H, halogen, CH₃ or OCH₃.
Embodiment 9E. A compound of Embodiment 9D wherein R⁴ is H, Cl, F, CH₃ or OCH₃.
Embodiment 9F. A compound of Embodiment 9E wherein R⁴ is H.
Embodiment 9G. A compound of Embodiment 9E wherein R⁴ is Cl.
Embodiment 9H. A compound of Embodiment 9E wherein R⁴ is F.
Embodiment 91. A compound of Embodiment 9E wherein R⁴ is CH₃ or OCH₃.
Embodiment 9J. A compound of Embodiment 9I wherein R⁴ is CH₃.
Embodiment 9K. A compound of Embodiment 9I wherein R⁴ is OCH₃.
Embodiment 10. A compound of Formula **1** or any one of Embodiments 1 through 9K wherein Q is 6-chloro-3-pyridinyl or 2-chloro-5-thiazolyl.
Embodiment 11. A compound of Embodiment 10 wherein Q is 6-chloro-3-pyridinyl.
Embodiment 12. A compound of Embodiment 10 wherein Q is 2-chloro-5-thiazolyl.
Embodiment 13. A compound of Formula **1** or any one of Embodiments 1 through 12 wherein R² is halogen, cyano, C₁-C₄ alkyl, C₁-C₄ haloalkyl, C₁-C₆ alkoxy or C₁-C₆ haloalkoxy.
Embodiment 14. A compound of Embodiment 13 wherein R² is halogen, C₁-C₄ alkyl, C₁-C₄ haloalkyl or C₁-C₆ alkoxy.
Embodiment 15. A compound of Embodiment 14 wherein R² is halogen, C₁-C₃ alkyl, C₁-C₂ haloalkyl or C₁-C₃ alkoxy.
Embodiment 16. A compound of Embodiment 15 wherein R² is halogen, CH₃,CH₂CH₃, CH(CH₃)₂, CF₃ or C₁-C₃ alkoxy.
Embodiment 16A. A compound of Embodiment 16 wherein R² is OCH₂CH₃, or OCH(CH₃)₂.
Embodiment 16B. A compound of Embodiment 16 wherein R² is OCH₂CH₃.
Embodiment 16C. A compound of Embodiment 16 wherein R² is OCH(CH₃)₂.
Embodiment 17. A compound of Embodiment 16 wherein R² is Cl, F, CH₃ or OCH₃.
Embodiment 18. A compound of Embodiment 17 wherein R² is Cl.
Embodiment 19. A compound of Embodiment 17 wherein R² is F.
Embodiment 20. A compound of Embodiment 17 wherein R² is CH₃ or OCH₃.
Embodiment 21. A compound of Embodiment 20 wherein R² is CH₃.
Embodiment 22. A compound of Embodiment 20 wherein R² is OCH₃.
Embodiment 23. A compound of Formula **1** or any one of Embodiments 1 through 22 wherein R² is bonded to a ring member distal relative to the ring member connecting the ring to the remainder of Formula **1.**
Embodiment 24. A compound of Formula **1** or any one of Embodiments 1 through 23 wherein n is 0, 1 or 2.
Embodiment 25. A compound of Embodiment 24 wherein n is 2.
Embodiment 26. A compound of Embodiment 24 wherein n is 0 or 1.
Embodiment 27. A compound of Embodiment 26 wherein n is 0.
Embodiment 28. A compound of Embodiment 26 wherein n is 1.
Embodiment 29. A compound of Formula **1** or any one of Embodiments 1 through 28 wherein R³ is C₁-C₆ alkyl, C₁-C₆ haloalkyl, C₃-C₆ cycloalkyl, C₃-C₆ halocycloalkyl or phenyl optionally substituted with halogen, cyano, C₁-C₄ alkyl, C₁-C₄ haloalkyl or C₁-C₆ alkoxy.
Embodiment 30. A compound of Embodiment 29 wherein R³ is phenyl optionally substituted with halogen, cyano, C₁-C₄ alkyl, C₁-C₄ haloalkyl or C₁-C₆ alkoxy.
Embodiment 31. A compound of Embodiment 29 wherein R³ is C₁-C₆ alkyl, C₁-C₆ haloalkyl, C₃-C₆ cycloalkyl or C₃-C₆ halocycloalkyl.
Embodiment 32. A compound of Embodiment 31 wherein R³ is C₁-C₆ alkyl or C₃-C₆ cycloalkyl.
Embodiment 33. A compound of Embodiments 32 wherein R³ is methyl, ethyl, isopropyl or cyclopropyl.
Embodiment 34. A compound of Embodiments 33 wherein R³ is methyl.
Embodiment 35. A compound of Embodiments 33 wherein R³ is ethyl.
Embodiment 36. A compound of Embodiments 33 wherein R³ is isopropyl.
Embodiment 37. A compound of Embodiments 33 wherein R³ is cyclopropyl.

Embodiments of this disclosure, including Embodiments 1-37 above as well as any other embodiments described herein, can be combined in any manner, and the descriptions of variables in the embodiments pertain not only to the compounds of Formula **1** but also to the starting compounds and intermediate compounds useful for preparing the compounds of Formula **1.** In addition, embodiments of this disclosure, including Embodiments 1-37 above as well as any other embodiments described herein, and any combination thereof, pertain to the compositions and methods of the present disclosure.

Combinations of Embodiments 1-37 are illustrated by:
Embodiment A. A compound of Formula **1** wherein
   R¹ is H, halogen, C₁-C₄ alkyl or C₁-C₄ alkoxy;
   Q is 6-chloro-3-pyridinyl or 2-chloro-5-thiazolyl;
   each R² is independently halogen, C₁-C₄ alkyl, C₁-C₄ haloalkyl or C₁-C₆ alkoxy;
   n is 0, 1 or 2;
   R⁴ is H, halogen, C₁-C₄ alkyl or C₁-C₆ alkoxy; and
   R³ is C₁-C₆ alkyl, C₁-C₆ haloalkyl, C₃-C₆ cycloalkyl, C₃-C₆ halocycloalkyl or phenyl optionally substituted with halogen, cyano, C₁-C₄ alkyl, C₁-C₄ haloalkyl or C₁-C₆ alkoxy.
Embodiment B. A compound of Embodiment A wherein
   R¹ is H, halogen, C₁-C₂ alkyl or C₁-C₂ alkoxy;
   each R² is independently halogen, C₁-C₂ alkyl, C₁-C₂ haloalkyl or C₁-C₃ alkoxy;
   R⁴ is H, halogen, C₁-C₂ alkyl or C₁-C₂ alkoxy; and
   R³ is C₁-C₆ alkyl, C₁-C₆ haloalkyl, C₃-C₆ cycloalkyl or C₃-C₆ halocycloalkyl.
Embodiment C. A compound of Embodiment B wherein
   X is CR⁴;
   Q is 2-chloro-5-thiazolyl; and
   R⁴ is H, Cl, F, CH₃ or OCH₃.
Embodiment D. A compound of Embodiment C wherein
   R¹ is H, Cl, CH₃ or OCH₃;
   R² is Cl, F, CH₃ or OCH₃;
   n is 0 or 1; and
   R³ is C₁-C₆ alkyl or C₃-C₆ cycloalkyl.
Embodiment E. A compound of Embodiment D wherein
   R¹ is CH₃;
   R² is CH₃ or OCH₃; and
   R³ is ethyl, isopropyl or cyclopropyl.

Specific embodiments include compounds of Formula **1** selected from the group consisting of:
1-[(2-chloro-5-thiazolyl)methyl]-3-[3-(2-cyclopropylethynyl)phenyl]-2-hydroxy-9-methyl-4-oxo-4*H*-pyrido[1,2-a]pyrimidinium inner salt (Compound 37);
1-[(2-chloro-5-thiazolyl)methyl]-2-hydroxy-9-methyl-3-[3-(3-methyl-1-butyn-1-yl)phenyl]-4-oxo-4*H*-pyrido[1,2-a]pyrimidinium inner salt (Compound 44);
1-[(2-chloro-5-thiazolyl)methyl]-3-[3-(2-cyclopropylethynyl)-5-methoxyphenyl]-2-hydroxy-9-methyl-4-oxo-4*H*-pyrido[1,2-a]pyrimidinium inner salt (Compound 4);
1-[(2-chloro-5-thiazolyl)methyl]-3-[3-(2-cyclopropylethynyl)-5-methylphenyl]-2-hydroxy-9-methyl-4-oxo-4*H*-pyrido[1,2-a]pyrimidinium inner salt (Compound 11);
1-[(2-chloro-5-thiazolyl)methyl]-2-hydroxy-9-methyl-3-[3-methyl-5-(3-methyl-1-butyn-1-yl)phenyl]-4-oxo-4*H*-pyrido[1,2-a]pyrimidinium inner salt (Compound 7);
3-[3-(1-butyn-1-yl)-5-chlorophenyl]-1-[(2-chloro-5-thiazolyl)methyl]-2-hydroxy-9-methyl-4-oxo-4*H*-pyrido[1,2-a]pyrimidinium inner salt (Compound 1);
1-[(2-chloro-5-thiazolyl)methyl]-3-[3-(2-cyclopropylethynyl)-5-ethoxyphenyl]-2-hydroxy-9-methyl-4-oxo-4*H*-pyrido[1,2-a]pyrimidinium inner salt (Compound 51);
3-[3-(1-butyn-1-yl)phenyl]-1-[(2-chloro-5-thiazolyl)methyl]-2-hydroxy-9-methyl-4-oxo-4*H*-pyrido[1,2-a]pyrimidinium inner salt (Compound 2); and
1-[(2-chloro-5-thiazolyl)methyl]-3-[3-ethoxy-5-(3-methyl-1-butyn-1-yl)phenyl]-2-hydroxy-9-methyl-4-oxo-4*H*-pyrido[1,2-a]pyrimidinium inner salt (Compound 54).

Of note is that compounds of this disclosure are characterized by favorable metabolic and/or soil residual patterns and exhibit activity controlling a spectrum of agronomic and nonagronomic invertebrate pests.

Of particular note, for reasons of invertebrate pest control spectrum and economic importance, protection of agronomic crops from damage or injury caused by invertebrate pests by controlling invertebrate pests are embodiments of this disclosure. Compounds of this disclosure because of their favorable translocation properties or systemicity in plants also protect foliar or other plant parts which are not directly contacted with a compound of Formula 1 or a composition comprising the compound.

Also noteworthy as embodiments of the present disclosure are compositions comprising a compound of any of the preceding Embodiments, as well as any other embodiments described herein, and any combinations thereof, and at least one additional component selected from the group consisting of a surfactant, a solid diluent and a liquid diluent, said compositions optionally further comprising at least one additional biologically active compound or agent.

Further noteworthy as embodiments of the present disclosure are compositions for controlling an invertebrate pest comprising a compound of any of the preceding Embodiments, as well as any other embodiments described herein, and any combinations thereof, and at least one additional component selected from the group consisting of a surfactant, a solid diluent and a liquid diluent, said compositions optionally further comprising at least one additional biologically active compound or agent. Embodiments of the disclosure further include methods for controlling an invertebrate pest comprising contacting the invertebrate pest or its environment with a biologically effective amount of a compound of any of the preceding Embodiments (e.g., as a composition described herein).

Embodiments of the disclosure also include a composition comprising a compound of any of the preceding Embodiments, in the form of a soil drench liquid formulation. Embodiments of the disclosure further include methods for controlling an invertebrate pest comprising contacting the soil with a liquid composition as a soil drench comprising a biologically effective amount of a compound of any of the preceding Embodiments.

Embodiments of the disclosure also include a spray composition for controlling an invertebrate pest comprising a biologically effective amount of a compound of any of the preceding Embodiments and a propellant. Embodiments of the disclosure further include a bait composition for controlling an invertebrate pest comprising a biologically effective amount of a compound of any of the preceding Embodiments, one or more food materials, optionally an attractant, and optionally a humectant. Embodiments of the disclosure also include a device for controlling an invertebrate pest comprising said bait composition and a housing adapted to receive said bait composition, wherein the housing has at least one opening sized to permit the invertebrate pest to pass through the opening so the invertebrate pest can gain access to said bait composition from a location outside the housing, and wherein the housing is further adapted to be placed in or near a locus of potential or known activity for the invertebrate pest.

Embodiments of the disclosure also include methods for protecting a seed from an invertebrate pest comprising contacting the seed with a biologically effective amount of a compound of any of the preceding Embodiments.

Embodiments of the disclosure also include methods for protecting an animal from an invertebrate parasitic pest comprising administering to the animal a parasiticidally effective amount of a compound of any of the preceding Embodiments.

Embodiments of the disclosure also include methods for controlling an invertebrate pest comprising contacting the invertebrate pest or its environment with a biologically effective amount of a compound of Formula **1,** an *N*-oxide or a salt thereof, (e.g., as a composition described herein), provided that the methods are not methods of medical treatment of a human or animal body by therapy.

This disclosure also relates to such methods wherein the invertebrate pest or its environment is contacted with a composition comprising a biologically effective amount of a compound of Formula **1,** an *N*-oxide or a salt thereof, and at least one additional component selected from the group consisting of surfactants, solid diluents and liquid diluents, said composition optionally further comprising a biologically effective amount of at least one additional biologically active compound or agent, provided that the methods are not methods of medical treatment of a human or animal body by therapy.

Embodiments of this disclosure also include use of an unmanned aerial vehicle (UAV) for the dispersion of the compositions disclosed herein over a planted area. In some embodiments the planted area is a crop-containing area. In some embodiments, the crop is selected from a monocot or dicot. In some embodiments, the crop is selected form rice, corn, barley, soybean, wheat, vegetable, tobacco, tea tree, fruit tree and sugar cane. In some embodiments, the compositions disclosed herein are formulated for spraying at an ultra-low volume. Products applied by drones may use water or oil as the spray carrier. Typical spray volume (including product) used for drone applications globally is 5.0 liters/ha - 100 liters/ha (approximately 0.5-10 gpa). This includes the range of ultra-low spray volume (ULV) to low spray volume (LV). Although not common there may be situations where even lower spray volumes could be used as low as 1.0 liter/ha (0.1 gpa).

One or more of the following methods and variations as described in Schemes 1-7 can be used to prepare the compounds of Formula **1.** The definitions of R¹, X, Q, R², n, R³ and R⁴ in the compounds of Formulae **1-10** below are as defined above in the Summary unless otherwise noted. Formula **3a** is a subset of Formula **3;** Formulae **4a** and **4b** are various subsets of the Formula **4,** and all substituents for each subset are as defined above for Formula **1** unless otherwise noted.

As shown in Scheme 1, compounds of Formula **1** can be prepared by the condensation of 2-aminopyridines of Formula **2** with activated malonic acids of Formula **3** in the presence of a base such as triethylamine, pyridine or *N*,*N*-diisopropylethylamine. The reaction is typically run in an organic solvent such as dichloromethane. The method of Scheme 1 is illustrated in present Example 1, Step D; and Example 2, Step C.

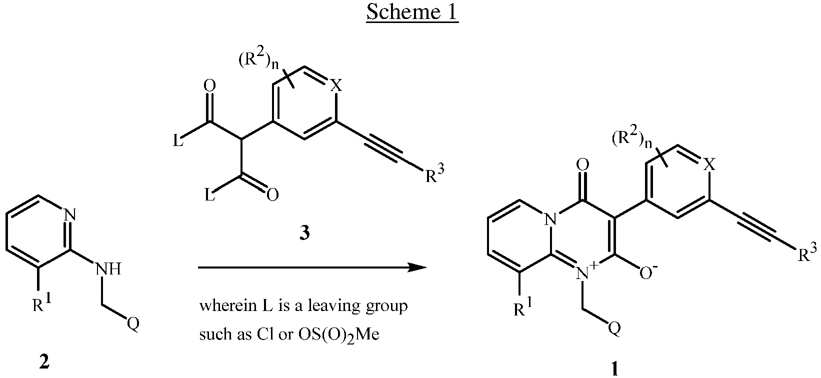

Alternatively, as shown in Scheme 2, compounds of Formula **1** can be prepared by the condensation of compounds of Formula **2** with compounds of Formula **4** or derivatives thereof, (e.g., Formula **4** wherein R^{a} is H or C₄-C₅ alkyl). Condensations with diester derivatives of Formula **4** (e.g., Formula **4** wherein R^{a} is C₁-C₅ alkyl) are typically run in high boiling solvents such as tetralin, xylene or *N*-methyl-2-pyrrolidinone at temperatures from room temperature to the boiling point of the solvent. For a representative procedure of the condensation of a diester, see WO 2009/099929. Other condensation procedures offer a number of alternatives for the condensation of diacid derivatives of Formula **4** (e.g., Formula **4** wherein R^{a} is H) in the presence of condensing agents such as dicyclohexyl carbodiimide (see, for example, Koch, A. et al. Tetrahedron 2004, 60, 10011-10018) or other agents well known in the art to form amide bonds with or without activating agents such as *N-*hydroxybenzotriazole as described in Science of Synthesis 2005, 21, 17-25 and Tetrahedron 2005, 61, 10827-10852.

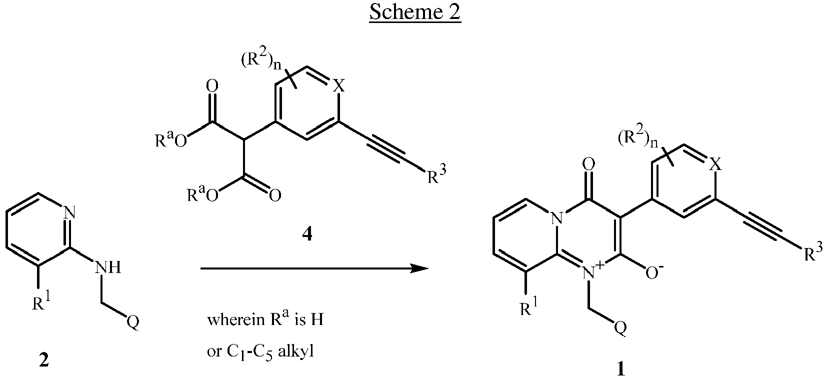

Diester compounds of Formula **4a** (i.e., Formula **4** wherein R^{a} is C₁-C₅ alkyl) can be prepared by reacting compounds of Formula **5** with alkynes of Formula **6** using Sonogashira coupling conditions as shown in Scheme 3. Sonogashira couplings are well known in the literature. See, for example, Molecules 2010, 15, 9157-9173; Sonogashira, K. In Handbook of Organopalladium Chemistry for Organic Synthesis; Negishi, E., Ed.; Wiley-Interscience: New York, 2002, pp 493-529; Palladium in Heterocyclic Chemistry, A guide for the Synthetic Chemist, Li, J.; Gribble, G., Eds. in Tetrahedron Organic Series, Volume 20; Pergamon Press: New York, 2000**.** The method of Scheme 3 is also illustrated in present Example 1, Step B.

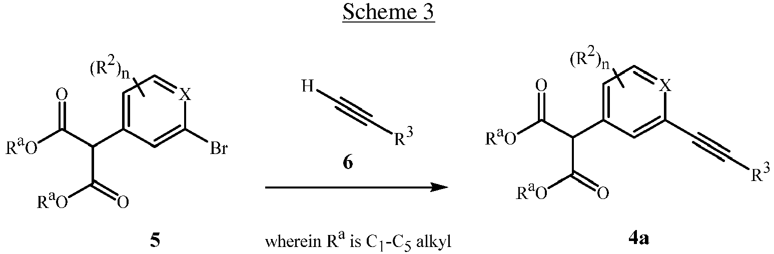

As shown in Scheme 4, compounds of Formula **5** can be prepared by the copper mediated coupling of aryl halides of Formula **7** with malonate esters of Formula **8** in the presence of a base such as cesium carbonate or potassium carbonate. For typical reactions conditions see, for example, Organic Letters 2009, 9(17), 3469; and Journal of the American Chemical Society 1980, 102(26), 7765-7774. The method of Scheme 4 is also illustrated in present Example 1, Step A.

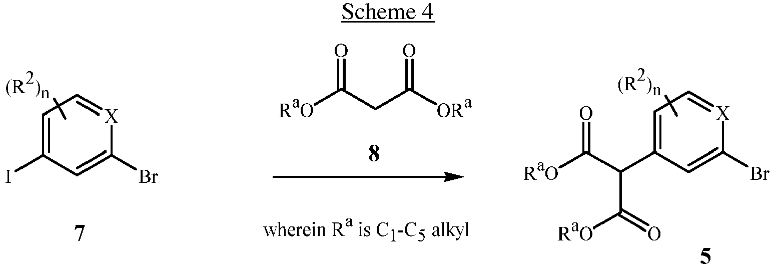

Alternatively, as shown in Scheme 5, compounds of Formula **4a** can be prepared by first reacting commercially available aryl dihalides of Formula **10** with alkynes of Formula **6** using Sonogashira coupling conditions similar to those in Scheme 3. The resulting compounds of Formula 9 can be coupled with malonate esters of Formula **8** using copper mediated conditions similar to those in Scheme 4.

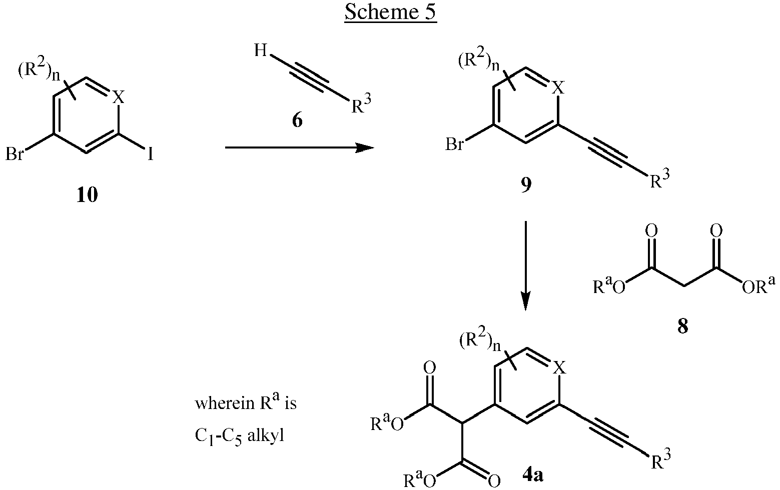

As shown in Scheme 6, compounds of Formula **4b** (i.e. Formula **4** wherein R^{a} is H) can be prepared from esters of Formula **4a** (i.e., Formula **4** wherein R^{a} is C₁-C₅ alkyl) using standard hydrolysis methods known to one skilled in the art. Hydrolysis reactions are typically run using aqueous solutions of inorganic bases such as sodium or potassium hydroxide followed by acidification with an acid such as hydrochloric acid. See, for example, Asian Journal of Chemistry, 23(3), 961; 2011. Also, present Example 1 Step C illustrates the method of Scheme 6.

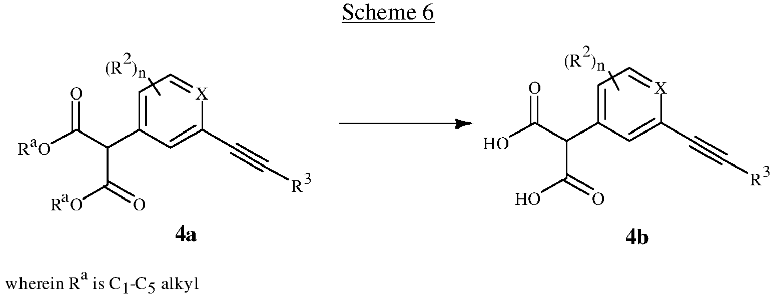

As shown in Scheme 7, acid chlorides of Formula **3a** (i.e. Formula **3** wherein L is Cl) can be prepared from acids of Formula **4b** (i.e. Formula **4** wherein R^{a} is H) in the presence of a chlorinating agent such as oxalyl chloride or thionyl chloride. Methods to prepare acid chlorides are known in the literature; for a general synthesis of compounds of Formula **3a,** see Advanced Organic Synthesis, 4th Edition, Wiley & Sons 1992, 437, and references cited therein. Also, present Example 1 Step D illustrates the method of Scheme 7.

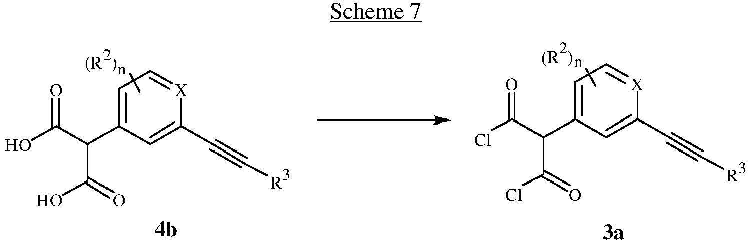

It is recognized by one skilled in the art that various functional groups can be converted into others to provide different compounds of Formula **1.** For example, compounds of Formula **1** wherein R² is halogen can be used to prepare compounds of Formula **1** wherein R² is cyano. Compounds of Formula **1,** or intermediates for their preparation, may contain aromatic nitro groups, which can be reduced to amino groups, and then converted via reactions well-known in the art (e.g., Sandmeyer reaction) to various halides. By similar known reactions, aromatic amines (anilines) can be converted via diazonium salts to phenols, which can then be alkylated to prepare compounds of Formula **1** with alkoxy substituents. Likewise, aromatic halides such as bromides or iodides prepared via the Sandmeyer reaction can react with alcohols under copper-catalyzed conditions, such as the Ullmann reaction or known modifications thereof, to provide compounds of Formula **1** that contain alkoxy substituents. Additionally, some halogen groups, such as fluorine or chlorine, can be displaced with alcohols under basic conditions to provide compounds of Formula **1** containing the corresponding alkoxy substituents. Compounds of Formula **1** or precursors thereof containing a halide, preferably bromide or iodide, are particularly useful intermediates for transition metal-catalyzed cross-coupling reactions to prepare compounds of Formula **1.** These types of reactions are well documented in the literature; see, for example, Tsuji in Transition Metal Reagents and Catalysts: Innovations in Organic Synthesis, John Wiley and Sons, Chichester, 2002; Tsuji in Palladium in Organic Synthesis, Springer, 2005; and Miyaura and Buchwald in Cross Coupling Reactions: A Practical Guide, 2002; and references cited therein.

It is recognized that some reagents and reaction conditions described above for preparing compounds of Formula **1** may not be compatible with certain functionalities present in the intermediates. In these instances, the incorporation of protection/deprotection sequences or functional group interconversions into the synthesis will aid in obtaining the desired products. The use and choice of the protecting groups will be apparent to one skilled in chemical synthesis (see, for example, Greene, T. W.; Wuts, P. G. M. Protective Groups in Organic Synthesis, 2nd ed.; Wiley: New York, 1991). One skilled in the art will recognize that, in some cases, after introduction of the reagents depicted in the individual schemes, additional routine synthetic steps not described in detail may be needed to complete the synthesis of compounds of Formula **1.** One skilled in the art will also recognize that it may be necessary to perform a combination of the steps illustrated in the above schemes in an order other than that implied by the particular sequence presented to prepare the compounds of Formula **1.**

One skilled in the art will also recognize that compounds of Formula **1** and the intermediates described herein can be subjected to various electrophilic, nucleophilic, radical, organometallic, oxidation, and reduction reactions to add substituents or modify existing substituents.

Without further elaboration, it is believed that one skilled in the art using the preceding description can utilize the present disclosure to its fullest extent. The following Synthesis Examples are, therefore, to be construed as merely illustrative, and not limiting of the disclosure in any way whatsoever. Steps in the following Synthesis Examples illustrate a procedure for each step in an overall synthetic transformation, and the starting material for each step may not have necessarily been prepared by a particular preparative run whose procedure is described in other Examples or Steps. Ambient or room temperature is defined as about 20-25 °C. Percentages are by weight except for chromatographic solvent mixtures or where otherwise indicated. Parts and percentages for chromatographic solvent mixtures are by volume unless otherwise indicated. ¹H NMR spectra are reported in ppm downfield from tetramethylsilane; "s" means singlet, "d" means doublet, "t" means triplet, "q" means quartet, "m" means multiplet and "dd" means doublet of doublets.

### EXAMPLE 1

### Preparation of 1-[(2-chloro-5-thiazolyl)methyl]-3-[3-(2-cyclopropylethynyl)phenyl]-2-hydroxy-9-methyl-4-oxo-4H-pyrido[1,2-a]pyrimidinium inner salt (Compound 37)

### Step A: Preparation of 1,3-diethyl 2-(3-bromophenyl)propanedioate

To a solution of 3-bromo-1-iodobenzene (50 g, 0.177 mol) in 1,4-dioxane (1000 mL) was added diethyl malonate (54 mL, 0.354 mol), cesium carbonate (143.8 g, 0.44 mol), cuprous iodide (3.3 g, 0.0177 mol), and 2-picolinic acid (4.3 g, 0.0354 mol). The reaction mixture was heated at 80 °C for 16 h. The reaction mixtue was cooled to room temperature, quenched with saturated ammonium chloride, extracted with ethyl acetate, dried over sodium sulfate, filtered and concentrated under reduced pressure. The resulting material was purified by silica gel column chromatography (eluting with a gradient of 0 to 15% methyl tert-butyl ether in petroleum ether) to provide the title compound as an oil (31 g).
¹H NMR (CDCl₃, 400 MHz) δ 7.59 (s, 1H), 7.49 (d, 1H), 7.37 (d, 1H), 7.25 (t, 1H), 4.58 (s, 1H), 4.22 (q, 4H), 1.30 (t, 6H).
LCMS: m/z: 315.3 [M+H]⁺

### Step B: Preparation of 1,3-diethyl 2-[3-(2-cyclopropylethynyl)phenyl]propanedioate

To a solution of 1,3-diethyl 2-(3-bromophenyl)propanedioate (i.e. the product of Step A) (2 g, 6.67 mmol) in triethylamine (15 mL) was added ethynylcyclopropane (0.6 mL, 7 mmol), cuprous iodide (121 mg, 0.667 mmol) and dichlorobis(triphenylphosphine)-palladium (II) (421 mg, 0.667 mmol). The reaction mixture was heated at 70 °C for 4 h in a sealed tube. The reaction mixture was cooled to room temperature and concentrated under reduced pressure. The resulting material was diluted with ethyl acetate, washed with water, followed by a saturated sodium chloride solution, dried over sodium sulfate, concentrated under reduced pressure and purified by silica gel column chromatography (eluting with a gradient of 0 to 15% methyl tert-butyl ether in petroleum ether) to provide the title compound as an oil (1.3 g).
¹H NMR (CDCl₃, 400 MHz) δ 7.59 (s, 1H), 7.3 (m, 3H), 4.57 (s, 1H), 4.20 (q, 4H), 1.45 (m, 1H), 1.30 (t, 6H), 0.8 (dd, 4H).
LCMS: m/z: 301.2 [M+H]⁺

### Step C: Preparation of 2-[3-(2-cyclopropylethynyl)phenyl]propanedioic acid

A solution of 1,3-diethyl 2-[3-(2-cyclopropylethynyl)phenyl]propanedioate (i.e. the product of Step B) (1.3 g, 4 mmol) in sodium hydroxide (2 M aqueous solution, 15 mL) was heated at 60 °C for 1 h. The reaction mixture was cooled to room temperature and acidified with hydrochloric acid (6 N aqueous solution) to adjust the pH to about 2 and extracted with diethyl ether. The organic extract was dried over sodium sulfate, filtered, concentrated under reduced pressure to provide the title compound, which was used directly in Step D.

### Step D: Preparation of 1-[(2-chloro-5-thiazolyl)methyl]-3-[3-(2-cyclopropylethynyl)-phenyl]-2-hydroxy-9-methyl-4-oxo-4H-pyrido[1,2-a]pyrimidinium inner salt

To a soulution of 2-[3-(2-cyclopropylethynyl)phenyl]propanedioic acid (i.e. the product of Step C) in dichloromethane (20 mL) at room temperature was added *N,N*-dimethylformamide (catalytic amount) followed by oxalyl chloride (0.6 mL, 6 mmol), stirred for 1 h, concentrated under reduced pressure to provide the intermediate compound 2-[3-(2-cyclopropylethynyl)phenyl]propanedioyl dichloride.

To a solution of *N*-[(2-chloro-5-thiazolyl)methyl]-3-methyl-2-pyridinamine (1.1 g, 4 mmol) and triethylamine (0.8 mL, 5 mmol) in dichloromethane (20 mL) at room temperature was added dropwise a solution of 2-[3-(2-cyclopropylethynyl)phenyl]propanedioyl dichloride in dichloromethane (20 mL), stirred for 30 minutes, filtered through Celite^{®} diatomaceous earth filter aid and concentrated under reduced pressure. The resulting material was purified by silica gel column chromatography (eluting with 50% ethyl acetate in petroleum ether) to provide the title compound, a compound of the present disclosure, as a solid (550 mg).
¹H NMR (CDCl₃, 400 MHz) δ 9.28 (d, 1H), 8.25 (d, 1H), 7.72 (m, 3H), 7.55 (d, 1H), 7.28 (t, 1H), 7.16 (d, 1H), 5.41 (s, 2H), 2.68 (s, 3H), 1.54 (m, 1H), 0.88 (dd, 4H).
LCMS: m/z: 448.4 [M+H]⁺

### EXAMPLE 2

### Preparation of 1-[(2-chloro-5-thiazolyl)methyl]-2-hydroxy-9-methyl-3-[3-(3-methyl-1-butyn-1-yl)phenyl]-4-oxo-4H-pyrido[1,2-a]pyrimidinium inner salt (Compound 44)

### Step A: Preparation of 1,3-diethyl 2-[3-(3-methyl-1-butyn-1-yl)phenyl]propanedioate

To a solution of 1,3-diethyl 2-(3-bromophenyl)propanedioate (i.e. the product of Example 1 Step A) (5 g, 15.9 mmol) in triethylamine (30 mL) was added 3-methyl-1-butyne (1.5 mL, 16.5 mmol), cuprous iodide (285 mg, 1.5 mmol) and dichlorobis(triphenylphosphine)palladium (II) (1.05 g, 1.5 mmol). The reaction mixture was heated at 70 °C for 4 h in a sealed tube. The reaction mixture was cooled to room temperature and concentrated under reduced pressure. The resulting material was diluted with ethyl acetate, washed with water, followed by a saturated sodium chloride solution, dried over sodium sulfate, concentrated under reduced pressure and purified by silica gel column chromatography (eluting with a gradient of 0 to 15% methyl tert-butyl ether in petroleum ether) to provide the title compound as an oil (2.5 g).
LCMS: m/z: 303.4 [M+H]⁺

### Step B: Preparation of 2-[3-(3-methyl-1-butyn-1-yl)phenyl]propanedioic acid

A solution of 1,3-diethyl 2-[3-(3-methyl-1-butyn-1-yl)phenyl]propanedioate (i.e. the product of Step A) (2.5 g, 8.27 mmol) in sodium hydroxide (2 M aqueous solution, 20 mL) was heated at 60 °C for 1 h. The reaction mixture was cooled to room temperature and acidified with hydrochloric acid (6 N aqueous solution) to adjust the pH to about 2 and extracted with diethyl ether. The organic extract was dried over sodium sulfate, filtered, concentrated under reduced pressure to provide the title compound, which was used directly in Step C.

### Step C: Preparation of 1-[(2-chloro-5-thiazolyl)methyl]-2-hydroxy-9-methyl-3-[3-(3-methyl-1-butyn-1-yl)phenyl]-4-oxo-4H-pyrido[1,2-a]pyrimidinium inner salt

To a soulution of 2-[3-(3-methyl-1-butyn-1-yl)phenyl]propanedioic acid (i.e. the product of Step B) in dichloromethane (25 mL) at room temperature was added *N*,*N*-dimethylformamide (catalytic amount) followed by oxalyl chloride (1.04 ml, 12 mmol), stirred for 1 h, concentrated under reduced pressure to provide the intermediate compound 2-[3-(3-methyl-1-butyn-1-yl)phenyl]propanedioyl dichloride.

To a solution of N-[(2-chloro-5-thiazolyl)methyl]-3-methyl-2-pyridinamine (2.17 g, 9 mmol) and triethylamine (1.3 ml, 9 mmol) in dichloromethane (25 mL) at room temperature was added dropwise a solution of 2-[3-(3-methyl-1-butyn-1-yl)phenyl]propanedioyl dichloride in dichloromethane (25 mL), stirred for 30 minutes, filtered through Celite^{®} diatomaceous earth filter aid and concentrated under reduced pressure. The resulting material was purified by silica gel column chromatography (eluting with 50% ethyl acetate in petroleum ether) to provide the title compound, a compound of the present disclosure, as a solid (0.55 g).
¹H NMR (CDCl₃, 400 MHz) δ 9.51 (d, 1H), 7.98 (d, 2H), 7.81 (s, 1H), 7.67 (t, 1H), 7.46 (s, 1H), 7.39 (d, 2H), 5.6 (s, 2H), 2.76 (m, 1H), 2.81 (s, 3H), 1.26 (d, 6H).
LCMS: m/z: 450 [M+H]⁺

By the procedures described herein together with methods known in the art, the following compounds of Tables 1 to 48 can be prepared. The following abbreviations are used in the Tables which follow: *i*-Pr means isopropyl, *c-*Pr cyclopropyl, *n*-Bu means *n*-butyl, *s-*Bu means sec-butyl, *t*-Bu means tertiary butyl, *c*-Bu means cyclobutyl, Ph means phenyl and CN means cyano.

**Table 1**

| 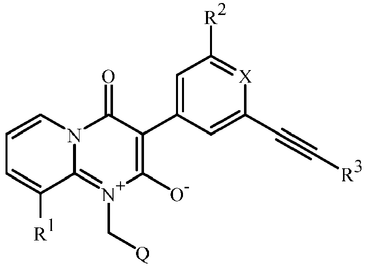 R¹ is CH₃, R² is H, X is C-H and Q is 2-Cl-5-thiazolyl. | | |
|---|---|---|
| R³ | R³ | R³ |
| CH₃ | CH₂CH₃ | *c*-Pr |
| *i*-Pr | *n*-Bu | *s*-Bu |
| *t*-Bu | *c*-Bu | Ph |
| 2-F-Ph | 3-F-Ph | 4-F-Ph |
| 2-Cl-Ph | 3-Cl-Ph | 4-Cl-Ph |

The present disclosure also includes Tables 2 through 48, each of which is constructed the same as Table 1 above, except that the row heading in Table 1 (i.e. "R¹ is CH₃, R² is H, X is C-H and Q is 2-Cl-5-thiazolyl") is replaced with the respective row headings shown below.

| Table | Row Heading |
|---|---|
| 2 | R¹ is CH₃, R² is F, X is C-H and Q is 2-Cl-5-thiazolyl. |
| 3 | R¹ is CH₃, R² is Cl, X is C-H and Q is 2-Cl-5-thiazolyl. |
| 4 | R¹ is CH₃, R² is CH₃, X is C-H and Q is 2-Cl-5-thiazolyl. |
| 5 | R¹ is CH₃, R² is OCH₃, X is C-H and Q is 2-Cl-5-thiazolyl. |
| 6 | R¹ is Cl, R² is H, X is C-H and Q is 2-Cl-5-thiazolyl. |
| 7 | R¹ is CH₃, R² is H, X is C-F and Q is 2-Cl-5-thiazolyl. |
| 8 | R¹ is CH₃, R² is H, X is C-Cl and Q is 2-Cl-5-thiazolyl. |
| 9 | R¹ is CH₃, R² is H, X is C-CH₃ and Q is 2-Cl-5-thiazolyl. |
| 10 | R¹ is CH₃, R² is H, X is C-OCH₃ and Q is 2-Cl-5-thiazolyl. |
| 11 | R¹ is CH₃, R² is H, X is N and Q is 2-Cl-5-thiazolyl. |
| 12 | R¹ is CH₃, R² is F, X is N and Q is 2-Cl-5-thiazolyl. |
| 13 | R¹ is CH₃, R² is Cl, X is N and Q is 2-Cl-5-thiazolyl. |
| 14 | R¹ is CH₃, R² is CH₃, X is N and Q is 2-Cl-5-thiazolyl. |
| 15 | R¹ is CH₃, R² is OCH₃, X is N and Q is 2-Cl-5-thiazolyl. |
| 16 | R¹ is Cl, R² is H, X is N and Q is 2-Cl-5-thiazolyl. |
| 17 | R¹ is CH₃, R² is H, X is C-H and Q is 6-Cl-3-pyridinyl. |
| 18 | R¹ is CH₃, R² is F, X is C-H and Q is 6-Cl-3-pyridinyl. |
| 19 | R¹ is CH₃, R² is Cl, X is C-H and Q is 6-Cl-3-pyridinyl. |
| 20 | R¹ is CH₃, R² is CH₃, X is C-H and Q is 6-Cl-3-pyridinyl. |
| 21 | R¹ is CH₃, R² is OCH₃, X is C-H and Q is 6-Cl-3-pyridinyl. |
| 22 | R¹ is Cl, R² is H, X is C-H and Q is 6-Cl-3-pyridinyl. |
| 23 | R¹ is CH₃, R² is H, X is C-F and Q is 6-Cl-3-pyridinyl. |
| 24 | R¹ is CH₃, R² is H, X is C-Cl and Q is 6-Cl-3-pyridinyl. |
| 25 | R¹ is CH₃, R² is H, X is C-CH₃ and Q is 6-Cl-3-pyridinyl. |
| 26 | R¹ is CH₃, R² is H, X is C- OCH₃ and Q is 6-Cl-3-pyridinyl. |
| 27 | R¹ is CH₃, R² is H, X is N and Q is 6-Cl-3-pyridinyl. |
| 28 | R¹ is CH₃, R² is F, X is N and Q is 6-Cl-3-pyridinyl. |
| 29 | R¹ is CH₃, R² is Cl, X is N and Q is 6-Cl-3-pyridinyl. |
| 30 | R¹ is CH₃, R² is CH₃, X is N and Q is 6-Cl-3-pyridinyl. |
| 31 | R¹ is CH₃, R² is OCH₃, X is N and Q is 6-Cl-3-pyridinyl. |
| 32 | R¹ is Cl, R² is H, X is N and Q is 6-Cl-3-pyridinyl. |
| 33 | R¹ is CH₃, R² is H, X is C-H and Q is CH₂CN. |
| 34 | R¹ is CH₃, R² is F, X is C-H and Q is CH₂CN. |
| 35 | R¹ is CH₃, R² is Cl, X is C-H and Q is CH₂CN. |
| 36 | R¹ is CH₃, R² is CH₃, X is C-H and Q is CH₂CN. |
| 37 | R¹ is CH₃, R² is OCH₃, X is C-H and Q is CH₂CN. |
| 38 | R¹ is Cl, R² is H, X is C-H and Q is CH₂CN. |
| 39 | R¹ is CH₃, R² is H, X is C-F and Q is CH₂CN. |
| 40 | R¹ is CH₃, R² is H, X is C-Cl and Q is CH₂CN. |
| 41 | R¹ is CH₃, R² is H, X is C-CH₃ and Q is CH₂CN. |
| 42 | R¹ is CH₃, R² is H, X is C-OCH₃ and Q is CH₂CN. |
| 43 | R¹ is CH₃, R² is H, X is N and Q is CH₂CN. |
| 44 | R¹ is CH₃, R² is F, X is N and Q is CH₂CN. |
| 45 | R¹ is CH₃, R² is Cl, X is N and Q is CH₂CN. |
| 46 | R¹ is CH₃, R² is CH₃, X is N and Q is CH₂CN. |
| 47 | R¹ is CH₃, R² is OCH₃, X is N and Q is CH₂CN. |
| 48 | R¹ is Cl, R² is H, X is N and Q is CH₂CN. |

### Formulation/Utility

A compound of this disclosure will generally be used as an invertebrate pest control active ingredient in a composition, i.e. formulation, with at least one additional component selected from the group consisting of surfactants, solid diluents and liquid diluents, which serves as a carrier. The formulation or composition ingredients are selected to be consistent with the physical properties of the active ingredient, mode of application and environmental factors such as soil type, moisture and temperature.

Useful formulations include both liquid and solid compositions. Liquid compositions include solutions (including emulsifiable concentrates), suspensions, emulsions (including microemulsions, oil in water emulsions, flowable concentrates and/or suspoemulsions) and the like, which optionally can be thickened into gels. The general types of aqueous liquid compositions are soluble concentrate, suspension concentrate, capsule suspension, concentrated emulsion, microemulsion, oil in water emulsion, flowable concentrate and suspoemulsion. The general types of nonaqueous liquid compositions are emulsifiable concentrate, microemulsifiable concentrate, dispersible concentrate and oil dispersion.

The general types of solid compositions are dusts, powders, granules, pellets, prills, pastilles, tablets, filled films (including seed coatings) and the like, which can be water-dispersible ("wettable") or water-soluble. Films and coatings formed from filmforming solutions or flowable suspensions are particularly useful for seed treatment. Active ingredient can be (micro)encapsulated and further formed into a suspension or solid formulation; alternatively the entire formulation of active ingredient can be encapsulated (or "overcoated"). Encapsulation can control or delay release of the active ingredient. An emulsifiable granule combines the advantages of both an emulsifiable concentrate formulation and a dry granular formulation. High-strength compositions are primarily used as intermediates for further formulation.

Sprayable formulations are typically extended in a suitable medium before spraying. Such liquid and solid formulations are formulated to be readily diluted in the spray medium, usually water, but occasionally another suitable medium like an aromatic or paraffinic hydrocarbon or vegetable oil. Spray volumes can range from about one to several thousand liters per hectare, but more typically are in the range from about ten to several hundred liters per hectare. Sprayable formulations can be tank mixed with water or another suitable medium for foliar treatment by aerial or ground application, or for application to the growing medium of the plant. Liquid and dry formulations can be metered directly into drip irrigation systems or metered into the furrow during planting. Liquid and solid formulations can be applied onto seeds of crops and other desirable vegetation as seed treatments before planting to protect developing roots and other subterranean plant parts and/or foliage through systemic uptake.

One way of dispensing the compositions disclosed herein over a target area, such as, but not limited to a crop-containing field, is by using drones. Use of drones or unmanned aerial vehicles (UAVs) in agricultural applications, such as for treating fields with chemical products, is rapidly expanding. A container of chemical products is coupled to the UAV and a material dispensing system mounted to the UAV, and the UAV is piloted above the area to be treated while the chemical product is dispensed.

The formulations will typically contain effective amounts of active ingredient, diluent and surfactant within the following approximate ranges which add up to 100 percent by weight.

### Weight Percent

| | Active Ingredient | Diluent | Surfactant |
|---|---|---|---|
| Water-Dispersible and Water-soluble Granules, Tablets and Powders | 0.001-90 | 0-99.999 | 0-15 |
| Oil Dispersions, Suspensions, Emulsions, Solutions (including Emulsifiable Concentrates) | 1-50 | 40-99 | 0-50 |
| Dusts | 1-25 | 70-99 | 0-5 |
| Granules and Pellets | 0.001-99 | 5-99.999 | 0-15 |
| High Strength Compositions | 90-99 | 0-10 | 0-2 |

Solid diluents include, for example, clays such as bentonite, montmorillonite, attapulgite and kaolin, gypsum, cellulose, titanium dioxide, zinc oxide, starch, dextrin, sugars (e.g., lactose, sucrose), silica, talc, mica, diatomaceous earth, urea, calcium carbonate, sodium carbonate and bicarbonate, and sodium sulfate. Typical solid diluents are described in Watkins et al., Handbook of Insecticide Dust Diluents and Carriers, 2nd Ed., Dorland Books, Caldwell, New Jersey.

Liquid diluents include, for example, water, *N*,*N*-dimethylalkanamides (e.g., *N*,*N*-dimethylformamide), limonene, dimethyl sulfoxide, N-alkylpyrrolidones (e.g., *N*-methylpyrrolidinone), alkyl phosphates (e.g., triethylphosphate), ethylene glycol, triethylene glycol, propylene glycol, dipropylene glycol, polypropylene glycol, propylene carbonate, butylene carbonate, paraffins (e.g., white mineral oils, normal paraffins, isoparaffins), alkylbenzenes, alkylnaphthalenes, glycerine, glycerol triacetate, sorbitol, aromatic hydrocarbons, dearomatized aliphatics, alkylbenzenes, alkylnaphthalenes, ketones such as cyclohexanone, 2-heptanone, isophorone and 4-hydroxy-4-methyl-2-pentanone, acetates such as isoamyl acetate, hexyl acetate, heptyl acetate, octyl acetate, nonyl acetate, tridecyl acetate and isobornyl acetate, other esters such as alkylated lactate esters, dibasic esters alkyl and aryl benzoates, γ-butyrolactone, and alcohols, which can be linear, branched, saturated or unsaturated, such as methanol, ethanol, *n*-propanol, isopropyl alcohol, *n*-butanol, isobutyl alcohol, *n*-hexanol, 2-ethylhexanol, *n*-octanol, decanol, isodecyl alcohol, isooctadecanol, cetyl alcohol, lauryl alcohol, tridecyl alcohol, oleyl alcohol, cyclohexanol, tetrahydrofurfuryl alcohol, diacetone alcohol, cresol and benzyl alcohol. Liquid diluents also include glycerol esters of saturated and unsaturated fatty acids (typically C₆-C₂₂), such as plant seed and fruit oils (e.g., oils of olive, castor, linseed, sesame, corn (maize), peanut, sunflower, grapeseed, safflower, cottonseed, soybean, rapeseed, coconut and palm kernel), animal-sourced fats (e.g., beef tallow, pork tallow, lard, cod liver oil, fish oil), and mixtures thereof. Liquid diluents also include alkylated fatty acids (e.g., methylated, ethylated, butylated) wherein the fatty acids may be obtained by hydrolysis of glycerol esters from plant and animal sources, and can be purified by distillation. Typical liquid diluents are described in Marsden, Solvents Guide, 2nd Ed., Interscience, New York, 1950.

The solid and liquid compositions of the present disclosure often include one or more surfactants. When added to a liquid, surfactants (also known as "surface-active agents") generally modify, most often reduce, the surface tension of the liquid. Depending on the nature of the hydrophilic and lipophilic groups in a surfactant molecule, surfactants can be useful as wetting agents, dispersants, emulsifiers or defoaming agents.

Surfactants can be classified as nonionic, anionic or cationic. Nonionic surfactants useful for the present compositions include, but are not limited to: alcohol alkoxylates such as alcohol alkoxylates based on natural and synthetic alcohols (which may be branched or linear) and prepared from the alcohols and ethylene oxide, propylene oxide, butylene oxide or mixtures thereof; amine ethoxylates, alkanolamides and ethoxylated alkanolamides; alkoxylated triglycerides such as ethoxylated soybean, castor and rapeseed oils; alkylphenol alkoxylates such as octylphenol ethoxylates, nonylphenol ethoxylates, dinonyl phenol ethoxylates and dodecyl phenol ethoxylates (prepared from the phenols and ethylene oxide, propylene oxide, butylene oxide or mixtures thereof); block polymers prepared from ethylene oxide or propylene oxide and reverse block polymers where the terminal blocks are prepared from propylene oxide; ethoxylated fatty acids; ethoxylated fatty esters and oils; ethoxylated methyl esters; ethoxylated tristyrylphenol (including those prepared from ethylene oxide, propylene oxide, butylene oxide or mixtures thereof); fatty acid esters, glycerol esters, lanolinbased derivatives, polyethoxylate esters such as polyethoxylated sorbitan fatty acid esters, polyethoxylated sorbitol fatty acid esters and polyethoxylated glycerol fatty acid esters; other sorbitan derivatives such as sorbitan esters; polymeric surfactants such as random copolymers, block copolymers, alkyd peg (polyethylene glycol) resins, graft or comb polymers and star polymers; polyethylene glycols (pegs); polyethylene glycol fatty acid esters; silicone-based surfactants; and sugar-derivatives such as sucrose esters, alkyl polyglycosides and alkyl polysaccharides.

Useful anionic surfactants include, but are not limited to: alkylaryl sulfonic acids and their salts; carboxylated alcohol or alkylphenol ethoxylates; diphenyl sulfonate derivatives; lignin and lignin derivatives such as lignosulfonates; maleic or succinic acids or their anhydrides; olefin sulfonates; phosphate esters such as phosphate esters of alcohol alkoxylates, phosphate esters of alkylphenol alkoxylates and phosphate esters of styryl phenol ethoxylates; protein-based surfactants; sarcosine derivatives; styryl phenol ether sulfate; sulfates and sulfonates of oils and fatty acids; sulfates and sulfonates of ethoxylated alkylphenols; sulfates of alcohols; sulfates of ethoxylated alcohols; sulfonates of amines and amides such as *N,N-*alkyltaurates; sulfonates of benzene, cumene, toluene, xylene, and dodecyl and tridecylbenzenes; sulfonates of condensed naphthalenes; sulfonates of naphthalene and alkyl naphthalene; sulfonates of fractionated petroleum; sulfosuccinamates; and sulfosuccinates and their derivatives such as dialkyl sulfosuccinate salts.

Useful cationic surfactants include, but are not limited to: amides and ethoxylated amides; amines such as N-alkyl propanediamines, tripropylenetriamines and dipropylenetetramines, and ethoxylated amines, ethoxylated diamines and propoxylated amines (prepared from the amines and ethylene oxide, propylene oxide, butylene oxide or mixtures thereof); amine salts such as amine acetates and diamine salts; quaternary ammonium salts such as quaternary salts, ethoxylated quaternary salts and diquaternary salts; and amine oxides such as alkyldimethylamine oxides and bis-(2-hydroxyethyl)-alkylamine oxides.

Also useful for the present compositions are mixtures of nonionic and anionic surfactants or mixtures of nonionic and cationic surfactants. Nonionic, anionic and cationic surfactants and their recommended uses are disclosed in a variety of published references including McCutcheon's Emulsifiers and Detergents, annual American and International Editions published by McCutcheon's Division, The Manufacturing Confectioner Publishing Co.; Sisely and Wood, Encyclopedia of Surface Active Agents, Chemical Publ. Co., Inc., New York, 1964; and A. S. Davidson and B. Milwidsky, Synthetic Detergents, Seventh Edition, John Wiley and Sons, New York, 1987.

Compositions of this disclosure may also contain formulation auxiliaries and additives, known to those skilled in the art as formulation aids (some of which may be considered to also function as solid diluents, liquid diluents or surfactants). Such formulation auxiliaries and additives may control: pH (buffers), foaming during processing (antifoams such polyorganosiloxanes), sedimentation of active ingredients (suspending agents), viscosity (thixotropic thickeners), in-container microbial growth (antimicrobials), product freezing (antifreezes), color (dyes/pigment dispersions), wash-off (film formers or stickers), evaporation (evaporation retardants), and other formulation attributes. Film formers include, for example, polyvinyl acetates, polyvinyl acetate copolymers, polyvinylpyrrolidone-vinyl acetate copolymer, polyvinyl alcohols, polyvinyl alcohol copolymers and waxes. Examples of formulation auxiliaries and additives include those listed in McCutcheon's Volume 2: Functional Materials, annual International and North American editions published by McCutcheon's Division, The Manufacturing Confectioner Publishing Co.; and PCT Publication WO 03/024222.

The compound of Formula 1 and any other active ingredients are typically incorporated into the present compositions by dissolving the active ingredient in a solvent or by grinding in a liquid or dry diluent. Solutions, including emulsifiable concentrates, can be prepared by simply mixing the ingredients. If the solvent of a liquid composition intended for use as an emulsifiable concentrate is water-immiscible, an emulsifier is typically added to emulsify the active-containing solvent upon dilution with water. Active ingredient slurries, with particle diameters of up to 2,000 µm can be wet milled using media mills to obtain particles with average diameters below 3 µm. Aqueous slurries can be made into finished suspension concentrates (see, for example, U.S. 3,060,084) or further processed by spray drying to form water-dispersible granules. Dry formulations usually require dry milling processes, which produce average particle diameters in the 2 to 10 µm range. Dusts and powders can be prepared by blending and usually grinding (such as with a hammer mill or fluid-energy mill). Granules and pellets can be prepared by spraying the active material upon preformed granular carriers or by agglomeration techniques. See Browning, "Agglomeration", Chemical Engineering, December 4, 1967, pp 147-48, Perry's Chemical Engineer's Handbook, 4th Ed., McGraw-Hill, New York, 1963, pages 8-57 and following, and WO 91/13546. Pellets can be prepared as described in U.S. 4,172,714. Water-dispersible and water-soluble granules can be prepared as taught in U.S. 4,144,050, U.S. 3,920,442 and DE 3,246,493. Tablets can be prepared as taught in U.S. 5,180,587, U.S. 5,232,701 and U.S. 5,208,030. Films can be prepared as taught in GB 2,095,558 and U.S. 3,299,566.

For further information regarding the art of formulation, see T. S. Woods, "The Formulator's Toolbox - Product Forms for Modern Agriculture" in Pesticide Chemistry and Bioscience, The Food-Environment Challenge, T. Brooks and T. R. Roberts, Eds., Proceedings of the 9th International Congress on Pesticide Chemistry, The Royal Society of Chemistry, Cambridge, 1999, pp. 120-133. See also U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, Weed Control as a Science, John Wiley and Sons, Inc., New York, 1961, pp 81-96; Hance et al., Weed Control Handbook, 8th Ed., Blackwell Scientific Publications, Oxford, 1989; and Developments in formulation technology, PJB Publications, Richmond, UK, 2000.

In the following Examples, all formulations are prepared in conventional ways. Compound numbers refer to compounds in Index Table A. Without further elaboration, it is believed that one skilled in the art using the preceding description can utilize the present disclosure to its fullest extent. The following Examples are, therefore, to be construed as merely illustrative, and not limiting of the disclosure in any way whatsoever. Percentages are by weight except where otherwise indicated.

### Example A

### High Strength Concentrate

| | |
|---|---|
| Compound 1 | 98.5% |
| silica aerogel | 0.5% |
| synthetic amorphous fine silica | 1.0% |

### Example B

### Wettable Powder

| | |
|---|---|
| Compound 5 | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0% |

### Example C

### Granule

| | |
|---|---|
| Compound 8 | 10.0% |
| attapulgite granules (low volatile matter, 0.71/0.30 mm; U.S.S. No. 25-50 sieves) | 90.0% |

### Example D

### Extruded Pellet

| | |
|---|---|
| Compound 12 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0% |

### Example E

### Emulsifiable Concentrate

| | |
|---|---|
| Compound 18 | 10.0% |
| polyoxyethylene sorbitol hexoleate | 20.0% |
| C₆-C₁₀ fatty acid methyl ester | 70.0% |

### Example F

### Microemulsion

| | |
|---|---|
| Compound 23 | 5.0% |
| polyvinylpyrrolidone-vinyl acetate copolymer | 30.0% |
| alkylpolyglycoside | 30.0% |
| glyceryl monooleate | 15.0% |
| water | 20.0% |

### Example G

### Seed Treatment

| | |
|---|---|
| Compound 36 | 20.00% |
| polyvinylpyrrolidone-vinyl acetate copolymer | 5.00% |
| montan acid wax | 5.00% |
| calcium ligninsulfonate | 1.00% |
| polyoxyethylene/polyoxypropylene block copolymers | 1.00% |
| stearyl alcohol (POE 20) | 2.00% |
| polyorganosilane | 0.20% |
| colorant red dye | 0.05% |
| water | 65.75% |

### Example H

### Fertilizer Stick

| | |
|---|---|
| Compound 42 | 2.5% |
| pyrrolidone-styrene copolymer | 4.8% |
| tristyrylphenyl 16-ethoxylate | 2.3% |
| talc | 0.8% |
| corn starch | 5.0% |
| slow-release fertilizer | 36.0% |
| kaolin | 38.0% |
| water | 10.6% |

### Example I

### Suspension Concentrate

| | |
|---|---|
| compound 43 | 35% |
| butyl polyoxyethylene/polypropylene block copolymer | 4.0% |
| stearic acid/polyethylene glycol copolymer | 1.0% |
| styrene acrylic polymer | 1.0% |
| xanthan gum | 0.1% |
| propylene glycol | 5.0% |
| silicone based defoamer | 0.1% |
| 1,2-benzisothiazolin-3-one | 0.1% |
| water | 53.7% |

### Example J

### Emulsion in Water

| | |
|---|---|
| compound 45 | 10.0% |
| butyl polyoxyethylene/polypropylene block copolymer | 4.0% |
| stearic acid/polyethylene glycol copolymer | 1.0% |
| styrene acrylic polymer | 1.0% |
| xanthan gum | 0.1% |
| propylene glycol | 5.0% |
| silicone based defoamer | 0.1% |
| 1,2-benzisothiazolin-3-one | 0.1% |
| aromatic petroleum based hydrocarbon | 20.0 |
| water | 58.7% |

### Example K

### Oil Dispersion

| | |
|---|---|
| compound 50 | 25% |
| polyoxyethylene sorbitol hexaoleate | 15% |
| organically modified bentonite clay | 2.5% |
| fatty acid methyl ester | 57.5% |

### Example L

### Suspoemulsion

| | |
|---|---|
| compound 51 | 10.0% |
| imidacloprid | 5.0% |
| butyl polyoxyethylene/polypropylene block copolymer | 4.0% |
| stearic acid/polyethylene glycol copolymer | 1.0% |
| styrene acrylic polymer | 1.0% |
| xanthan gum | 0.1% |
| propylene glycol | 5.0% |
| silicone based defoamer | 0.1% |
| 1,2-benzisothiazolin-3-one | 0.1% |
| aromatic petroleum based hydrocarbon | 20.0% |
| water | 53.7% |

Compounds of this disclosure exhibit activity against a wide spectrum of invertebrate pests. These pests include invertebrates inhabiting a variety of environments such as, for example, plant foliage, roots, soil, harvested crops or other foodstuffs, building structures or animal integuments. These pests include, for example, invertebrates feeding on foliage (including leaves, stems, flowers and fruits), seeds, wood, textile fibers or animal blood or tissues, and thereby causing injury or damage to, for example, growing or stored agronomic crops, forests, greenhouse crops, ornamentals, nursery crops, stored foodstuffs or fiber products, or houses or other structures or their contents, or being harmful to animal health or public health. Those skilled in the art will appreciate that not all compounds are equally effective against all growth stages of all pests.

These present compounds and compositions are thus useful agronomically for protecting field crops from phytophagous invertebrate pests, and also nonagronomically for protecting other horticultural crops and plants from phytophagous invertebrate pests. This utility includes protecting crops and other plants (i.e. both agronomic and nonagronomic) that contain genetic material introduced by genetic engineering (i.e. transgenic) or modified by mutagenesis to provide advantageous traits. Examples of such traits include tolerance to herbicides, resistance to phytophagous pests (e.g., insects, mites, aphids, spiders, nematodes, snails, plant-pathogenic fungi, bacteria and viruses), improved plant growth, increased tolerance of adverse growing conditions such as high or low temperatures, low or high soil moisture, and high salinity, increased flowering or fruiting, greater harvest yields, more rapid maturation, higher quality and/or nutritional value of the harvested product, or improved storage or process properties of the harvested products. Transgenic plants can be modified to express multiple traits. Examples of plants containing traits provided by genetic engineering or mutagenesis include varieties of corn, cotton, soybean and potato expressing an insecticidal *Bacillus thuringiensis* toxin such as YIELD GARD^{®}, KNOCKOUT^{®}, STARLINK^{®}, BOLLGARD^{®}, NuCOTN^{®} and NEWLEAF^{®}, INVICTA RR2 PRO^{™}, and herbicide-tolerant varieties of corn, cotton, soybean and rapeseed such as ROUNDUP READY^{®}, LIBERTY LINK^{®}, IMI^{®}, STS^{®} and CLEARFIELD^{®}, as well as crops expressing *N*-acetyltransferase (GAT) to provide resistance to glyphosate herbicide, or crops containing the HRA gene providing resistance to herbicides inhibiting acetolactate synthase (ALS). The present compounds and compositions may interact in a greater-than-additive (i.e. enhanced) effect with traits introduced by genetic engineering or modified by mutagenesis, thus enhancing phenotypic expression or effectiveness of the traits or increasing the invertebrate pest control effectiveness of the present compounds and compositions. In particular, the present compounds and compositions may interact in a greater-than-additive (i.e. enhanced) effect with the phenotypic expression of proteins or other natural products toxic to invertebrate pests to provide greater-than-additive control of these pests.

Compositions of this disclosure can also optionally comprise plant nutrients, e.g., a fertilizer composition comprising at least one plant nutrient selected from nitrogen, phosphorus, potassium, sulfur, calcium, magnesium, iron, copper, boron, manganese, zinc, and molybdenum. Of note are compositions comprising at least one fertilizer composition comprising at least one plant nutrient selected from nitrogen, phosphorus, potassium, sulfur, calcium and magnesium. Compositions of the present disclosure which further comprise at least one plant nutrient can be in the form of liquids or solids. Of note are solid formulations in the form of granules, small sticks or tablets. Solid formulations comprising a fertilizer composition can be prepared by mixing the compound or composition of the present disclosure with the fertilizer composition together with formulating ingredients and then preparing the formulation by methods such as granulation or extrusion. Alternatively solid formulations can be prepared by spraying a solution or suspension of a compound or composition of the present disclosure in a volatile solvent onto a previous prepared fertilizer composition in the form of dimensionally stable mixtures, e.g., granules, small sticks or tablets, and then evaporating the solvent.

Nonagronomic uses refer to invertebrate pest control in the areas other than fields of crop plants. Nonagronomic uses of the present compounds and compositions include control of invertebrate pests in stored grains, beans and other foodstuffs, and in textiles such as clothing and carpets. Nonagronomic uses of the present compounds and compositions also include invertebrate pest control in ornamental plants, forests, in yards, along roadsides and railroad rights of way, and on turf such as lawns, golf courses and pastures. Nonagronomic uses of the present compounds and compositions also include invertebrate pest control in houses and other buildings which may be occupied by humans and/or companion, farm, ranch, zoo or other animals. Nonagronomic uses of the present compounds and compositions also include the control of pests such as termites that can damage wood or other structural materials used in buildings.

Nonagronomic uses of the present compounds and compositions also include protecting human and animal health by controlling invertebrate pests that are parasitic or transmit infectious diseases. The controlling of animal parasites includes controlling external parasites that are parasitic to the surface of the body of the host animal (e.g., shoulders, armpits, abdomen, inner part of the thighs) and internal parasites that are parasitic to the inside of the body of the host animal (e.g., stomach, intestine, lung, veins, under the skin, lymphatic tissue). External parasitic or disease transmitting pests include, for example, chiggers, ticks, lice, mosquitoes, flies, mites and fleas. Internal parasites include heartworms, hookworms and helminths. Compounds and compositions of this present disclosure are suitable for systemic and/or non-systemic control of infestation or infection by parasites on animals. Compounds and compositions of the present disclosure are particularly suitable for combating external parasitic or disease transmitting pests. Compounds and compositions of the present disclosure are suitable for combating parasites that infest agricultural working animals, such as cattle, sheep, goats, horses, pigs, donkeys, camels, buffalos, rabbits, hens, turkeys, ducks, geese and bees; pet animals and domestic animals such as dogs, cats, pet birds and aquarium fish; as well as so-called experimental animals, such as hamsters, guinea pigs, rats and mice. By combating these parasites, fatalities and performance reduction (in terms of meat, milk, wool, skins, eggs, honey, etc.) are reduced, so that applying a composition comprising a compound of the present disclosure allows more economic and simple husbandry of animals.

Examples of agronomic or nonagronomic invertebrate pests include eggs, larvae and adults of the order Lepidoptera, such as army worms, cutworms, loopers, and heliothines in the family Noctuidae (e.g., pink stem borer (*Sesamia inferens* Walker), corn stalk borer (*Sesamia nonagrioides* Lefebvre), southern armyworm (*Spodoptera eridania* Cramer), fall armyworm (*Spodoptera frugiperda* J. E. Smith), beet armyworm (*Spodoptera exigua* Hübner), cotton leafworm (*Spodoptera littoralis* Boisduval), yellowstriped armyworm (*Spodoptera ornithogalli* Guenée), black cutworm (*Agrotis ipsilon* Hufnagel), velvetbean caterpillar (*Anticarsia gemmatalis* Hübner), green fruitworm (*Lithophane antennata* Walker), cabbage armyworm (*Barathra brassicae* Linnaeus), soybean looper (*Pseudoplusia includens* Walker), cabbage looper (*Trichoplusia ni* Hübner), tobacco budworm (*Heliothis virescens* Fabricius)); borers, casebearers, webworms, coneworms, cabbageworms and skeletonizers from the family Pyralidae (e.g., European corn borer (*Ostrinia nubilalis* Hübner), navel orangeworm (*Amyelois transitella* Walker), corn root webworm (*Crambus caliginosellus* Clemens), sod webworms (Pyralidae: *Crambinae*) such as sod worm (*Herpetogramma licarsisalis* Walker), sugarcane stem borer (*Chilo infuscatellus* Snellen), tomato small borer (*Neoleucinodes elegantalis* Guenée), green leafroller (*Cnaphalocrocis medinalis* Guenée), grape leaffolder (*Desmia funeralis* Hübner), pickleworm (*Diaphania nitidalis* Stoll), cabbage center grub (*Hellula hydralis* Guenée), yellow stem borer (*Scirpophaga incertulas* Walker), white stem borer (*Scirpophaga innotata* Walker), top shoot borer (*Scirpophaga nivella* Fabricius), dark-headed rice borer (*Chilo polychrysus* Meyrick), striped riceborer (*Chilo suppressalis* Walker), cabbage cluster caterpillar (*Crocidolomia binotalis* Zeller)); leafrollers, budworms, seed worms, and fruit worms in the family Tortricidae (e.g., codling moth (*Cydia pomonella* Linnaeus), grape berry moth (*Paralobesia viteana* Clemens), oriental fruit moth *IGrapholita molesta* Busck), citrus false codling moth (*Cryptophlebia leucotreta* Meyrick), citrus borer (*Gymnandrosoma aurantianum* Lima), redbanded leafroller (*Argyrotaenia velutinana* Walker), obliquebanded leafroller (*Choristoneura rosaceana* Harris), light brown apple moth (*Epiphyas postvittana* Walker), European grape berry moth (*Eupoecilia ambiguella* Hübner), apple bud moth (*Pandemis pyrusana* Kearfott), omnivorous leafroller (*Platynota stultana* Walsingham), barred fruit-tree tortrix (*Pandemis cerasana* Hübner), apple brown tortrix (*Pandemis heparana* Denis & Schiffermüller)); and many other economically important lepidoptera (e.g., diamondback moth (*Plutella xylostella* Linnaeus), pink bollworm (*Pectinophora gossypiella* Saunders), gypsy moth (*Lymantria dispar* Linnaeus), peach fruit borer (*Carposina niponensis* Walsingham), peach twig borer (*Anarsia lineatella* Zeller), potato tuberworm (*Phthorimaea operculella* Zeller), spotted teniform leafminer (*Phyllonorycter blancardella* Fabricius), Asiatic apple leafminer (*Lithocolletis ringoniella* Matsumura), rice leaffolder (*Lerodea eufala* Edwards), apple leafminer (*Leucoptera scitella* Zeller)); eggs, nymphs and adults of the order Blattodea including cockroaches from the families Blattellidae and Blattidae (e.g., oriental cockroach (*Blatta orientalis* Linnaeus), Asian cockroach (*Blatella asahinai* Mizukubo), German cockroach (*Blattella germanica* Linnaeus), brownbanded cockroach (*Supella longipalpa* Fabricius), American cockroach (*Periplaneta americana* Linnaeus), brown cockroach (*Periplaneta brunnea* Burmeister), Madeira cockroach (*Leucophaea maderae* Fabricius)), smoky brown cockroach (*Periplaneta fuliginosa* Serville), Australian Cockroach (*Periplaneta australasiae* Fabr.), lobster cockroach (*Nauphoeta cinerea* Olivier) and smooth cockroach (*Symploce pallens* Stephens)); eggs, foliar feeding, fruit feeding, root feeding, seed feeding and vesicular tissue feeding larvae and adults of the order Coleoptera including weevils from the families Anthribidae, Bruchidae, and Curculionidae (e.g., boll weevil (*Anthonomus grandis* Boheman), rice water weevil (*Lissorhoptrus oryzophilus* Kuschel), granary weevil (*Sitophilus granarius* Linnaeus), rice weevil (*Sitophilus oryzae* Linnaeus)), annual bluegrass weevil (*Listronotus maculicollis* Dietz), bluegrass billbug (*Sphenophorus parvulus* Gyllenhal), hunting billbug (*Sphenophorus venatus vestitus* Chittenden), Rocky Mountain billbug (*Sphenophorus cicatristriatus* Fahraeus)); flea beetles, cucumber beetles, rootworms, leaf beetles, potato beetles, and leafminers in the family Chrysomelidae (e.g., Colorado potato beetle (*Leptinotarsa decemlineata* Say), western corn rootworm (*Diabrotica virgifera* LeConte)); chafers and other beetles from the family Scarabaeidae (e.g., Japanese beetle (*Popillia japonica* Newman), oriental beetle (*Anomala orientalis* Waterhouse, northern masked chafer (*Cyclocephala borealis* Arrow), southern masked chafer (*Cyclocephala immaculata* Olivier or C. *lurida* Bland), dung beetle and white grub (*Aphodius* spp.), black turfgrass ataenius (*Ataenius spretulus* Haldeman), green June beetle (*Cotinis nitida* Linnaeus), Asiatic garden beetle (*Maladera castanea* Arrow), May/June beetles (*Phyllophaga* spp.) and European chafer (*Rhizotrogus majalis* Razoumowsky)); carpet beetles from the family Dermestidae; wireworms from the family Elateridae; bark beetles from the family Scolytidae and flour beetles from the family Tenebrionidae.

In addition, agronomic and nonagronomic pests include: eggs, adults and larvae of the order Dermaptera including earwigs from the family Forficulidae (e.g., European earwig (*Forficula auricularia* Linnaeus), black earwig (*Chelisoches morio* Fabricius)); eggs, immatures, adults and nymphs of the order Hemiptera such as, plant bugs from the family Miridae, cicadas from the family Cicadidae, leafhoppers (e.g. *Empoasca* spp.) from the family Cicadellidae, bed bugs (e.g., *Cimex lectularius* Linnaeus) from the family Cimicidae, planthoppers from the families Fulgoridae and Delphacidae, treehoppers from the family Membracidae, psyllids from the families Liviidae, Psyllidae, and Triozidae, whiteflies from the family Aleyrodidae, aphids from the family Aphididae, phylloxera from the family Phylloxeridae, mealybugs from the family Pseudococcidae, scales from the families Coccidae, Diaspididae and Margarodidae, lace bugs from the family Tingidae, stink bugs from the family Pentatomidae, chinch bugs (e.g., hairy chinch bug (*Blissus leucopterus hirtus* Montandon) and southern chinch bug (*Blissus insularis* Barber)) and other seed bugs from the family Lygaeidae, spittlebugs from the family Cercopidae squash bugs from the family Coreidae, and red bugs and cotton stainers from the family Pyrrhocoridae.

Agronomic and nonagronomic pests also include : eggs, larvae, nymphs and adults of the order Acari (mites) such as spider mites and red mites in the family Tetranychidae (e.g., European red mite *(Panonychus ulmi* Koch), twospotted spider mite (*Tetranychus urticae* Koch), McDaniel spider mite (*Tetranychus mcdanieli* McGregor)); flat mites in the family Tenuipalpidae (e.g., citrus flat mite (*Brevipalpus lewisi* McGregor)); rust and bud mites in the family Eriophyidae and other foliar feeding mites and mites important in human and animal health, i.e. dust mites in the family Epidermoptidae, follicle mites in the family Demodecidae, grain mites in the family Glycyphagidae; ticks in the family Ixodidae, commonly known as hard ticks (e.g., deer tick (*Ixodes scapularis* Say), Australian paralysis tick (*Ixodes holocyclus* Neumann), American dog tick (*Dermacentor variabilis* Say), lone star tick (*Amblyomma americanum* Linnaeus)) and ticks in the family Argasidae, commonly known as soft ticks (e.g., relapsing fever tick (*Ornithodoros turicata* Duges), common fowl tick (*Argas radiatus* Raillet)); scab and itch mites in the families Psoroptidae, Pyemotidae, and Sarcoptidae; eggs, adults and immatures of the order Orthoptera including grasshoppers, locusts and crickets (e.g., migratory grasshoppers (e.g., *Melanoplus sanguinipes* Fabricius, *M. differentialis* Thomas), American grasshoppers (e.g., *Schistocerca americana* Drury), desert locust (*Schistocerca gregaria* Forsskål), migratory locust (*Locusta migratoria* Linnaeus), bush locust (*Zonocerus* spp.), house cricket (*Acheta domesticus* Linnaeus), mole crickets (e.g., tawny mole cricket (*Scapteriscus vicinus* Scudder) and southern mole cricket (*Scapteriscus borellii* Giglio-Tos)); eggs, adults and immatures of the order Diptera including leafminers (e.g., *Liriomyza* spp. such as serpentine vegetable leafminer (*Liriomyza sativae* Blanchard)), midges, fruit flies (Tephritidae), frit flies (e.g., *Oscinella frit* Linnaeus), soil maggots, house flies (e.g., *Musca domestica* Linnaeus), lesser house flies (e.g., *Fannia canicularis* Linnaeus, *F. femoralis* Stein), stable flies (e.g., *Stomoxys calcitrans* Linnaeus), face flies, horn flies, blow flies (e.g., *Chrysomya* spp., *Phormia* spp.), and other muscoid fly pests, horse flies (e.g., *Tabanus* spp.), bot flies (e.g., *Gasterophilus* spp., *Oestrus* spp.), cattle grubs (e.g., *Hypoderma* spp.), deer flies (e.g., *Chrysops* spp.), keds (e.g., *Melophagus ovinus* Linnaeus) and other Brachycera, mosquitoes (e.g., *Aedes* spp., *Anopheles* spp., *Culex* spp.), black flies (e.g., *Prosimulium* spp., *Simulium* spp.), biting midges, sand flies, sciarids, and other Nematocera; eggs, adults and immatures of the order Thysanoptera including onion thrips (*Thrips tabaci* Lindeman), flower thrips (*Frankliniella* spp.), and other foliar feeding thrips; insect pests of the order Hymenoptera including ants of the Family Formicidae including the Florida carpenter ant (*Camponotus floridanus* Buckley), red carpenter ant (*Camponotus ferrugineus* Fabricius), black carpenter ant (*Camponotus pennsylvanicus* De Geer), white-footed ant (*Technomyrmex albipes* F. Smith), big headed ants (*Pheidole* sp.), ghost ant (*Tapinoma melanocephalum* Fabricius); Pharaoh ant (*Monomorium pharaonis* Linnaeus), little fire ant (*Wasmannia auropunctata* Roger), fire ant (*Solenopsis geminata* Fabricius), red imported fire ant (*Solenopsis invicta* Buren), Argentine ant (*Iridomyrmex humilis* Mayr), crazy ant (*Paratrechina longicornis* Latreille), pavement ant (*Tetramorium caespitum* Linnaeus), cornfield ant (*Lasius alienus* Förster) and odorous house ant (*Tapinoma sessile* Say). Other Hymenoptera including bees (including carpenter bees), hornets, yellow jackets, wasps, and sawflies (*Neodiprion* spp.; *Cephus* spp.); insect pests of the order Isoptera including termites in the Termitidae (e.g., *Macrotermes* sp., *Odontotermes obesus* Rambur), Kalotermitidae (e.g., *Cryptotermes* sp.), and Rhinotermitidae (e.g., *Reticulitermes* sp., *Coptotermes* sp., *Heterotermes tenuis* Hagen) families, the eastern subterranean termite (*Reticulitermes flavipes* Kollar), western subterranean termite (*Reticulitermes hesperus* Banks), Formosan subterranean termite (*Coptotermes formosanus* Shiraki), West Indian dry wood termite (*Incisitermes immigrans* Snyder), powder post termite (*Cryptotermes brevis* Walker), dry wood termite (*Incisitermes snyderi* Light), southeastern subterranean termite (*Reticulitermes virginicus* Banks), western drywood termite (*Incisitermes minor* Hagen), arboreal termites such as *Nasutitermes* sp. and other termites of economic importance; insect pests of the order Thysanura such as silverfish (*Lepisma saccharina* Linnaeus) and firebrat (*Thermobia domestica* Packard); insect pests of the orders Mallophaga and Phthiraptera, and including the head louse (*Pediculus humanus capitis* De Geer), body louse (*Pediculus humanus* Linnaeus), chicken body louse (*Menacanthus stramineus* Nitzsch), dog biting louse (*Trichodectes canis* De Geer), fluff louse (*Goniocotes gallinae* De Geer), sheep body louse (*Bovicola ovis* Schrank), short-nosed cattle louse (*Haematopinus eurysternus* Nitzsch), longnosed cattle louse (*Linognathus vituli* Linnaeus) and other sucking and chewing parasitic lice that attack man and animals; insect pests of the order Siphonoptera including the oriental rat flea (*Xenopsylla cheopis* Rothschild), cat flea (*Ctenocephalides felis* Bouché), dog flea (*Ctenocephalides canis* Curtis), hen flea (*Ceratophyllus gallinae* Schrank), sticktight flea (*Echidnophaga gallinacea* Westwood), human flea (*Pulex irritans* Linnaeus) and other fleas afflicting mammals and birds. Additional arthropod pests covered include: spiders in the order Araneae such as the brown recluse spider (*Loxosceles reclusa* Gertsch & Mulaik) and the black widow spider (*Latrodectus mactans* Fabricius), and centipedes in the order Scutigeromorpha such as the house centipede (*Scutigera coleoptrata* Linnaeus).

Examples of invertebrate pests of stored grain include larger grain borer (*Prostephanus truncatus* Horn), lesser grain borer (*Rhyzopertha dominica* Fabricius), rice weevil (*Sitophilus oryzae* Linnaeus), maize weevil (*Sitophilus zeamais* Motschulsky), cowpea weevil (*Callosobruchus maculatus* Fabricius), red flour beetle (*Tribolium castaneum* Herbst), granary weevil (*Sitophilus granarius* Linnaeus), Indian meal moth (*Plodia interpunctella* Hübner), Mediterranean flour beetle (*Ephestia kuehniella* Zeller) and flat or rusty grain beetle (*Cryptolestes ferrugineus* Stephens).

Compounds of the present disclosure may have activity on members of the Classes Nematoda, Cestoda, Trematoda, and Acanthocephala including economically important members of the orders Strongylida, Ascaridida, Oxyurida, Rhabditida, Spirurida, and Enoplida such as but not limited to economically important agricultural pests (i.e. root knot nematodes in the genus *Meloidogyne,* lesion nematodes in the genus *Pratylenchus,* stubby root nematodes in the genus *Trichodorus,* etc.) and animal and human health pests (i.e. all economically important flukes, tapeworms, and roundworms, such as *Strongylus vulgaris* in horses, *Toxocara canis* in dogs, *Haemonchus contortus* in sheep, *Dirofilaria immitis* Leidy in dogs, *Anoplocephala perfoliata* in horses, *Fasciola hepatica* Linnaeus in ruminants, etc.).

Compounds of the disclosure may have activity against pests in the order Lepidoptera (e.g., *Alabama argillacea* Hübner (cotton leaf worm), *Archips argyrospila* Walker (fruit tree leaf roller), A. *rosana* Linnaeus (European leaf roller) and other *Archips* species, *Chilo suppressalis* Walker (rice stem borer), *Cnaphalocrocis medinalis* Guenée (rice leaf roller), *Crambus caliginosellus* Clemens (corn root webworm), *Crambus teterrellus* Zincken (bluegrass webworm), *Cydia pomonella* Linnaeus (codling moth), *Earias insulana* Boisduval (spiny bollworm), *Earias vittella* Fabricius (spotted bollworm), *Helicoverpa armigera* Hübner (Old World bollworm), *Helicoverpa zea* Boddie (corn earworm), *Heliothis virescens* Fabricius (tobacco budworm), *Herpetogramma licarsisalis* Walker (sod webworm), *Lobesia botrana* Denis & Schiffermüller (grape berry moth), *Pectinophora gossypiella* Saunders (pink bollworm), *Phyllocnistis citrella* Stainton (citrus leafminer), *Pieris brassicae* Linnaeus (large white butterfly), *Pieris rapae* Linnaeus (small white butterfly), *Plutella xylostella* Linnaeus (diamondback moth), *Spodoptera exigua* Hübner (beet armyworm), *Spodoptera litura* Fabricius (tobacco cutworm, cluster caterpillar), *Spodoptera frugiperda* J. E. Smith (fall armyworm), *Trichoplusia ni* Hübner (cabbage looper) and *Tula absoluta* Meyrick (tomato leafminer)).

Compounds of the disclosure have significant activity on members from the order Hemiptera including: *Acyrthosiphon pisum* Harris (pea aphid), *Aphis craccivora* Koch (cowpea aphid), *Aphis fabae* Scopoli (black bean aphid), *Aphis gossypii* Glover (cotton aphid, melon aphid), *Aphis pomi* De Geer (apple aphid), *Aphis spiraecola* Patch (spirea aphid), *Aulacorthum solani* Kaltenbach (foxglove aphid), *Chaetosiphon fragaefolii* Cockerell (strawberry aphid), *Diuraphis noxia* Kurdjumov/Mordvilko (Russian wheat aphid), *Dysaphis plantaginea* Passerini (rosy apple aphid), *Eriosoma lanigerum* Hausmann (woolly apple aphid), *Hyalopterus pruni* Geoffroy (mealy plum aphid), *Lipaphis pseudobrassicae* Davis (turnip aphid), *Metopolophium dirrhodum* Walker (rose-grain aphid), *Macrosiphum euphorbiae* Thomas (potato aphid), *Myzus persicae* Sulzer (peach-potato aphid, green peach aphid), *Nasonovia ribisnigri* Mosley (lettuce aphid), *Pemphigus* spp. (root aphids and gall aphids), *Rhopalosiphum maidis* Fitch (corn leaf aphid), *Rhopalosiphum padi* Linnaeus (bird cherry-oat aphid), *Schizaphis graminum* Rondani (greenbug), *Sitobion avenae* Fabricius (English grain aphid), *Therioaphis maculata* Buckton (spotted alfalfa aphid), *Toxoptera aurantii* Boyer de Fonscolombe (black citrus aphid), and *Toxoptera citricidus* Kirkaldy (brown citrus aphid); *Adelges* spp. (adelgids); *Phylloxera devastatrix* Pergande (pecan phylloxera); *Bemisia tabaci* Gennadius (tobacco whitefly, sweetpotato whitefly), *Bemisia argentifolii* Bellows & Perring (silverleaf whitefly), *Dialeurodes citri* Ashmead (citrus whitefly) and *Trialeurodes vaporariorum* Westwood (greenhouse whitefly); *Empoasca fabae* Harris (potato leafhopper), *Laodelphax striatellus* Fallen (smaller brown planthopper), *Macrosteles quadrilineatus* Forbes (aster leafhopper), *Nephotettix cincticeps* Uhler (green rice leafhopper), *Nephotettix nigropictus* Stål (rice leafhopper), *Nilaparvata lugens* Stål (brown planthopper), *Peregrinus maidis* Ashmead (corn planthopper), *Sogatella furcifera* Horváth (white-backed planthopper), *Tagosodes orizicolus* Muir (rice delphacid), *Typhlocyba pomaria* McAtee (white apple leafhopper), Erythroneura spp. (grape leafhoppers); *Magicidada septendecim* Linnaeus (periodical cicada); *Icerya purchasi* Maskell (cottony cushion scale), *Quadraspidiotus perniciosus* Comstock (San Jose scale); *Pianococcus citri* Risso (citrus mealybug); *Pseudococcus* spp. (other mealybug complex); *Cacopsylla pyricola* Foerster (pear psylla), *Trioza diospyri* Ashmead (persimmon psylla).

Compounds of this disclosure also have activity on members from the order Hemiptera including: *Acrosternum hilare* Say (green stink bug), *Anasa tristis* De Geer (squash bug), *Blissus leucopterus leucopterus* Say (chinch bug), *Cimex lectularius* Linnaeus (bed bug) *Corythucha gossypii* Fabricius (cotton lace bug), *Cyrtopeltis modesta* Distant (tomato bug), *Dysdercus suturellus* Herrich-Sch5ffer (cotton stainer), *Euschistus servus* Say (brown stink bug), *Euschistus variolarius* Palisot de Beauvois (one-spotted stink bug), *Graptostethus* spp. (complex of seed bugs), *Halyomorpha halys* Stål (brown marmorated stink bug), *Leptoglossus corculus* Say (leaf-footed pine seed bug), *Lygus lineolaris* Palisot de Beauvois (tarnished plant bug), *Nezara viridula* Linnaeus (southern green stink bug), *Oebalus pugnax* Fabricius (rice stink bug), *Oncopeltus fasciatus* Dallas (large milkweed bug), *Pseudatomoscelis seriatus* Reuter (cotton fleahopper). Other insect orders controlled by compounds of the disclosure include Thysanoptera (e.g., *Frankliniella occidentalis* Pergande (western flower thrips), *Scirtothrips citri* Moulton (citrus thrips), *Scirtothrips variabilis* Beach (soybean thrips), and *Thrips tabaci* Lindeman (onion thrips); and the order Coleoptera (e.g., *Leptinotarsa decemlineata* Say (Colorado potato beetle), *Epilachna varivestis* Mulsant (Mexican bean beetle) and wireworms of the genera *Agriotes, Athous* or *Limonius).*

Of note is use of compounds of this disclosure for controlling western flower thrips *(Frankliniella occidentalis).* Of note is use of compounds of this disclosure for controlling potato leafhopper (*Empoasca fabae*)*.* Of note is use of compounds of this disclosure for controlling cotton melon aphid (*Aphis gossypii*)*.* Of note is use of compounds of this disclosure for controlling green peach aphid (*Myzus persicae*)*.* Of note is use of compounds of this disclosure for controlling sweetpotato whitefly (*Bemisia tabaci*)*.*

Of note is use of compounds of this disclosure for controlling western flower thrips (*Frankliniella occidentalis*)*.* Of note is use of compounds of this disclosure for controlling potato leafhopper (*Empoasca fabae*)*.* Of note is use of compounds of this disclosure for controlling cotton melon aphid (*Aphis gossypii*)*.* Of note is use of compounds of this disclosure for controlling green peach aphid (*Myzus persicae*)*.* Of note is use of compounds of this disclosure for controlling sweetpotato whitefly (*Bemisia tabaci*)*.*

Compounds of the present disclosure may also be useful for increasing vigor of a crop plant. This method comprises contacting the crop plant (e.g., foliage, flowers, fruit or roots) or the seed from which the crop plant is grown with a compound of Formula **1** in amount sufficient to achieve the desired plant vigor effect (i.e. biologically effective amount). Typically the compound of Formula **1** is applied in a formulated composition. Although the compound of Formula **1** is often applied directly to the crop plant or its seed, it can also be applied to the locus of the crop plant, i.e. the environment of the crop plant, particularly the portion of the environment in close enough proximity to allow the compound of Formula **1** to migrate to the crop plant. The locus relevant to this method most commonly comprises the growth medium (i.e. medium providing nutrients to the plant), typically soil in which the plant is grown. Treatment of a crop plant to increase vigor of the crop plant thus comprises contacting the crop plant, the seed from which the crop plant is grown or the locus of the crop plant with a biologically effective amount of a compound of Formula **1.**

Increased crop vigor can result in one or more of the following observed effects: (a) optimal crop establishment as demonstrated by excellent seed germination, crop emergence and crop stand; (b) enhanced crop growth as demonstrated by rapid and robust leaf growth (e.g., measured by leaf area index), plant height, number of tillers (e.g., for rice), root mass and overall dry weight of vegetative mass of the crop; (c) improved crop yields, as demonstrated by time to flowering, duration of flowering, number of flowers, total biomass accumulation (i.e. yield quantity) and/or fruit or grain grade marketability of produce (i.e. yield quality); (d) enhanced ability of the crop to withstand or prevent plant disease infections and arthropod, nematode or mollusk pest infestations; and (e) increased ability of the crop to withstand environmental stresses such as exposure to thermal extremes, suboptimal moisture or phytotoxic chemicals.

The compounds of the present disclosure may increase the vigor of treated plants compared to untreated plants by killing or otherwise preventing feeding of phytophagous invertebrate pests in the environment of the plants. In the absence of such control of phytophagous invertebrate pests, the pests reduce plant vigor by consuming plant tissues or sap, or transmiting plant pathogens such as viruses. Even in the absence of phytophagous invertebrate pests, the compounds of the disclosure may increase plant vigor by modifying metabolism of plants. Generally, the vigor of a crop plant will be most significantly increased by treating the plant with a compound of the disclosure if the plant is grown in a nonideal environment, i.e. an environment comprising one or more aspects adverse to the plant achieving the full genetic potential it would exhibit in an ideal environment.

Of note is a method for increasing vigor of a crop plant wherein the crop plant is grown in an environment comprising phytophagous invertebrate pests. Also of note is a method for increasing vigor of a crop plant wherein the crop plant is grown in an environment not comprising phytophagous invertebrate pests. Also of note is a method for increasing vigor of a crop plant wherein the crop plant is grown in an environment comprising an amount of moisture less than ideal for supporting growth of the crop plant. Of note is a method for increasing vigor of a crop plant wherein the crop is rice. Also of note is a method for increasing vigor of a crop plant wherein the crop is maize (corn). Also of note is a method for increasing vigor of a crop plant wherein the crop is soybean.

Compounds of this disclosure can also be mixed with one or more other biologically active compounds or agents including insecticides, fungicides, nematocides, bactericides, acaricides, herbicides, herbicide safeners, growth regulators such as insect molting inhibitors and rooting stimulants, chemosterilants, semiochemicals, repellents, attractants, pheromones, feeding stimulants, other biologically active compounds or entomopathogenic bacteria, virus or fungi to form a multi-component pesticide giving an even broader spectrum of agronomic and nonagronomic utility. Thus, the present disclosure also pertains to a composition comprising a biologically effective amount of a compound of Formula **1,** at least one additional component selected from the group consisting of surfactants, solid diluents and liquid diluents, and at least one additional biologically active compound or agent. For mixtures of the present disclosure, the other biologically active compounds or agents can be formulated together with the present compounds, including the compounds of Formula **1,** to form a premix, or the other biologically active compounds or agents can be formulated separately from the present compounds, including the compounds of Formula **1,** and the two formulations combined together before application (e.g., in a spray tank) or, alternatively, applied in succession.

Examples of such biologically active compounds or agents with which compounds of this disclosure can be formulated are insecticides such as abamectin, acephate, acequinocyl, acetamiprid, acrinathrin, afidopyropen ([(3*S*,4*R*,4a*R*,6*S*,6a*S*,12*R*,12a*S*,12b*S*)-3-[(cyclopropylcarbonyl)oxy]-1,3,4,4a,5,6,6a,12,12a,12b-decahydro-6,12-dihydroxy-4,6a,12b-trimethyl-11-oxo-9-(3-pyridinyl)-2*H*,11*H*-naphtho[2,1-*b*]pyrano[3,4-*e*]pyran-4-yl]methyl cyclopropanecarboxylate), amidoflumet, amitraz, avermectin, azadirachtin, azinphos-methyl, benfuracarb, bensultap, bifenthrin, bifenazate, bistrifluron, borate, buprofezin, cadusafos, carbaryl, carbofuran, cartap, carzol, chlorantraniliprole, chlorfenapyr, chlorfluazuron, chlorpyrifos, chlorpyrifos-methyl, chromafenozide, clofentezin, clothianidin, cyantraniliprole (3-bromo-1-(3-chloro-2-pyridinyl)-*N*-[4-cyano-2-methyl-6-[(methylamino)carbonyl]phenyl]-1*H*-pyrazole-5-carboxamide), cyclaniliprole (3-bromo-*N*-[2-bromo-4-chloro-6-[[(1-cyclopropylethyl) amino carbonyl]phenyl] -1-(3-chloro-2-pyridinyl)-1*H*-pyrazole-5-carboxamide), cycloprothrin, cycloxaprid ((5*S*,8*R*)-1-[(6-chloro-3-pyridinyl)methyl]-2,3,5,6,7,8-hexahydro-9-nitro-5 ,8-Epoxy-1*H*-imidazo[1,2-*a*]azepine) cyflumetofen, cyfluthrin, beta-cyfluthrin, cyhalothrin, gamma-cyhalothrin, lambda-cyhalothrin, cypermethrin, alpha-cypermethrin, zeta-cypermethrin, cyromazine, deltamethrin, diafenthiuron, diazinon, dieldrin, diflubenzuron, dimefluthrin, dimehypo, dimethoate, dinotefuran, diofenolan, emamectin, endosulfan, esfenvalerate, ethiprole, etofenprox, etoxazole, fenbutatin oxide, fenitrothion, fenothiocarb, fenoxycarb, fenpropathrin, fenvalerate, fipronil, flometoquin (2-ethyl-3,7-dimethyl-6-[4-(trifluoromethoxy)phenoxy]-4-quinolinyl methyl carbonate), flonicamid, flubendiamide, flucythrinate, flufenerim, flufenoxuron, flufenoxystrobin (methyl (α*E*)-2-[[2-chloro-4-(trifluoromethyl)phenoxylmethyl]-α-(methoxymethylene)benzeneacetate), flufensulfone (5-chloro-2-[(3,4,4-trifluoro-3-buten-1-yl)sulfonyl]thiazole), fluhexafon, fluopyram, flupiprole (1-[2,6-dichloro-4-(trifluoromethyl)phenyl]-5-[(2-methyl-2-propen-1-yl)amino]-4-[(trifluoromethyl)sulfinyl]-1*H*-pyrazole-3-carbonitrile), flupyradifurone (4-[[(6-chloro-3-pyridinyl)methyl](2,2-difluoroethyl)amino]-2(5*H*)-furanone), fluvalinate, tau-fluvalinate, fonophos, formetanate, fosthiazate, halofenozide, heptafluthrin ([2,3,5,6-tetrafluoro-4-(methoxymethyl)phenyl]methyl 2,2-dimethyl-3-[(1*Z*)-3,3,3-trifluoro-1-propen-1-yl]cyclopropanecarboxylate), hexaflumuron, hexythiazox, hydramethylnon, imidacloprid, indoxacarb, insecticidal soaps, isofenphos, lufenuron, malathion, meperfluthrin ([2,3,5,6-tetrafluoro-4-(methoxymethyl)phenyl]methyl (1*R*,3*S*)-3-(2,2-dichloroethenyl)-2,2-dimethylcyclopropanecarboxylate), metaflumizone, metaldehyde, methamidophos, methidathion, methiodicarb, methomyl, methoprene, methoxychlor, metofluthrin, methoxyfenozide, metofluthrin, monocrotophos, monofluorothrin ([2,3,5,6-tetrafluoro-4-(methoxymethyl)phenyl]methyl 3-(2-cyano-1-propen-1-yl)-2,2-dimethylcyclopropanecarboxylate), nicotine, nitenpyram, nithiazine, novaluron, noviflumuron, oxamyl, parathion, parathion-methyl, permethrin, phorate, phosalone, phosmet, phosphamidon, pirimicarb, profenofos, profluthrin, propargite, protrifenbute, pyflubumide (1,3,5-trimethyl-*N*-(2-methyl-1-oxopropyl)-*N*-[3-(2-methylpropyl)-4-[2,2,2-trifluoro-1-methoxy-1-(trifluoromethyl)ethyl]phenyl]-1*H*-pyrazole-4-carboxamide), pymetrozine, pyrafluprole, pyrethrin, pyridaben, pyridalyl, pyrifluquinazon, pyriminostrobin (methyl (α*E*)-2-[[[2-[(2,4-dichlorophenyl)amino]-6-(trifluoromethyl)-4-pyrimidinyl]oxy]methyl]-α-(methoxymethylene)benzeneacetate), pyriprole, pyriproxyfen, rotenone, ryanodine, silafluofen, spinetoram, spinosad, spirodiclofen, spiromesifen, spirotetramat, sulprofos, sulfoxaflor (*N*-[methyloxido [1-[6-(trifluoromethyl)-3-pyridinyl]ethyl]-λ⁴-sulfanylidene]cyanamide), tebufenozide, tebufenpyrad, teflubenzuron, tefluthrin, terbufos, tetrachlorvinphos, tetramethrin, tetramethylfluthrin ([2,3,5,6-tetrafluoro-4-(methoxymethyl)phenyl]methyl 2,2,3,3-tetramethylcyclopropanecarboxylate), tetraniliprole, thiacloprid, thiamethoxam, thiodicarb, thiosultap-sodium, tioxazafen (3-phenyl-5-(2-thienyl)-1,2,4-oxadiazole), tolfenpyrad, tralomethrin, triazamate, trichlorfon, triflumezopyrim (2,4-dioxo-1-(5-pyrimidinylmethyl)-3-[3-(trifluoromethyl)phenyl]-2*H*-pyrido[1,2-*a*]pyrimidinium inner salt), triflumuron, *Bacillus thuringiensis* delta-endotoxins, entomopathogenic bacteria, entomopathogenic viruses and entomopathogenic fungi.

Of note are insecticides such as abamectin, acetamiprid, acrinathrin, afidopyropen, amitraz, avermectin, azadirachtin, benfuracarb, bensultap, bifenthrin, buprofezin, cadusafos, carbaryl, cartap, chlorantraniliprole, chlorfenapyr, chlorpyrifos, clothianidin, cyantraniliprole, cyclaniliprole, cycloprothrin, cyfluthrin, beta-cyfluthrin, cyhalothrin, gamma-cyhalothrin, lambda-cyhalothrin, cypermethrin, alpha-cypermethrin, zeta-cypermethrin, cyromazine, deltamethrin, dieldrin, dinotefuran, diofenolan, emamectin, endosulfan, esfenvalerate, ethiprole, etofenprox, etoxazole, fenitrothion, fenothiocarb, fenoxycarb, fenvalerate, fipronil, flometoquin, flonicamid, flubendiamide, flufenoxuron, flufenoxystrobin, flufensulfone, flupiprole, flupyradifurone, fluvalinate, formetanate, fosthiazate, heptafluthrin, hexaflumuron, hydramethylnon, imidacloprid, indoxacarb, lufenuron, meperfluthrin, metaflumizone, methiodicarb, methomyl, methoprene, methoxyfenozide, metofluthrin, monofluorothrin, nitenpyram, nithiazine, novaluron, oxamyl, pyflubumide, pymetrozine, pyrethrin, pyridaben, pyridalyl, pyriminostrobin, pyriproxyfen, ryanodine, spinetoram, spinosad, spirodiclofen, spiromesifen, spirotetramat, sulfoxaflor, tebufenozide, tetramethrin, tetramethylfluthrin, thiacloprid, thiamethoxam, thiodicarb, thiosultap-sodium, tralomethrin, triazamate, triflumezopyrim, triflumuron, *Bacillus thuringiensis* delta-endotoxins, all strains of *Bacillus thuringiensis* and all strains of nucleo polyhedrosis viruses.

One embodiment of biological agents for mixing with compounds of this disclosure include entomopathogenic bacteria such as *Bacillus thuringiensis,* and the encapsulated delta-endotoxins of *Bacillus thuringiensis* such as MVP^{®} and MVPII^{®} bioinsecticides prepared by the CellCap^{®} process (CellCap^{®}, MVP^{®} and MVPII^{®} are trademarks of Mycogen Corporation, Indianapolis, Indiana, USA); entomopathogenic fungi such as green muscardine fungus; and entomopathogenic (both naturally occurring and genetically modified) viruses including baculovirus, nucleopolyhedro virus (NPV) such as *Helicoverpa zea* nucleopolyhedrovirus (HzNPV), *Anagrapha falcifera* nucleopolyhedrovirus (AfNPV); and granulosis virus (GV) such as *Cydia pomonella* granulosis virus (CpGV).

Of particular note is such a combination where the other invertebrate pest control active ingredient belongs to a different chemical class or has a different site of action than the compound of Formula **1.** In certain instances, a combination with at least one other invertebrate pest control active ingredient having a similar spectrum of control but a different site of action will be particularly advantageous for resistance management. Thus, a composition of the present disclosure can further comprise a biologically effective amount of at least one additional invertebrate pest control active ingredient having a similar spectrum of control but belonging to a different chemical class or having a different site of action. These additional biologically active compounds or agents include, but are not limited to, acetylcholinesterase (AChE) inhibitors such as the carbamates methomyl, oxamyl, thiodicarb, triazamate, and the organophosphates chlorpyrifos; GABA-gated chloride channel antagonists such as the cyclodienes dieldrin and endosulfan, and the phenylpyrazoles ethiprole and fipronil; sodium channel modulators such as the pyrethroids bifenthrin, cyfluthrin, *beta-*cyfluthrin, cyhalothrin, lambda-cyhalothrin, cypermethrin, deltamethrin, dimefluthrin, esfenvalerate, metofluthrin and profluthrin; nicotinic acetylcholinereceptor (nAChR) agonists such as the neonicotinoids acetamiprid, clothianidin, dinotefuran, imidacloprid, nitenpyram, nithiazine, thiacloprid, and thiamethoxam, the sulfoximine sulfoxaflor, the butenolide flupyradifurone, and the mesoionic triflumezopyrim; nicotinic acetylcholine receptor (nAChR) allosteric activators such as the spinosyns spinetoram and spinosad; chloride channel activators such as the avermectins abamectin and emamectin; juvenile hormone mimics such as diofenolan, methoprene, fenoxycarb and pyriproxyfen; chordotonal organ modulators such as pymetrozine, pyrifluquinazon and flonicamid; mite growth inhibitors such as etoxazole; inhibitors of mitochondrial ATP synthase such as propargite; uncouplers of oxidative phosphorylation via disruption of the proton gradient such as chlorfenapyr; nicotinic acetylcholine receptor (nAChR) channel blockers such as the nereistoxin analogs cartap; inhibitors of chitin biosynthesis such as the benzoylureas flufenoxuron, hexaflumuron, lufenuron, novaluron, noviflumuron and triflumuron, and buprofezin; dipteran moulting disrupters such as cyromazine; ecdysone receptor agonists such as the diacylhydrazines methoxyfenozide and tebufenozide; octopamine receptor agonists such as amitraz; mitochondrial complex III electron transport inhibitors such as hydramethylnon and bifenazate; mitochondrial complex I electron transport inhibitors such as pyridaben; voltagedependent sodium channel blockers such as indoxacarb; inhibitors of acetyl CoA carboxylase such as the tetronic and tetramic acids spirodiclofen, spiromesifen and spirotetramat; mitochondrial complex II electron transport inhibitors such as the β-ketonitriles cyenopyrafen and cyflumetofen; ryanodine receptor modulators such as the anthranilic diamides chlorantraniliprole and cyantraniliprole, diamides such as flubendiamide, and ryanodine receptor ligands such as ryanodine; compounds wherein the target site responsible for biological activity is unknown or uncharacterized such as azadirachtinand pyridalyl; microbial disrupters of insect midgut membranes such as *Bacillus thuringiensis* and the delta-endotoxins they produce and *Bacillus sphaericus;* and biological agents including nuclear polyhedrosis (NPV) and other naturally occurring or genetically modified insecticidal viruses.

Further examples of biologically active compounds or agents with which compounds of this disclosure can be formulated are: fungicides such as acibenzolar-S-methyl, aldimorph, ametoctradin, amisulbrom, anilazine, azaconazole, azoxystrobin, benalaxyl (including benalaxyl-M), benodanil, benomyl, benthiavalicarb (including benthiavalicarb-isopropyl), benzovindiflupyr, bethoxazin, binapacryl, biphenyl, bitertanol, bixafen, blasticidin-S, boscalid, bromuconazole, bupirimate, buthiobate, carboxin, carpropamid, captafol, captan, carbendazim, chloroneb, chlorothalonil, chlozolinate, copper hydroxide, copper oxychloride, copper sulfate, coumoxystrobin, cyazofamid, cyflufenamid, cymoxanil, cyproconazole, cyprodinil, dichlofluanid, diclocymet, diclomezine, dicloran, diethofencarb, difenoconazole, diflumetorim, dimethirimol, dimethomorph, dimoxystrobin, diniconazole (including diniconazole-M), dinocap, dithianon, dithiolanes, dodemorph, dodine, econazole, etaconazole, edifenphos, enoxastrobin (also known as enestroburin), epoxiconazole, ethaboxam, ethirimol, etridiazole, famoxadone, fenamidone, fenaminstrobin, fenarimol, fenbuconazole, fenfuram, fenhexamide, fenoxanil, fenpiclonil, fenpropidin, fenpropimorph, fenpyrazamine, fentin acetate, fentin hydroxide, ferbam, ferimzone, flometoquin, fluazinam, fludioxonil, flufenoxystrobin, flumorph, fluopicolide, fluopyram, fluoxastrobin, fluquinconazole, flusilazole, flusulfamide, flutianil, flutolanil, flutriafol, fluxapyroxad, folpet, fthalide (also known as phthalide), fuberidazole, furalaxyl, furametpyr, hexaconazole, hymexazole, guazatine, imazalil, imibenconazole, iminoctadine albesilate, iminoctadine triacetate, iodicarb, ipconazole, isofetamid, iprobenfos, iprodione, iprovalicarb, isoprothiolane, isopyrazam, isotianil, kasugamycin, kresoxim-methyl, mancozeb, mandipropamid, mandestrobin, maneb, mapanipyrin, mepronil, meptyldinocap, metalaxyl (including metalaxyl-M/mefenoxam), metconazole, methasulfocarb, metiram, metominostrobin, metrafenone, myclobutanil, naftitine, neo-asozin (ferric methanearsonate), nuarimol, octhilinone, ofurace, orysastrobin, oxadixyl, oxathiapiprolin, oxolinic acid, oxpoconazole, oxycarboxin, oxytetracycline, penconazole, pencycuron, penflufen, penthiopyrad, perfurazoate, phosphorous acid (including salts thereof, e.g., fosetyl-aluminm), picoxystrobin, piperalin, polyoxin, probenazole, prochloraz, procymidone, propamocarb, propiconazole, propineb, proquinazid, prothiocarb, prothioconazole, pyraclostrobin, pyrametostrobin, pyraoxystrobin, pyrazophos, pyribencarb, pyributacarb, pyrifenox, pyriofenone, perisoxazole, pyrimethanil, pyrifenox, pyrrolnitrin, pyroquilon, quinconazole, quinmethionate, quinoxyfen, quintozene, silthiofam, sedaxane, simeconazole, spiroxamine, streptomycin, sulfur, tebuconazole, tebufloquin, teclofthalam, tecloftalam, tecnazene, terbinafine, tetraconazole, thiabendazole, thifluzamide, thiophanate, thiophanate-methyl, thiram, tiadinil, tolclofosmethyl, tolprocarb, tolyfluanid, triadimefon, triadimenol, triarimol, triazoxide, tribasic copper sulfate, triclopyricarb, tridemorph, trifloxystrobin, triflumizole, trimoprhamide tricyclazole, trifloxystrobin, triforine, triticonazole, uniconazole, validamycin, valifenalate (also known as valifenal), vinclozolin, zineb, ziram, zoxamide and 1-[4-[4-[5-(2,6-difluorophenyl)-4,5-dihydro-3-isoxazolyl]-2-thiazolyl]-1-piperidinyl]-2-[5-methyl-3-(trifluoromethyl)-1H-pyrazol-1-yl]ethanone; nematocides such as fluopyram, spirotetramat, thiodicarb, fosthiazate, abamectin, iprodione, fluensulfone, dimethyl disulfide, tioxazafen, 1,3-dichloropropene (1,3-D), metam (sodium and potassium), dazomet, chloropicrin, fenamiphos, ethoprophos, cadusaphos, terbufos, imicyafos, oxamyl, carbofuran, tioxazafen, *Bacillus firmus* and *Pasteuria nishizawae;* bactericides such as streptomycin; acaricides such as amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben and tebufenpyrad.

In certain instances, combinations of a compound of this disclosure with other biologically active (particularly invertebrate pest control) compounds or agents (i.e. active ingredients) can result in a greater-than-additive (i.e. enhanced) effect. Reducing the quantity of active ingredients released in the environment while ensuring effective pest control is always desirable. When enhancement of invertebrate pest control active ingredients occurs at application rates giving agronomically satisfactory levels of invertebrate pest control, such combinations can be advantageous for reducing crop production cost and decreasing environmental load.

Compounds of this disclosure and compositions thereof can be applied to plants genetically transformed to express proteins toxic to invertebrate pests (such as *Bacillus thuringiensis* delta-endotoxins). Such an application may provide a broader spectrum of plant protection and be advantageous for resistance management. The effect of the exogenously applied invertebrate pest control compounds of this disclosure may be enhanced with the expressed toxin proteins.

General references for these agricultural protectants (i.e. insecticides, fungicides, nematocides, acaricides, herbicides and biological agents) include The Pesticide Manual, 13th Edition, C. D. S. Tomlin, Ed., British Crop Protection Council, Farnham, Surrey, U.K., 2003 and The BioPesticide Manual, 2nd Edition, L. G. Copping, Ed., British Crop Protection Council, Farnham, Surrey, U.K., 2001.

For embodiments where one or more of these various mixing partners are used, the weight ratio of these various mixing partners (in total) to the compound of Formula 1 is typically between about 1:3000 and about 3000:1. Of note are weight ratios between about 1:300 and about 300:1 (for example ratios between about 1:30 and about 30:1). One skilled in the art can easily determine through simple experimentation the biologically effective amounts of active ingredients necessary for the desired spectrum of biological activity. It will be evident that including these additional components can expand the spectrum of invertebrate pests controlled beyond the spectrum controlled by the compound of Formula 1 alone.

Invertebrate pests are controlled in agronomic and nonagronomic applications by applying one or more compounds of this disclosure, typically in the form of a composition, in a biologically effective amount, to the environment of the pests, including the agronomic and/or nonagronomic locus of infestation, to the area to be protected, or directly on the pests to be controlled.

Thus the present disclosure comprises a method for controlling an invertebrate pest in agronomic and/or nonagronomic applications, comprising contacting the invertebrate pest or its environment with a biologically effective amount of one or more of the compounds of the disclosure, or with a composition comprising at least one such compound or a composition comprising at least one such compound and a biologically effective amount of at least one additional biologically active compound or agent. Examples of suitable compositions comprising a compound of the disclosure and a biologically effective amount of at least one additional biologically active compound or agent include granular compositions wherein the additional active compound is present on the same granule as the compound of the disclosure or on granules separate from those of the compound of the disclosure.

To achieve contact with a compound or composition of the disclosure to protect a field crop from invertebrate pests, the compound or composition is typically applied to the seed of the crop before planting, to the foliage (e.g., leaves, stems, flowers, fruits) of crop plants, or to the soil or other growth medium before or after the crop is planted.

One embodiment of a method of contact is by spraying. Alternatively, a granular composition comprising a compound of the disclosure can be applied to the plant foliage or the soil. Compounds of this disclosure can also be effectively delivered through plant uptake by contacting the plant with a composition comprising a compound of this disclosure applied as a soil drench of a liquid formulation, a granular formulation to the soil, a nursery box treatment or a dip of transplants. Of note is a composition of the present disclosure in the form of a soil drench liquid formulation. Also of note is a method for controlling an invertebrate pest comprising contacting the invertebrate pest or its environment with a biologically effective amount of a compound of the present disclosure or with a composition comprising a biologically effective amount of a compound of the present disclosure. Of further note is this method wherein the environment is soil and the composition is applied to the soil as a soil drench formulation. Of further note is that compounds of this disclosure are also effective by localized application to the locus of infestation. Other methods of contact include application of a compound or a composition of the disclosure by direct and residual sprays, aerial sprays, gels, seed coatings, microencapsulations, systemic uptake, baits, ear tags, boluses, foggers, fumigants, aerosols, dusts and many others. One embodiment of a method of contact is a dimensionally stable fertilizer granule, stick or tablet comprising a compound or composition of the disclosure. The compounds of this disclosure can also be impregnated into materials for fabricating invertebrate control devices (e.g., insect netting).

Compounds of the disclosure are useful in treating all plants, plant parts and seeds. Plant and seed varieties and cultivars can be obtained by conventional propagation and breeding methods or by genetic engineering methods. Genetically modified plants or seeds (transgenic plants or seeds) are those in which a heterologous gene (transgene) has been stably integrated into the plant's or seed's genome. A transgene that is defined by its particular location in the plant genome is called a transformation or transgenic event.

Genetically modified plant and seed cultivars which can be treated according to the disclosure include those that are resistant against one or more biotic stresses (pests such as nematodes, insects, mites, fungi, etc.) or abiotic stresses (drought, cold temperature, soil salinity, etc.), or that contain other desirable characteristics. Plants and seeds can be genetically modified to exhibit traits of, for example, herbicide tolerance, insect-resistance, modified oil profiles or drought tolerance.

Treatment of genetically modified plants and seeds with compounds of the disclosure may result in super-additive or enhanced effects. For example, reduction in application rates, broadening of the activity spectrum, increased tolerance to biotic/abiotic stresses or enhanced storage stability may be greater than expected from just simple additive effects of the application of compounds of the disclosure on genetically modified plants and seeds.

Compounds of this disclosure are also useful in seed treatments for protecting seeds from invertebrate pests. In the context of the present disclosure and claims, treating a seed means contacting the seed with a biologically effective amount of a compound of this disclosure, which is typically formulated as a composition of the disclosure. This seed treatment protects the seed from invertebrate soil pests and generally can also protect roots and other plant parts in contact with the soil of the seedling developing from the germinating seed. The seed treatment may also provide protection of foliage by translocation of the compound of this disclosure or a second active ingredient within the developing plant. Seed treatments can be applied to all types of seeds, including those from which plants genetically transformed to express specialized traits will germinate. Representative examples include those expressing proteins toxic to invertebrate pests, such as *Bacillus thuringiensis* toxin or those expressing herbicide resistance such as glyphosate acetyltransferase, which provides resistance to glyphosate. Seed treatments with compounds of this disclosure can also increase vigor of plants growing from the seed.

One method of seed treatment is by spraying or dusting the seed with a compound of the disclosure (i.e. as a formulated composition) before sowing the seeds. Compositions formulated for seed treatment generally comprise a film former or adhesive agent. Therefore typically a seed coating composition of the present disclosure comprises a biologically effective amount of a compound of Formula **1,** an N-oxide or salt thereof, and a film former or adhesive agent. Seed can be coated by spraying a flowable suspension concentrate directly into a tumbling bed of seeds and then drying the seeds. Alternatively, other formulation types such as wetted powders, solutions, suspoemulsions, emulsifiable concentrates and emulsions in water can be sprayed on the seed. This process is particularly useful for applying film coatings on seeds. Various coating machines and processes are available to one skilled in the art. Suitable processes include those listed in P. Kosters et al., Seed Treatment: Progress and Prospects, 1994 BCPC Mongraph No. 57, and references listed therein.

Compounds of Formula **1** and their compositions, both alone and in combination with other insecticides, nematicides, and fungicides, are particularly useful in seed treatment for crops including, but not limited to, maize or corn, soybeans, cotton, cereal (e.g., wheat, oats, barley, rye and rice), potatoes, vegetables and oilseed rape.

Other insecticides with which compounds of Formula **1** can be formulated to provide mixtures useful in seed treatment include abamectin, acetamiprid, acrinathrin, amitraz, avermectin, azadirachtin, bensultap, bifenthrin, buprofezin, cadusafos, carbaryl, carbofuran, cartap, chlorantraniliprole, chlorfenapyr, chlorpyrifos, clothianidin, cyantraniliprole, cyfluthrin, beta-cyfluthrin, cyhalothrin, gamma-cyhalothrin, lambda-cyhalothrin, cypermethrin, alpha-cypermethrin, zeta-cypermethrin, cyromazine, deltamethrin, dieldrin, dinotefuran, diofenolan, emamectin, endosulfan, esfenvalerate, ethiprole, etofenprox, etoxazole, fenothiocarb, fenoxycarb, fenvalerate, fipronil, flonicamid, flubendiamide, flufenoxuron, fluvalinate, formetanate, fosthiazate, hexaflumuron, hydramethylnon, imidacloprid, indoxacarb, lufenuron, metaflumizone, methiocarb, methomyl, methoprene, methoxyfenozide, nitenpyram, nithiazine, novaluron, oxamyl, pymetrozine, pyrethrin, pyridaben, pyridalyl, pyriproxyfen, ryanodine, spinetoram, spinosad, spirodiclofen, spiromesifen, spirotetramat, sulfoxaflor, tebufenozide, tetramethrin, thiacloprid, thiamethoxam, thiodicarb, thiosultap-sodium, tralomethrin, triazamate, triflumuron, *Bacillus thuringiensis* delta-endotoxins, all strains of *Bacillus thuringiensis* and all strains of nucleo polyhedrosis viruses.

Fungicides with which compounds of Formula **1** can be formulated to provide mixtures useful in seed treatment include amisulbrom, azoxystrobin, boscalid, carbendazim, carboxin, cymoxanil, cyproconazole, difenoconazole, dimethomorph, fluazinam, fludioxonil, fluquinconazole, fluopicolide, fluoxastrobin, flutriafol, fluxapyroxad, ipconazole, iprodione, metalaxyl, mefenoxam, metconazole, myclobutanil, paclobutrazole, penflufen, picoxystrobin, prothioconazole, pyraclostrobin, sedaxane, silthiofam, tebuconazole, thiabendazole, thiophanate-methyl, thiram, trifloxystrobin and triticonazole.

Compositions comprising compounds of Formula **1** useful for seed treatment can further comprise bacteria and fungi that have the ability to provide protection from the harmful effects of plant pathogenic fungi or bacteria and/or soil born animals such as nematodes. Bacteria exhibiting nematicidal properties may include but are not limited to *Bacillus firmus, Bacillus cereus, Bacillius subtiliis* and *Pasteuria penetrans.* A suitable *Bacillus firmus* strain is strain CNCM I-1582 (GB-126) which is commercially available as BioNem^{™}. A suitable *Bacillus cereus* strain is strain NCMM I-1592. Both *Bacillus* strains are disclosed in US 6,406,690. Other suitable bacteria exhibiting nematicidal activity are *B. amyloliquefaciens* IN937a and *B. subtilis* strain GB03. Bacteria exhibiting fungicidal properties may include but are not limited to *B. pumilus* strain GB34. Fungal species exhibiting nematicidal properties may include but are not limited to *Myrothecium verrucaria, Paecilomyces lilacinus* and *Purpureocillium lilacinum.*

Seed treatments can also include one or more nematicidal agents of natural origin such as the elicitor protein called harpin which is isolated from certain bacterial plant pathogens such as *Erwinia amylovora.* An example is the Harpin-N-Tek seed treatment technology available as N-Hibit^{™} Gold CST.

Seed treatments can also include one or more species of legume-root nodulating bacteria such as the microsymbiotic nitrogen-fixing bacteria *Bradyrhizobium japonicum.* These inocculants can optionally include one or more lipo-chitooligosaccharides (LCOs), which are nodulation (Nod) factors produced by rhizobia bacteria during the initiation of nodule formation on the roots of legumes. For example, the Optimize^{®} brand seed treatment technology incorporates LCO Promoter Technology^{™} in combination with an inocculant.

Seed treatments can also include one or more isoflavones which can increase the level of root colonization by mycorrhizal fungi. Mycorrhizal fungi improve plant growth by enhancing the root uptake of nutrients such as water, sulfates, nitrates, phosphates and metals. Examples of isoflavones include, but are not limited to, genistein, biochanin A, formononetin, daidzein, glycitein, hesperetin, naringenin and pratensein. Formononetin is available as an active ingredient in mycorrhizal inocculant products such as PHC Colonize^{®} AG.

Seed treatments can also include one or more plant activators that induce systemic acquired resistance in plants following contact by a pathogen. An example of a plant activator which induces such protective mechanisms is acibenzolar-*S*-methyl.

The treated seed typically comprises a compound of the present disclosure in an amount from about 0.1 g to 1 kg per 100 kg of seed (i.e. from about 0.0001 to 1% by weight of the seed before treatment). A flowable suspension formulated for seed treatment typically comprises from about 0.5 to about 70% of the active ingredient, from about 0.5 to about 30% of a film-forming adhesive, from about 0.5 to about 20% of a dispersing agent, from 0 to about 5% of a thickener, from 0 to about 5% of a pigment and/or dye, from 0 to about 2% of an antifoaming agent, from 0 to about 1% of a preservative, and from 0 to about 75% of a volatile liquid diluent.

The compounds of this disclosure can be incorporated into a bait composition that is consumed by an invertebrate pest or used within a device such as a trap, bait station, and the like. Such a bait composition can be in the form of granules which comprise (a) active ingredients, namely a biologically effective amount of a compound of Formula **1,** an *N*-oxide, or salt thereof; (b) one or more food materials; optionally (c) an attractant, and optionally (d) one or more humectants. Of note are granules or bait compositions which comprise between about 0.001-5% active ingredients, about 40-99% food material and/or attractant; and optionally about 0.05-10% humectants, which are effective in controlling soil invertebrate pests at very low application rates, particularly at doses of active ingredient that are lethal by ingestion rather than by direct contact. Some food materials can function both as a food source and an attractant. Food materials include carbohydrates, proteins and lipids. Examples of food materials are vegetable flour, sugar, starches, animal fat, vegetable oil, yeast extracts and milk solids. Examples of attractants are odorants and flavorants, such as fruit or plant extracts, perfume, or other animal or plant component, pheromones or other agents known to attract a target invertebrate pest. Examples of humectants, i.e. moisture retaining agents, are glycols and other polyols, glycerine and sorbitol. Of note is a bait composition (and a method utilizing such a bait composition) used to control at least one invertebrate pest selected from the group consisting of ants, termites and cockroaches. A device for controlling an invertebrate pest can comprise the present bait composition and a housing adapted to receive the bait composition, wherein the housing has at least one opening sized to permit the invertebrate pest to pass through the opening so the invertebrate pest can gain access to the bait composition from a location outside the housing, and wherein the housing is further adapted to be placed in or near a locus of potential or known activity for the invertebrate pest.

One embodiment of the present disclosure relates to a method for controlling invertebrate pests, comprising diluting the pesticidal composition of the present disclosure (a compound of Formula **1** formulated with surfactants, solid diluents and liquid diluents or a formulated mixture of a compound of Formula **1** and at least one other pesticide) with water, and optionally adding an adjuvant to form a diluted composition, and contacting the invertebrate pest or its environment with an effective amount of said diluted composition.

Although a spray composition formed by diluting with water a sufficient concentration of the present pesticidal composition can provide sufficient efficacy for controlling invertebrate pests, separately formulated adjuvant products can also be added to spray tank mixtures. These additional adjuvants are commonly known as "spray adjuvants" or "tank-mix adjuvants", and include any substance mixed in a spray tank to improve the performance of a pesticide or alter the physical properties of the spray mixture. Adjuvants can be surfactants, emulsifying agents, petroleum-based crop oils, crop-derived seed oils, acidifiers, buffers, thickeners or defoaming agents. Adjuvants are used to enhancing efficacy (e.g., biological availability, adhesion, penetration, uniformity of coverage and durability of protection), or minimizing or eliminating spray application problems associated with incompatibility, foaming, drift, evaporation, volatilization and degradation. To obtain optimal performance, adjuvants are selected with regard to the properties of the active ingredient, formulation and target (e.g., crops, insect pests).

Among the spray adjuvants, oils including crop oils, crop oil concentrates, vegetable oil concentrates and methylated seed oil concentrates are most commonly used to improve the efficacy of pesticides, possibly by means of promoting more even and uniform spray deposits. In situations where phytotoxicity potentially caused by oils or other water-immiscible liquids are of concern, spray compositions prepared from the composition of the present disclosure will generally not contain oil-based spray adjuvants. However, in situations where phytotoxicity caused by oil-based spray adjuvants is commercially insignificant, spray compositions prepared from the composition of the present composition can also contain oil-based spray adjuvants, which can potentially further increase control of invertebrate pests, as well as rainfastness.

Products identified as "crop oil" typically contain 95 to 98% paraffin or naphtha-based petroleum oil and 1 to 2% of one or more surfactants functioning as emulsifiers. Products identified as "crop oil concentrates" typically consist of 80 to 85% of emulsifiable petroleum-based oil and 15 to 20% of nonionic surfactants. Products correctly identified as "vegetable oil concentrates" typically consist of 80 to 85% of vegetable oil (i.e. seed or fruit oil, most commonly from cotton, linseed, soybean or sunflower) and 15 to 20% of nonionic surfactants. Adjuvant performance can be improved by replacing the vegetable oil with methyl esters of fatty acids that are typically derived from vegetable oils. Examples of methylated seed oil concentrates include MSO^{®} Concentrate (UAP-Loveland Products, Inc.) and Premium MSO Methylated Spray Oil (Helena Chemical Company).

The amount of adjuvants added to spray mixtures generally does not exceed about 2.5% by volume, and more typically the amount is from about 0.1 to about 1% by volume. The application rates of adjuvants added to spray mixtures are typically between about 1 to 5 L per hectare. Representative examples of spray adjuvants include: Adigor^{®} (Syngenta) 47% methylated rapeseed oil in liquid hydrocarbons, Silwet^{®} (Helena Chemical Company) polyalkyleneoxide modified heptamethyltrisiloxane and Assist^{®} (BASF) 17% surfactant blend in 83% paraffin based mineral oil.

The compounds of this disclosure can be applied without other adjuvants, but most often application will be of a formulation comprising one or more active ingredients with suitable carriers, diluents, and surfactants and possibly in combination with a food depending on the contemplated end use. One method of application involves spraying a water dispersion or refined oil solution of a compound of the present disclosure. Combinations with spray oils, spray oil concentrations, spreader stickers, adjuvants, other solvents, and enhancing agents such as piperonyl butoxide often enhance compound efficacy. For nonagronomic uses such sprays can be applied from spray containers such as a can, a bottle or other container, either by means of a pump or by releasing it from a pressurized container, e.g., a pressurized aerosol spray can. Such spray compositions can take various forms, for example, sprays, mists, foams, fumes or fog. Such spray compositions thus can further comprise propellants, foaming agents, etc. as the case may be. Of note is a spray composition comprising a biologically effective amount of a compound or a composition of the present disclosure and a carrier. One embodiment of such a spray composition comprises a biologically effective amount of a compound or a composition of the present disclosure and a propellant. Representative propellants include, but are not limited to, methane, ethane, propane, butane, isobutane, butene, pentane, isopentane, neopentane, pentene, hydrofluorocarbons, chlorofluorocarbons, dimethyl ether, and mixtures of the foregoing. Of note is a spray composition (and a method utilizing such a spray composition dispensed from a spray container) used to control at least one invertebrate pest selected from the group consisting of mosquitoes, black flies, stable flies, deer flies, horse flies, wasps, yellow jackets, hornets, ticks, spiders, ants, gnats, and the like, including individually or in combinations.

The following Tests demonstrate the control efficacy of compounds of this disclosure on specific pests. "Control efficacy" represents inhibition of invertebrate pest development (including mortality) that causes significantly reduced feeding. The pest control protection afforded by the compounds is not limited, however, to these species. See Index Table A below for compound descriptions. The following abbreviations are used in Index Table A: *t*-Bu means *tert*-butyl *i*-Pr means *iso*-propyl, *c*-Pr means cyclopropyl, *c*-pentyl means cyclopentyl, *c*-hexyl means cyclohexyl and Ph means phenyl. The abbreviation "Cmpd. No." stands for "Compound Number", and the abbreviation "Ex." stands for "Example" and is followed by a number indicating in which example the compound is prepared. The abbreviation "m.p." stands for melting point.

**INDEX TABLE A**

| 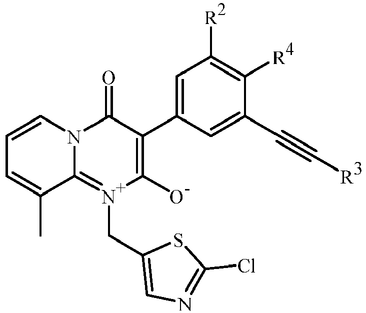 | | | | | |
|---|---|---|---|---|---|
| Cmpd.-No. | R² | R³ | R⁴ | m.p. (°C) | AP⁺ (M+1) |
| 1 | Cl | CH₂CH₃ | H | 96-100 | |
| 2 | H | CH₂CH₃ | H | 151-155 | |
| 3 | H | -C(CH₃)₂OH | H | 96-100 | |
| 4 | OCH₃ | *c*-Pr | H | 92-96 | |
| 5 | *i*-Pr | *t*-Bu | H | 218.9-231.9 | |
| 6 | *i*-Pr | *c*-pentyl | H | 75.7-104 | |
| 7 | CH₃ | *i*-Pr | H | 169.5-175.6 | |
| 8 | CF₃ | *t*-Bu | H | 213-227 | |
| 9 | CH₃ | *c*-pentyl | H | 154.5-164.4 | |
| 11 | CH₃ | *c*-Pr | H | 94.1-121.5 | |
| 12 | *i*-Pr | *c*-hexyl | H | 180.8-200.8 | |
| 13 | *i*-Pr | *i*-Pr | H | 161.3-174.6 | |
| 14 | *i*-Pr | *c*-Pr | H | 89.5-123.9 | |
| 15 | OCH₃ | *c*-pentyl | H | 95.9-110.4 | |
| 16 | OCH₃ | *c*-hexyl | H | 92.3-104 | |
| 17 | OCH₃ | *t*-Bu | H | 134-192 | |
| 18 | OCH₃ | *i*-Pr | H | 128.3-194.6 | |
| 19 | F | *c*-pentyl | H | 140.3-162.6 | |
| 20 | F | *c*-hexyl | H | 183.6-192 | |
| 21 | F | *i*-Pr | H | 150.3-173.3 | |
| 22 | F | CH₂-*c*-hexyl | H | 130-146 | |
| 23 | F | *t*-Bu | H | 165.7-182.5 | |
| 24 | CF₃ | *c*-pentyl | H | 178.3-200.8 | |
| 25 | CF₃ | *c*-hexyl | H | 197.9-209.2 | |
| 26 | CF₃ | *i*-Pr | H | 179.6-184.6 | |
| 27 | CF₃ | CH₂-*c*-hexyl | H | 166.4-186.6 | |
| 28 | CF₃ | *c*-Pr | H | 147.5-152 | |
| 29 | F | *c*-Pr | H | 144-166 | |
| 30 | Cl | *c*-pentyl | H | 144.6-169.5 | |
| 31 | Cl | *c*-hexyl | H | 129.5-157.7 | |
| 32 | Cl | *i*-Pr | H | 155.8-163.3 | |
| 33 | Cl | CH₂-*c*-hexyl | H | 127-145.8 | |
| 34 | Cl | *t-*Bu | H | 174-183.9 | |
| 35 | Cl | *c*-Pr | H | 147.5-152 | |
| 36 | H | *c*-hexyl | H | 142.5-186.6 | |
| 37 | H | *c*-Pr | H | 64.8-87.2 | |
| 38 | H | *c*-pentyl | H | 106.4-114.4 | |
| 39 | H | 4-F-Ph | H | 185-189 | |
| 40 | H | 3-F-Ph | H | 191-195 | |
| 41 | H | CH₂-*c*-pentyl | H | 145-156 | |
| 42 | H | *t*-Bu | H | 160.8-205.2 | |
| 43 | H | CH₂-*c*-hexyl | H | 80-180 | |
| 44 | H | *i*-Pr | H | 79.9-106.4 | |
| 45 | H | 2-F-Ph | H | 178-182 | |
| 46 | H | Ph | H | 172-176 | |
| 47 | Cl | Ph | H | | 518 |
| 48 | H | 4-Cl-Ph | H | | 518 |
| 49 | H | 3-Cl-Ph | H | | 518 |
| 50 | O-*i*-Pr | *c*-Pr | H | 89-151 | |
| 51 | OCH₂CH₃ | *c*-Pr | H | 92-106.8 | |
| 52 | OCH₂CH₃ | *c*-pentyl | H | 75.5-94.5 | |
| 53 | O-*i*-Pr | *c*-pentyl | H | 78-94 | |
| 54 | OCH₂CH₃ | *i*-Pr | H | 90-97 | |
| 55 | O-*i*-Pr | *i*-Pr | H | 78-100 | |
| 56 | H | *c*-Pr | Cl | | 483 |
| 57 | H | *i*-Pr | OCH₃ | 154-158 | |
| 58 | O-*c*-Pr | *c*-Pr | H | | 505 |
| 59 | H | CH₂-CH₃ | H | 147-151 | 436 |
| 60 | Cl | CH₂-CH₃ | H | 107-110 | 470 |
| 61 | H | CH₃ | H | 188-192 | 422 |
| 62 | Cl | CH₃ | H | 222-225 | 456 |
| 63 | H | *i*-Pr | OCH₃ | 154-188 | 481 |
| 64 | O-*c*-Pr | *c*-Pr | H | | 506 |
| 65 | H | *c*-Pr | OH | | 478 |
| 66 | O-*c*-Pr | *i*-Pr | H | 85-101 | 506 |
| 67 | O-*c*-Pr | *c*-pentyl | H | | 532 |
| 68 | H | *c*-Pr | OCH₃ | | 478 |
| 69 | O-CH₂-CH2-OMe | *c*-Pr | H | 150-154 | 522 |
| 70 | O-CH₂-CH₂-OMe | *i*-Pr | H | 65-79 | 526 |
| 71 | O-CH₂-CH₂-OMe | *c*-pentyl | H | 67-84 | 550 |
| 72 | O-CH₂-C(Me)₂ | *c*-Pr | H | 80-99 | 522 |
| 73 | O-CH₂-C(Me)₂ | *i*-Pr | H | 114-143 | 522 |
| 74 | O-CH₂-C(Me)₂ | *c*-pentyl | H | 80-96 | 548 |
| 75 | O-CH₂-C(Me)₂ | CH₂-CH₃ | H | 79-101 | 508 |
| 77 | Me | CH₂-CH₃ | H | 88-109 | 450 |
| 78 | H | CH₂-CH₃ | OCH₃ | 75-191 | 466 |
| 79 | O-CH₂-CH₃ | CH₂-CH₃ | H | 70-88 | 480 |
| 80 | O-CH₂-CH₃ | *t*-Bu | H | 92-109 | 508 |
| 81 | O-CH₂-CH₃ | *c*-hexyl | H | 82-105 | 534 |
| 82 | O-C(Me)₂ | CH₂-CH₃ | H | 74-85 | 494 |
| 83 | O-*c*-Pr | *t*-Bu | H | 93-114 | 520 |
| 84 | O-*c*-Pr | CH₂-CH₃ | H | 83-134 | 492 |
| 85 | O-CH₂-CH₂-OMe | CH₂-CH₃ | H | 52-70 | 512 |
| 86 | H | *i*-Pr | CH₃ | 169-175 | 464 |
| 87 | H | *t*-Bu | CH₃ | 125-189 | 478 |

### BIOLOGICAL EXAMPLES

The following Tests demonstrate the control efficacy of compounds of this disclosure on specific pests. "Control efficacy" represents inhibition of invertebrate pest development (including mortality) that causes significantly reduced feeding. The pest control protection afforded by the compounds is not limited, however, to these species. See Index Table A for compound descriptions.

### Formulation and Spray Methodology for Tests A-H

Test compounds were formulated using a solution containing 10% acetone, 90% water and 300 ppm Activator 90^{®} non-ionic surfactant (Loveland Products, Loveland, Colorado, USA). The formulated compounds were applied in 1 mL of liquid through an atomizer nozzle positioned 1.27 cm (0.5 inches) above the top of each test unit. Test compounds were sprayed at the rates indicated, and each test was replicated three times.

### Test A

For evaluating control of diamondback moth (*Plutella xylostella* (L.)) the test unit consisted of a small open container with a 12-14-day-old mustard plant inside. This was pre-infested with ~50 neonate larvae that were dispensed into the test unit via corn cob grits using an inoculator. The larvae moved onto the test plant after being dispensed into the test unit.

Test compounds were formulated and sprayed at 10 and/or 2 and/or 0.4 ppm. After spraying of the formulated test compound, each test unit was allowed to dry for 1 hour and then a black, screened cap was placed on top. The test units were held for 6 days in a growth chamber at 25 °C and 70% relative humidity. Plant feeding damage was then visually assessed based on foliage consumed, and larvae were assessed for mortality.

Of the compounds of Formula **1** tested at 10 ppm, the following provided very good to excellent levels of control efficacy (40% or less feeding damage and/or 100% mortality): 1, 2, 3, 4, 7, 8, 9, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 56, 57, 61, 62, 63, 64, 65, 66, 67, 68, 69, 70, 71, 72, 73, 74, 75, 77, 78, 79, 80, 81, 82, 83, 84, 85, 86, and 87.

Of the compounds of Formula **1** tested at 2 ppm, the following provided very good to excellent levels of control efficacy (40% or less feeding damage and/or 100% mortality): 1, 2, 3, 4, 7, 8, 9, 11, 12, 13, 14, 15, 16, 17, 18, 19, 20, 21, 22, 23, 24, 25, 26, 27, 28, 29, 30, 31, 32, 34, 35, 36, 37, 38, 39, 40, 41, 42, 43, 44, 45, 46, 47, 48, 49, 50, 51, 56, 57, 61, 62, 63, 64, 65, 66, 67, 68, 69, 70, 71, 72, 73, 74, 75, 77, 78, 79, 80, 81, 82, 83, 84, 85, 86, and 87.

Of the compounds of Formula **1** tested at 0.4 ppm, the following provided very good to excellent levels of control efficacy (40% or less feeding damage and/or 100% mortality): 1, 2, 4, 7, 9, 11, 12, 13, 14, 15, 16, 17, 18, 19, 20, 21, 23, 24, 25, 26, 28, 29, 30, 32, 34, 35, 37, 38, 39, 40, 42, 44, 45, 46, 48, 49, 50, 51, 56, 57, 61, 63, 64, 66, 67, 68, 69, 70, 71, 72, 73, 74, 75, 77, 78, 79, 80, 81, 82, 83, 84, 85, and 86.

### Test B

For evaluating control of fall armyworm *(Spodoptera frugiperda* (J.E. Smith)) the test unit consisted of a small open container with a 4-5-day-old corn (maize) plant inside. This was pre-infested with 10-15 1-day-old larvae on a piece of insect diet.

Test compounds were formulated and sprayed at 10 and/or 2 and/or 0.4 ppm. After spraying of the formulated test compound, the test units were maintained in a growth chamber for 6 days at 25 °C and 70% relative humidity. Plant feeding damage was then visually assessed based on foliage consumed, and larvae were assessed for mortality.

Of the compounds of Formula **1** tested at 10 ppm, the following provided very good to excellent levels of control efficacy (40% or less feeding damage and/or 100% mortality): 1, 2, 3, 4, 5, 6, 7, 8, 9, 11, 12, 13, 14, 15, 16, 17, 18, 19, 20, 21, 22, 23, 24, 26, 27, 28, 29, 30, 31, 32, 33, 34, 35, 36, 37, 38, 39, 40, 41, 42, 43, 44, 45, 46, 47, 48, 49, 50, 51, 56, 57, 61, 63, 64, 65, 66, 67, 68, 69, 70, 71, 72, 73, 74, 75, 77, 78, 79, 80, 81, 82, 83, 84, 85, 86, and 87.

Of the compounds of Formula **1** tested at 2 ppm, the following provided very good to excellent levels of control efficacy (40% or less feeding damage and/or 100% mortality): 1, 2, 4, 5, 7, 8, 9, 11, 13, 14, 16, 17, 18, 19, 20, 21, 22, 23, 24, 26, 28, 29, 30, 31, 32, 34, 35, 36, 37, 38, 39, 40, 42, 44, 45, 46, 47, 48, 49, 50, 51, 56, 57, 61, 63, 64, 66, 67, 68, 69, 70, 71, 72, 73, 74, 75, 77, 78, 79, 80, 81, 82, 83, 84, 85, 86, and 87.

Of the compounds of Formula **1** tested at 0.4 ppm, the following provided very good to excellent levels of control efficacy (40% or less feeding damage and/or 100% mortality1, 2, 4, 7, 9, 11, 13, 14, 16, 18, 19, 21, 23, 24, 29, 30, 32, 34, 35, 37, 38, 39, 40, 42, 44, 45, 46, 48, 50, 51, 61, 64, 66, 69, 70, 71, 72, 75, 79, 80, 81, 82, 84, 85, and 86.

### Test C

For evaluating control of corn planthopper (*Peregrinus maidis* (Ashmead)) through contact and/or systemic means, the test unit consisted of a small open container with a 3-4-day-old corn (maize) plant inside. White sand was added to the top of the soil prior to application of the test compound.

Test compounds were formulated and sprayed at 50 ppm. After spraying of the formulated test compound, the test units were allowed to dry for 1 h before they were post-infested with ~15-20 nymphs (18-to-21-day-old). A black, screened cap was placed on the top of each test unit, and the test units were held for 6 days in a growth chamber at 22-24 °C and 50-70% relative humidity. Each test unit was then visually assessed for insect mortality.

Of the compounds of Formula **1** tested at 50 ppm, the following resulted in at least 80% mortality: 1, 2, 3, 4, 7, 11, 16, 36, 37, 38, 43, 44, 48, 50, 51, 61, 64, 66, 72, 77, 79, 80, and 86.

### Test D

For evaluating control of potato leafhopper (*Empoasca fabae* (Harris)) through contact and/or systemic means, the test unit consisted of a small open container with a 5-6-day-old Soleil bean plant (primary leaves emerged) inside. White sand was added to the top of the soil, and one of the primary leaves was excised prior to application of the test compound.

Test compounds were formulated and sprayed at 50 ppm. After spraying of the formulated test compound, the test units were allowed to dry for 1 hour before they were post-infested with 5 potato leafhoppers (18-to-21-day-old adults). A black, screened cap was placed on the top of the test unit, and the test units were held for 6 days in a growth chamber at 20 °C and 70% relative humidity. Each test unit was then visually assessed for insect mortality.

Of the compounds of Formula **1** tested at 50 ppm, the following resulted in at least 80% mortality: 3, 4, 7, 11, 21, 26, 36, 37, 39, 43, 44, 47, 48, 61, 77, 85, and 86.

### Test E

For evaluating control of green peach aphid *(Myzus persicae* (Sulzer)) through contact and/or systemic means, the test unit consisted of a small open container with a 12-15-day-old radish plant inside. This was pre-infested by placing on a leaf of the test plant 30-40 aphids on a piece of leaf excised from a culture plant (cut-leaf method). The aphids moved onto the test plant as the leaf piece desiccated. After pre-infestation, the soil of the test unit was covered with a layer of sand.

Test compounds were formulated and sprayed at 50 ppm. After spraying of the formulated test compound, each test unit was allowed to dry for 1 hour and then a black, screened cap was placed on top. The test units were held for 6 days in a growth chamber at 19-21 °C and 50-70% relative humidity. Each test unit was then visually assessed for insect mortality.

Of the compounds of Formula 1 tested at 50 ppm, the following resulted in at least 80% mortality: 1, 2, 3, 4, 7, 11, 14, 15, 16, 18, 21, 29, 35, 36, 37, 43, 44, 48, 50, 51, 64, 66, 70, 72, 73, 75, 77, 79, 80, 81, 82, 83, 84, 85, and 86.

### Test F

For evaluating control of cotton melon aphid (*Aphis gossypii* (Glover)) through contact and/or systemic means, the test unit consisted of a small open container with a 5-day-old okra plant inside. This was pre-infested with 30-40 insects on a piece of leaf according to the cut-leaf method, and the soil of the test unit was covered with a layer of sand.

Test compounds were formulated and sprayed at 50 ppm. After spraying, the test units were maintained in a growth chamber for 6 days at 19 °C and 70% relative humidity. Each test unit was then visually assessed for insect mortality.

Of the compounds of Formula **1** tested at 50 ppm, the following resulted in at least 80% mortality: 1, 2, 3, 4, 7, 11, 14, 15, 16, 18, 21, 35, 36, 37, 38, 43, 44, 47, 48, 50, 51, 61, 64, 66, 70, 72, 73, 74, 75, 77, 78, 79, 80, 82, 83, 84, 85, and 86.

### Test G

For evaluating control of the sweetpotato whitefly (*Bemisia tabaci* (Gennadius)) through contact and/or systemic means, the test unit consisted of a small open container with a 12-14-day-old cotton plant inside. Prior to the spray application, both cotyledons were removed from the plant, leaving one true leaf for the assay. Adult whiteflies were allowed to lay eggs on the plant and then were removed from the test unit. Cotton plants infested with at least 15 eggs were submitted to the test for spraying.

Test compounds were formulated and sprayed at 250 and/or 50 ppm. After spraying, the test units were allowed to dry for 1 hour. The cylinders were then removed, and the units were taken to a growth chamber and held for 13 days at 28 °C and 50-70% relative humidity. Each test unit was then visually assessed for insect mortality.

Of the compounds of Formula **1** tested at 250 ppm, the following resulted in at least 70% mortality: NONE.

Of the compounds of Formula **1** tested at 50 ppm, the following resulted in at least 70% mortality: NONE.

### Test H

For evaluating control of the Western Flower Thrips (*Frankliniellla occidentalis* (Pergande)) through contact and/or systemic means, the test unit consisted of a small open container with a 5-7-day-old Soleil bean plant inside.

Test compounds were formulated and sprayed at 250 and/or 50 ppm. After spraying, the test units were allowed to dry for 1 hour, and then about 60 thrips (adults and nymphs) were added to each unit. A black, screened cap was placed on top, and the test units were held for 6 days at 25 °C and 45-55% relative humidity. Each test unit was then visually assessed for plant damage and insect mortality.

Of the compounds of Formula **1** tested at 250 ppm, the following provided very good to excellent levels of control efficacy (30% or less plant damage and/or 100% mortality): 4, 11, and 42.

Of the compounds of Formula **1** tested at 50 ppm, the following provided very good to excellent levels of control efficacy (30% or less plant damage and/or 100% mortality): 4, 11, and 42.

## Claims

1. A compound selected from Formula **1,** an *N*-oxide or salt thereof, 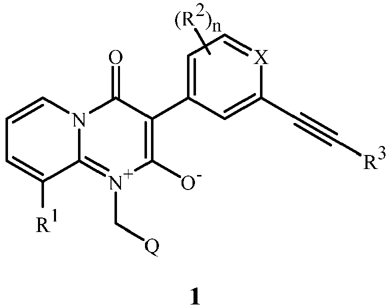 wherein
R¹ is H, halogen, C₁-C₄ alkyl, C₁-C₄ haloalkyl, C₁-C₄ alkoxy or C₁-C₄ haloalkoxy;
Xis CR⁴ or N;
Q is CH₂CN, 6-chloro-3-pyridinyl or 2-chloro-5-thiazolyl;
each R² is independently halogen, cyano, nitro, C₁-C₄ alkyl, C₁-C₄ haloalkyl, C₃-C₆ cycloalkyl, C₁-C₆ alkoxy, C₁-C₆ haloalkoxy, C₃-C₆ cycloalkoxy, C₁-C₆ alkoxyalkoxy, C₁-C₆ alkylthio, C₁-C₆ alkylsulfinyl, C₁-C₆ alkylsulfonyl, C₁-C₆ haloalkylthio, C₁-C₆ haloalkylsulfinyl, or C₁-C₆ haloalkylsulfonyl;
n is 0, 1, 2 or 3;
R⁴ is H, halogen, cyano, nitro, C₁-C₄ alkyl, C₁-C₄ haloalkyl, C₃-C₆ cycloalkyl, C₁-C₆ alkoxy, C₁-C₆ haloalkoxy, C₁-C₆ alkylthio, C₁-C₆ alkylsulfinyl, C₁-C₆ alkylsulfonyl, C₁-C₆ haloalkylthio, C₁-C₆ haloalkylsulfinyl, or C₁-C₆ haloalkylsulfonyl; and
R³ is C₁-C₆ alkyl, C₁-C₆ haloalkyl, C₁-C₆ alkoxyalkyl, C₁-C₆ hydroxyalkyl, C₃-C₆ cycloalkyl, C₃-C₆ halocycloalkyl, C₄-C₇ alkylcycloalkyl or phenyl optionally substituted with halogen, cyano, nitro, C₁-C₄ alkyl, C₁-C₄ haloalkyl, C₃-C₆ cycloalkyl, C₁-C₆ alkoxy, C₁-C₆ haloalkoxy, C₁-C₆ alkylthio, C₁-C₆ alkylsulfinyl, C₁-C₆ alkylsulfonyl, C₁-C₆ haloalkylthio, C₁-C₆ haloalkylsulfinyl, or C₁-C₆ haloalkylsulfonyl;
provided that when Q is CH₂CN, then R³ is other than CH₃.

2. The compound of Claim 1 wherein
R¹ is H, halogen, C₁-C₄ alkyl or C₁-C₄ alkoxy;
Q is 6-chloro-3-pyridinyl or 2-chloro-5-thiazolyl;
each R² is independently halogen, C₁-C₄ alkyl, C₁-C₄ haloalkyl or C₁-C₆ alkoxy;
n is 0, 1 or 2;
R⁴ is H, halogen, C₁-C₄ alkyl or C₁-C₆ alkoxy; and
R³ is C₁-C₆ alkyl, C₁-C₆ haloalkyl, C₃-C₆ cycloalkyl, C₃-C₆ halocycloalkyl or phenyl optionally substituted with halogen, cyano, C₁-C₄ alkyl, C₁-C₄ haloalkyl or C₁-C₆ alkoxy.

3. The compound of Claim 2 wherein
R¹ is H, halogen, C₁-C₂ alkyl or C₁-C₂ alkoxy;
each R² is independently halogen, C₁-C₂ alkyl, C₁-C₂ haloalkyl or C₁-C₃ alkoxy;
R⁴ is H, halogen, C₁-C₂ alkyl or C₁-C₂ alkoxy; and
R³ is C₁-C₆ alkyl, C₁-C₆ haloalkyl, C₃-C₆ cycloalkyl or C₃-C₆ halocycloalkyl.

4. The compound of Claim 3 wherein
X is CR⁴;
Q is 2-chloro-5-thiazolyl; and
R⁴ is H, Cl, F, CH₃ or OCH₃.

5. The compound of Claim 4 wherein
R¹ is H, Cl, CH₃ or OCH₃;
R² is Cl, F, CH₃ or OCH₃;
n is 0 or 1; and
R³ is C₁-C₆ alkyl or C₃-C₆ cycloalkyl.

6. The compound of Claim 5 wherein
R¹ is CH₃;
R² is CH₃ or OCH₃; and
R³ is ethyl, isopropyl or cyclopropyl.

7. The compound of Claim 1 wherein the compound is selected from:
1-[(2-chloro-5-thiazolyl)methyl]-3-[3-(2-cyclopropylethynyl)phenyl]-2-hydroxy-9-methyl-4-oxo-4*H*-pyrido[1,2-a]pyrimidinium inner salt;
1-[(2-chloro-5-thiazolyl)methyl]-2-hydroxy-9-methyl-3-[3-(3-methyl-1-butyn-1-yl)phenyl]-4-oxo-4*H*-pyrido[1,2-a]pyrimidinium inner salt;
1-[(2-chloro-5-thiazolyl)methyl]-3-[3-(2-cyclopropylethynyl)-5-methoxyphenyl]-2-hydroxy-9-methyl-4-oxo-4*H*-pyrido[1,2-a]pyrimidinium inner salt;
1-[(2-chloro-5-thiazolyl)methyl]-3-[3-(2-cyclopropylethynyl)-5-methylphenyl]-2-hydroxy-9-methyl-4-oxo-4*H*-pyrido[1,2-a]pyrimidinium inner salt;
1-[(2-chloro-5-thiazolyl)methyl]-2-hydroxy-9-methyl-3-[3-methyl-5-(3-methyl-1-butyn-1-yl)phenyl]-4-oxo-4*H-*pyrido[1,2-a]pyrimidinium inner salt;
3-[3-(1-butyn-1-yl)-5-chlorophenyl]-1-[(2-chloro-5-thiazolyl)methyl]-2-hydroxy-9-methyl-4-oxo-4*H*-pyrido[1,2-a]pyrimidinium inner salt;
1-[(2-chloro-5-thiazolyl)methyl]-3-[3-(2-cyclopropylethynyl)-5-ethoxyphenyl]-2-hydroxy-9-methyl-4-oxo-4*H*-pyrido[1,2-a]pyrimidinium inner salt;
3-[3-(1-butyn-1-yl)phenyl]-1-[(2-chloro-5-thiazolyl)methyl]-2-hydroxy-9-methyl-4-oxo-4*H*-pyrido[1,2-a]pyrimidinium inner salt; and
1-[(2-chloro-5-thiazolyl)methyl]-3-[3-ethoxy-5-(3-methyl-1-butyn-1-yl)phenyl]-2-hydroxy-9-methyl-4-oxo-4*H*-pyrido[1,2-a]pyrimidinium inner salt.

8. A composition comprising the compound of any one of Claims 1-7 and at least one additional component selected from the group consisting of surfactants, solid diluents and liquid diluents, said composition optionally further comprising at least one additional biologically active compound or agent.

9. The composition of Claim 8, further comprising the at least one additional biologically active compound or agent and wherein the at least one additional biologically active compound or agent is selected from the group consisting of abamectin, acephate, acequinocyl, acetamiprid, acrinathrin, afidopyropen, amidoflumet, amitraz, avermectin, azadirachtin, azinphos-methyl, benfuracarb, bensultap, bifenthrin, bifenazate, bistrifluron, borate, bromantraniliprole, buprofezin, carbaryl, carbofuran, cartap, carzol, chlorantraniliprole, chlorfenapyr, chlorfluazuron, chlorpyrifos, chlorpyrifos-methyl, chromafenozide, clofentezin, clothianidin, cyantraniliprole, cyclaniliprole, cycloprothrin, cycloxaprid, cyflumetofen, cyfluthrin, beta-cyfluthrin, cyhalodiamide, cyhalothrin, gamma-cyhalothrin, lambda-cyhalothrin, cypermethrin, alpha-cypermethrin, zeta-cypermethrin, cyromazine, deltamethrin, diafenthiuron, diazinon, dichlorantraniliprole, dieldrin, diflubenzuron, dimefluthrin, dimehypo, dimethoate, dinotefuran, diofenolan, emamectin, endosulfan, esfenvalerate, ethiprole, etofenprox, etoxazole, fenbutatin oxide, fenitrothion, fenothiocarb, fenoxycarb, fenpropathrin, fenvalerate, fipronil, flometoquin, flonicamid, flubendiamide, flucythrinate, flufenerim, flufenoxuron, flufenoxystrobin, flufensulfone, fluorpyram, flupiprole, flupyradifurone, fluvalinate, tau-fluvalinate, fonophos, formetanate, fosthiazate, halofenozide, heptafluthrin, hexaflumuron, hexythiazox, hydramethylnon, imidacloprid, indoxacarb, insecticidal soaps, isofenphos, lufenuron, malathion, meperfluthrin, metaflumizone, metaldehyde, methamidophos, methidathion, methiodicarb, methomyl, methoprene, methoxychlor, metofluthrin, monocrotophos, monofluthrin, methoxyfenozide, *N-*[1,1-dimethyl-2-(methylthio)ethyl]-7-fluoro-2-(3-pyridinyl)-2*H-*indazole-4-carboxamide, *N-*[1,1-dimethyl-2-(methylsulfinyl)ethyl]-7-fluoro-2-(3-pyridinyl)-2*H*-indazole-4-carboxamide, *N*-[1,1-dimethyl-2-(methylsulfonyl)ethyl]-7-fluoro-2-(3-pyridinyl)-2*H*-indazole-4-carboxamide, *N*-(1-methylcyclopropyl)-2-(3-pyridinyl)-2H-indazole-4-carboxamide, and *N*-[1-(difluoromethyl)cyclopropyl]-2-(3-pyridinyl)-2*H-*indazole-4-carboxamide, nitenpyram, nithiazine, novaluron, noviflumuron, oxamyl, parathion, parathion-methyl, permethrin, phorate, phosalone, phosmet, phosphamidon, pirimicarb, profenofos, profluthrin, propargite, protrifenbute, pyflubumide, pymetrozine, pyrafluprole, pyrethrin, pyridaben, pyridalyl, pyrifluquinazon, pyriminostrobin, pyriprole, pyriproxyfen, rotenone, ryanodine, silafluofen, spinetoram, spinosad, spirodiclofen, spiromesifen, spirotetramat, sulprofos, sulfoxaflor, tebufenozide, tebufenpyrad, teflubenzuron, tefluthrin, terbufos, tetrachlorantraniliprole, tetrachlorvinphos, tetramethrin, tetramethylfluthrin, thiacloprid, thiamethoxam, thiodicarb, thiosultap-sodium, tolfenpyrad, tralomethrin, triazamate, trichlorfon, triflumuron, all strains of *Bacillus thuringiensis,* entomopathogenic bacteria, all strains of *Nucleo polyhedrosis* viruses, entomopathogenic viruses and entomopathogenic fungi.

10. The composition of Claim 9 wherein the at least one additional biologically active compound or agent is selected from the group consisting of abamectin, acetamiprid, acrinathrin, afidopyropen, amitraz, avermectin, azadirachtin, benfuracarb, bensultap, bifenthrin, 3 -bromo-1-(3-chloro-2-pyridinyl)-*N*-[4-cyano-2-methyl-6-[(methylamino)carbonyl]phenyl]-1*H*-pyrazole-5-carboxamide, buprofezin, carbaryl, cartap, chlorantraniliprole, chlorfenapyr, chlorpyrifos, clothianidin, cyantraniliprole, cyclaniliprole, cycloprothrin, cyfluthrin, beta-cyfluthrin, cyhalothrin, lambda-cyhalothrin, gamma-cyhalothrin, cypermethrin, alpha-cypermethrin, zeta-cypermethrin, cyromazine, deltamethrin, dieldrin, dinotefuran, diofenolan, emamectin, endosulfan, esfenvalerate, ethiprole, etofenprox, etoxazole, fenitrothion, fenothiocarb, fenoxycarb, fenvalerate, fipronil, flometoquin, flonicamid, flubendiamide, flufenoxuron, flufenoxystrobin, flufensulfone, flupiprole, flupyradifurone, fluvalinate, formetanate, fosthiazate, heptafluthrin, hexaflumuron, hydramethylnon, imidacloprid, indoxacarb, lufenuron, meperfluthim, metaflumizone, methiodicarb, methomyl, methoprene, methoxyfenozide, metofluthrin, monofluthrin, nitenpyram, nithiazine, novaluron, oxamyl, pyflubumide, pymetrozine, pyrethrin, pyridaben, pyridalyl, pyriminostrobin, pyriproxyfen, ryanodine, spinetoram, spinosad, spirodiclofen, spiromesifen, spirotetramat, sulfoxaflor, tebufenozide, tetramethrin, thiacloprid, thiamethoxam, thiodicarb, thiosultap-sodium, tralomethrin, tetramethylfluthrin, triazamate, triflumuron, all strains of *Bacillus thuringiensis* and all strains of *Nucleo polyhedrosis* viruses.

11. The composition of any one of claims 8-10 further comprising a liquid fertilizer, such as wherein said liquid fertilizer is aqueous-based.

12. A method for controlling an invertebrate pest comprising contacting the invertebrate pest or its environment with a biologically effective amount of a compound or composition of any one of Claims 1-11, with the proviso that the method is not a method of treatment of the human or animal body by therapy.

13. The method of claim 12 wherein the compound, composition or formulation is dispensed by a drip irrigation system, furrow during planting, handheld sprayer, backpack sprayer, boom sprayer, ground sprayer, aerial application, or an unmanned aerial vehicle.

14. The method of claim 12 comprising spraying said composition or formulation at an ultra-low volume.

15. A treated seed comprising the compound, composition or formulation of any one of claims 1-11 in an amount of from about 0.0001 to 1 % by weight of the seed before treatment.

## Patentansprüche

1. Verbindung, ausgewählt aus Formel **1,** *N*-Oxid oder Salz davon, 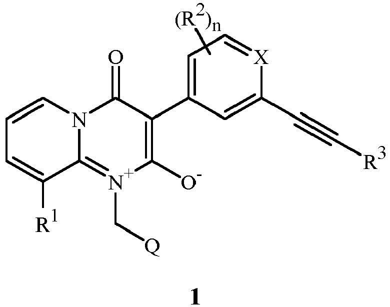 wobei
R¹ für H, Halogen, C₁-C₄-Alkyl, C₁-C₄-Halogenalkyl, C₁-C₄-Alkoxy oder C₁-C₄-Halogenalkoxy steht;
X für CR⁴ oder N steht;
Q für CH₂CN, 6-Chlor-3-pyridinyl oder 2-Chlor-5-thiazolyl steht;
R² jeweils unabhängig für Halogen, Cyano, Nitro, C₁-C₄-Alkyl, C₁-C₄-Halogenalkyl, C₃-C₆-Cycloalkyl, C₁-C₆-Alkoxy, C₁-C₆-Halogenalkoxy, C₃-C₆-Cycloalkoxy, C₁-C₆-Alkoxyalkoxy, C₁-C₆-Alkylthio, C₁-C₆-Alkylsulfinyl, C₁-C₆-Alkylsulfonyl, C₁-C₆-Halogenalkylthio, C₁-C₆-Halogenalkylsulfinyl oder C₁-C₆-Halogenalkylsulfonyl steht;
n für 0, 1, 2 oder 3 steht;
R⁴ für H, Halogen, Cyano, Nitro, C₁-C₄-Alkyl, C₁-C₄-Halogenalkyl, C₃-C₆-Cycloalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, C₁-C₆-Halogenalkylsulfinyl oder C₁-C₆-Halogenalkylsulfonyl steht; und
R³ für C₁-C₆-Alkyl, C₁-C₆-Halogenalkyl, C₁-C₆-Alkoxyalkyl, C₁-C₆-Hydroxyalkyl, C₃-C₆-Cycloalkyl, C₃-C₆-Halogencycloalkyl, C₄-C₇-Alkylcycloalkyl oder Phenyl, das gegebenenfalls durch Halogen, Cyano, Nitro, C₁-C₄-Alkyl, C₁-C₄-Halogenalkyl, C₃-C₆-Cycloalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, C₁-C₆-Halogenalkylsulfinyl oder C₁-C₆-Halogenalkylsulfonyl substituiert ist, steht;
mit der Maßgabe, dass dann, wenn Q für CH₂CN steht, R³ von CH₃ verschieden ist.

2. Verbindung nach Anspruch 1, wobei
R¹ für H, Halogen, C₁-C₄-Alkyl oder C₁-C₄-Alkoxy steht;
Q für 6-Chlor-3-pyridinyl oder 2-Chlor-5-thiazolyl steht;
R² jeweils unabhängig für Halogen, C₁-C₄-Alkyl, C₁-C₄-Halogenalkyl oder C₁-C₆-Alkoxy steht;
n für 0, 1 oder 2 steht;
R⁴ für H, Halogen, C₁-C₄-Alkyl oder C₁-C₆-Alkoxy steht; und
R³ für C₁-C₆-Alkyl, C₁-C₆-Halogenalkyl, C₃-C₆-Cycloalkyl, C₃-C₆-Halogencycloalkyl oder Phenyl, das gegebenenfalls durch Halogen, Cyano, C₁-C₄-Alkyl, C₁-C₄-Halogenalkyl oder C₁-C₆-Alkoxy substituiert ist, steht.

3. Verbindung nach Anspruch 2, wobei
R¹ für H, Halogen, C₁-C₂-Alkyl oder C₁-C₂-Alkoxy steht;
R² jeweils unabhängig für Halogen, C₁-C₂-Alkyl, C₁-C₂-Halogenalkyl oder C₁-C₃-Alkoxy steht;
R⁴ für H, Halogen, C₁-C₂-Alkyl oder C₁-C₂-Alkoxy steht; und
R³ für C₁-C₆-Alkyl, C₁-C₆-Halogenalkyl, C₃-C₆-Cycloalkyl oder C₃-C₆-Halogencycloalkyl steht.

4. Verbindung nach Anspruch 3, wobei
X für CR⁴ steht;
Q für 2-Chlor-5-thiazolyl steht; und
R⁴ für H, Cl, F, CH₃ oder OCH₃ steht.

5. Verbindung nach Anspruch 4, wobei
R¹ für H, Cl, CH₃ oder OCH₃ steht;
R² für Cl, F, CH₃ oder OCH₃ steht;
n für 0 oder 1 steht; und
R³ für C₁-C₆-Alkyl oder C₃-C₆-Cycloalkyl steht.

6. Verbindung nach Anspruch 5, wobei
R¹ für CH₃ steht;
R² für CH₃ oder OCH₃ steht; und
R³ für Ethyl, Isopropyl oder Cyclopropyl steht.

7. Verbindung nach Anspruch 1, wobei die Verbindung ausgewählt ist aus:
innerem 1-[(2-Chlor-5-thiazolyl)methyl]-3-[3-(2-cyclopropylethinyl)phenyl]-2-hydroxy-9-methyl-4-oxo-4H-pyrido[1,2-a]pyrimidinium-Salz;
innerem 1-[(2-Chlor-5-thiazolyl)methyl]-2-hydroxy-9-methyl-3-[3-(3-methyl-1-butin-1-yl)phenyl]-4-oxo-4H-pyrido[1,2-a]pyrimidinium-Salz;
innerem 1-[(2-Chlor-5-thiazolyl)methyl]-3-[3-(2-cyclopropylethinyl)-5-methoxyphenyl]-2-hydroxy-9-methyl-4-oxo-4H-pyrido[1,2-a]pyrimidinium-Salz;
innerem 1-[(2-Chlor-5-thiazolyl)methyl]-3-[3-(2-cyclopropylethinyl)-5-methylphenyl]-2-hydroxy-9-methyl-4-oxo-4H-pyrido[1,2-a]pyrimidinium-Salz;
innerem 1-[(2-Chlor-5-thiazolyl)methyl]-2-hydroxy-9-methyl-3-[3-methyl-5-(3-methyl-1-butin-1-yl)phenyl]-4-oxo-4H-pyrido[1,2-a]pyrimidinium-Salz;
innerem 3-[3-(1-Butin-1-yl)-5-chlorophenyl]-1-[(2-chlor-5-thiazolyl)methyl]-2-hydroxy-9-methyl-4-oxo-4H-pyrido[1,2-a]pyrimidinium-Salz;
innerem 1-[(2-Chlor-5-thiazolyl)methyl]-3-[3-(2-cyclopropylethinyl)-5-ethoxyphenyl]-2-hydroxy-9-methyl-4-oxo-4H-pyrido[1,2-a]pyrimidinium-Salz;
innerem 3-[3-(1-Butin-1-yl)phenyl]-1-[(2-chlor-5-thiazolyl)methyl]-2-hydroxy-9-methyl-4-oxo-4H-pyrido[1,2-a]pyrimidinium-Salz und
innerem 1-[(2-Chlor-5-thiazolyl)methyl]-3-[3-ethoxy-5-(3-methyl-1-butin-1-yl)phenyl]-2-hydroxy-9-methyl-4-oxo-4H-pyrido[1,2-a]pyrimidinium-Salz.

8. Zusammensetzung, umfassend die Verbindung nach einem der Ansprüche 1-7 und mindestens eine zusätzliche Komponente aus der Gruppe bestehend aus Tensiden, festen Verdünnungsmitteln und flüssigen Verdünnungsmitteln, wobei die Zusammensetzung gegebenenfalls ferner mindestens eine zusätzliche biologisch aktive Verbindung oder mindestens ein zusätzliches biologisch aktives Mittel umfasst.

9. Zusammensetzung nach Anspruch 8, die ferner die mindestens eine zusätzliche biologisch aktive Verbindung oder das mindestens eine zusätzliche biologisch aktive Mittel umfasst, wobei die mindestens eine zusätzliche biologisch aktive Verbindung oder das mindestens eine zusätzliche biologisch aktive Mittel aus der Gruppe bestehend aus Abamectin, Acephat, Acequinocyl, Acetamiprid, Acrinathrin, Afidopyropen, Amidoflumet, Amitraz, Avermectin, Azadirachtin, Azinphos-methyl, Benfuracarb, Bensultap, Bifenthrin, Bifenazat, Bistrifluron, Borat, Bromantraniliprol, Buprofezin, Carbaryl, Carbofuran, Cartap, Carzol, Chlorantraniliprol, Chlorfenapyr, Chlorfluazuron, Chlorpyrifos, Chlorpyrifos-methyl, Chromafenozid, Clofentezin, Clothianidin, Cyantraniliprol, Cyclaniliprol, Cycloprothrin, Cycloxaprid, Cyflumetofen, Cyfluthrin, beta-Cyfluthrin, Cyhalodiamid, Cyhalothrin, gamma-Cyhalothrin, lambda-Cyhalothrin, Cypermethrin, alpha-Cypermethrin, zeta-Cypermethrin, Cyromazin, Deltamethrin, Diafenthiuron, Diazinon, Dichlorantraniliprol, Dieldrin, Diflubenzuron, Dimefluthrin, Dimehypo, Dimethoat, Dinotefuran, Diofenolan, Emamectin, Endosulfan, Esfenvalerat, Ethiprol, Etofenprox, Etoxazol, Fenbutatinoxid, Fenitrothion, Fenothiocarb, Fenoxycarb, Fenpropathrin, Fenvalerat, Fipronil, Flometoquin, Flonicamid, Flubendiamid, Flucythrinat, Flufenerim, Flufenoxuron, Flufenoxystrobin, Flufensulfon, Fluorpyram, Flupiprol, Flupyradifuron, Fluvalinat, tau-Fluvalinat, Fonophos, Formetanat, Fosthiazat, Halofenozid, Heptafluthrin, Hexaflumuron, Hexythiazox, Hydramethylnon, Imidacloprid, Indoxacarb, insektiziden Seifen, Isofenphos, Lufenuron, Malathion, Meperfluthrin, Metaflumizon, Metaldehyd, Methamidophos, Methidathion, Methiodicarb, Methomyl, Methopren, Methoxychlor, Metofluthrin, Monocrotophos, Monofluthrin, Methoxyfenozid, *N*-[1,1-Dimethyl-2-(methylthio)ethyl]-7-fluor-2-(3-pyridinyl)-2*H*-indazol-4-carboxamid, *N*-[1,1-Dimethyl-2-(methylsulfinyl)ethyl]-7-fluor-2-(3-pyridinyl)-2*H*-indazol-4-carboxamid, *N-*[1,1-Dimethyl-2-(methylsulfonyl)ethyl]-7-fluor-2-(3-pyridinyl)-2*H*-indazol-4-carboxamid, N-(1-Methylcyclopropyl)-2-(3-pyridinyl)-2H-indazol-4-carboxamid und *N*-[1-(Difluormethyl)cyclopropyl]-2-(3-pyridinyl)-2*H*-indazol-4-carboxamid, Nitenpyram, Nithiazin, Novaluron, Noviflumuron, Oxamyl, Parathion, Parathion-methyl, Permethrin, Phorat, Phosalon, Phosmet, Phosphamidon, Pirimicarb, Profenofos, Profluthrin, Propargit, Protrifenbut, Pyflubumid, Pymetrozin, Pyrafluprol, Pyrethrin, Pyridaben, Pyridalyl, Pyrifluquinazon, Pyriminostrobin, Pyriprol, Pyriproxyfen, Rotenon, Ryanodin, Silafluofen, Spinetoram, Spinosad, Spirodiclofen, Spiromesifen, Spirotetramat, Sulprofos, Sulfoxaflor, Tebufenozid, Tebufenpyrad, Teflubenzuron, Tefluthrin, Terbufos, Tetrachlorantraniliprol, Tetrachlorvinphos, Tetramethrin, Tetramethylfluthrin, Thiacloprid, Thiamethoxam, Thiodicarb, Thiosultap-Natrium, Tolfenpyrad, Tralomethrin, Triazamat, Trichlorfon, Triflumuron, allen Stämmen von *Bacillus thuringiensis,* entomopathogenen Bakterien, allen Stämmen von *Nucleo-polyhedrosis-Viren,* entomopathogenen Viren und entomopathogenen Pilzen ausgewählt ist.

10. Zusammensetzung nach Anspruch 9, wobei die mindestens eine zusätzliche biologisch aktive Verbindung oder das mindestens eine zusätzliche biologisch aktive Mittel aus der Gruppe bestehend aus Abamectin, Acetamiprid, Acrinathrin, Afidopyropen, Amitraz, Avermectin, Azadirachtin, Benfuracarb, Bensultap, Bifenthrin, 3-Brom-1-(3-chlor-2-pyridinyl)-*N*-[4-cyano-2-methyl-6-[(methylamino)carbonyl]phenyl]-1*H*-pyrazol-5-carboxamid, Buprofezin, Carbaryl, Cartap, Chlorantraniliprol, Chlorfenapyr, Chlorpyrifos, Clothianidin, Cyantraniliprol, Cyclaniliprol, Cycloprothrin, Cyfluthrin, beta-Cyfluthrin, Cyhalothrin, lambda-Cyhalothrin, gamma-Cyhalothrin, Cypermethrin, alpha-Cypermethrin, zeta-Cypermethrin, Cyromazin, Deltamethrin, Dieldrin, Dinotefuran, Diofenolan, Emamectin, Endosulfan, Esfenvalerat, Ethiprol, Etofenprox, Etoxazol, Fenitrothion, Fenothiocarb, Fenoxycarb, Fenvalerat, Fipronil, Flometoquin, Flonicamid, Flubendiamid, Flufenoxuron, Flufenoxystrobin, Flufensulfon, Flupiprol, Flupyradifuron, Fluvalinat, Formetanat, Fosthiazat, Heptafluthrin, Hexaflumuron, Hydramethylnon, Imidacloprid, Indoxacarb, Lufenuron, Meperfluthrin, Metaflumizon, Methiodicarb, Methomyl, Methopren, Methoxyfenozid, Metofluthrin, Monofluthrin, Nitenpyram, Nithiazin, Novaluron, Oxamyl, Pyflubumid, Pymetrozin, Pyrethrin, Pyridaben, Pyridalyl, Pyriminostrobin, Pyriproxyfen, Ryanodin, Spinetoram, Spinosad, Spirodiclofen, Spiromesifen, Spirotetramat, Sulfoxaflor, Tebufenozid, Tetramethrin, Thiacloprid, Thiamethoxam, Thiodicarb, Thiosultap-Natrium, Tralomethrin, Tetramethylfluthrin, Triazamat, Triflumuron, allen Stämmen von *Bacillus thuringiensis* und allen Stämmen von *Nucleo-polyhedrosis-Viren* ausgewählt ist.

11. Zusammensetzung nach einem der Ansprüche 8-10, ferner umfassend ein flüssiges Düngemittel, wie wobei das flüssige Düngemittel wasserbasiert ist.

12. Verfahren zur Bekämpfung eines wirbellosen Schädlings, umfassend das Inkontaktbringen des wirbellosen Schädlings oder seiner Umgebung mit einer biologisch wirksamen Menge einer Verbindung oder Zusammensetzung nach einem der Ansprüche 1-11, mit der Maßgabe, dass es sich bei dem Verfahren nicht um ein Verfahren zur therapeutischen Behandlung des menschlichen oder tierischen Körpers handelt.

13. Verfahren nach Anspruch 12, wobei die Verbindung, Zusammensetzung oder Formulierung in einem Berieselungssystem, in der Furche während des Pflanzens, einer Handspritze, einer Rückenspritze, einem Spritzbalken, einer Bodenspritze, einer Flugzeugapplikation oder einem unbemannten Luftfahrzeug ausgebracht wird.

14. Verfahren nach Anspruch 12, umfassend das Spritzen der Zusammensetzung oder Formulierung im Ultra-Low-Volume-Verfahren.

15. Behandelter Samen, umfassend die Verbindung, Zusammensetzung oder Formulierung nach einem der Ansprüche 1-11 in einer Menge von etwa 0,0001 bis 1 Gew.-% des Samens vor der Behandlung.

## Revendications

1. Composé choisi parmi la formule 1, un *N*-oxyde ou sel correspondant, 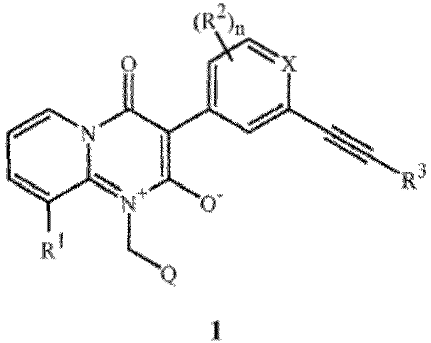
R¹ étant H, halogène, C₁-C₄ alkyle, C₁-C₄ halogénoalkyle, C₁-C₄ alcoxy ou C₁-C₄ halogénoalcoxy ;
X étant CR⁴ ou N ;
Q étant CH₂CN, 6-chloro-3-pyridinyle ou 2-chloro-5-thiazolyle ;
chaque R² étant indépendamment halogène, cyano, nitro, C₁-C₄ alkyle, C₁-C₄ halogénoalkyle, C₃-C₆ cycloalkyle, C₁-C₆ alcoxy, C₁-C₆ halogénoalcoxy, C₃-C₆ cycloalcoxy, C₁-C₆ alcoxyalcoxy, C₁-C₆ alkylthio, C₁-C₆ alkylsulfinyle, C₁-C₆ alkylsulfonyle, C₁-C₆ halogénoalkylthio, C₁-C₆ halogénoalkylsulfinyle, ou C₁-C₆ halogénoalkylsulfonyle ;
n étant 0, 1, 2 ou 3 ;
R⁴ étant H, halogène, cyano, nitro, C₁-C₄ alkyle, C₁-C₄ halogénoalkyle, C₃-C₆ cycloalkyle, C₁-C₆ alcoxy, C₁-C₆ halogénoalcoxy, C₁-C₆ alkylthio, C₁-C₆ alkylsulfinyle, C₁-C₆ alkylsulfonyle, C₁-C₆ halogénoalkylthio, C₁-C₆ halogénoalkylsulfinyle, ou C₁-C₆ halogénoalkylsulfonyle ; et
R³ étant C₁-C₆ alkyle, C₁-C₆ halogénoalkyle, C₁-C₆ alcoxyalkyle, C₁-C₆ hydroxyalkyle, C₃-C₆ cycloalkyle, C₃-C₆ halogénocycloalkyle, C₄-C₇ alkylcycloalkyle ou phényle éventuellement substitué par halogène, cyano, nitro, C₁-C₄ alkyle, C₁-C₄ halogénoalkyle, C₃-C₆ cycloalkyle, C₁-C₆ alcoxy, C₁-C₆ halogénoalcoxy, C₁-C₆ alkylthio, C₁-C₆ alkylsulfinyle, C₁-C₆ alkylsulfonyle, C₁-C₆ halogénoalkylthio, C₁-C₆ halogénoalkylsulfinyle, ou C₁-C₆ halogénoalkylsulfonyle ;
à la condition que lorsque Q est CH₂CN, alors R³ est différent de CH₃.

2. Composé selon la revendication 1,
R¹ étant H, halogène, C₁-C₄ alkyle ou C₁-C₄ alcoxy ;
Q étant 6-chloro-3-pyridinyle ou 2-chloro-5-thiazolyle ;
chaque R² étant indépendamment halogène, C₁-C₄ alkyle, C₁-C₄ halogénoalkyle ou C₁-C₆ alcoxy ;
n étant 0, 1 ou 2 ;
R⁴ étant H, halogène, C₁-C₄ alkyle ou C₁-C₆ alcoxy ; et
R³ étant C₁-C₆ alkyle, C₁-C₆ halogénoalkyle, C₃-C₆ cycloalkyle, C₃-C₆ halogénocycloalkyle ou phényle éventuellement substitué par halogène, cyano, C₁-C₄ alkyle, C₁-C₄ halogénoalkyle ou C₁-C₆ alcoxy.

3. Composé selon la revendication 2,
R¹ étant H, halogène, C₁-C₂ alkyle ou C₁-C₂ alcoxy ;
chaque R² étant indépendamment halogène, C₁-C₂ alkyle, C₁-C₂ halogénoalkyle ou C₁-C₃ alcoxy ;
R⁴ étant H, halogène, C₁-C₂ alkyle ou C₁-C₂ alcoxy ; et
R³ étant C₁-C₆ alkyle, C₁-C₆ halogénoalkyle, C₃-C₆ cycloalkyle ou C₃-C₆ halogénocycloalkyle.

4. Composé selon la revendication 3,
X étant CR⁴ ;
Q étant 2-chloro-5-thiazolyle ; et
R⁴ étant H, Cl, F, CH₃ ou OCH₃.

5. Composé selon la revendication 4,
R¹ étant H, Cl, CH₃ ou OCH₃ ;
R² étant Cl, F, CH₃ ou OCH₃ ;
n étant 0 ou 1 ; et
R³ étant C₁-C₆ alkyle ou C₃-C₆ cycloalkyle.

6. Composé selon la revendication 5,
R¹ étant CH₃ ;
R² étant CH₃ ou OCH₃ ; et
R³ étant éthyle, isopropyle ou cyclopropyle.

7. Composé selon la revendication 1, le composé étant choisi parmi :
1-[(2-chloro-5-thiazolyl)méthyl]-3-[3-(2-cyclopropyléthynyl)phényl]-2-hydroxy-9-méthyl-4-oxo-4H-pyrido[1,2-a]pyrimidinium sel interne ;
1-[(2-chloro-5-thiazolyl)méthyl]-2-hydroxy-9-méthyl-3-[3-(3-méthyl-1-butyn-1-yl)phényl]-4-oxo-4*H*-pyrido[1,2-a]pyrimidinium sel interne ;
1-[(2-chloro-5-thiazolyl)méthyl]-3-[3-(2-cyclopropyléthynyl)-5-méthoxyphényl]-2-hydroxy-9-méthyl-4-oxo-4*H*-pyrido[1,2-a]pyrimidinium sel interne ;
1-[(2-chloro-5-thiazolyl)méthyl]-3-[3-(2-cyclopropyléthynyl)-5-méthylphényl]-2-hydroxy-9-méthyl-4-oxo-4*H*-pyrido[1,2-a]pyrimidinium sel interne ;
1-[(2-chloro-5-thiazolyl)méthyl]-2-hydroxy-9-méthyl-3-[3-méthyl-5-(3-méthyl-1-butyn-1-yl)phényl]-4-oxo-4*H*-pyrido[1,2-a]pyrimidinium sel interne ;
3-[3-(1-butyn-1-yl)-5-chlorophényl]-1-[(2-chloro-5-thiazolyl)méthyl]-2-hydroxy-9-méthyl-4-oxo-4*H*-pyrido[1,2-a]pyrimidinium sel interne ;
1-[(2-chloro-5-thiazolyl)méthyl]-3-[3-(2-cyclopropyléthynyl)-5-éthoxyphényl]-2-hydroxy-9-méthyl-4-oxo-4*H*-pyrido[1,2-a]pyrimidinium sel interne ;
3-[3-(1-butyn-1-yl)phényl]-1-[(2-chloro-5-thiazolyl)méthyl]-2-hydroxy-9-méthyl-4-oxo-4*H*-pyrido[1,2-a]pyrimidinium sel interne ; et
1-[(2-chloro-5-thiazolyl)méthyl]-3-[3-éthoxy-5-(3-méthyl-1-butyn-1-yl)phényl]-2-hydroxy-9-méthyl-4-oxo-4*H*-pyrido[1,2-a]pyrimidinium sel interne.

8. Composition comprenant le composé selon l'une quelconque des revendications 1 à 7 et au moins un composant supplémentaire choisi dans le groupe constitué par des tensioactifs, des diluants solides et des diluants liquides, ladite composition comprenant en outre éventuellement au moins un composé ou agent biologiquement actif supplémentaire.

9. Composition selon la revendication 8, comprenant en outre l'au moins un composé ou agent biologiquement actif supplémentaire et l'au moins un composé ou agent biologiquement actif supplémentaire étant choisi dans le groupe constitué par abamectine, acéphate, acéquinocyle, acétamipride, acrinathrine, afidopyropène, amidoflumet, amitraze, avermectine, azadirachtine, azinphos-méthyle, benfuracarbe, bensultap, bifenthrine, bifénazate, bistrifluron, borate, bormantraniliprole, buprofézine, carbaryle, carbofuran, cartap, carzol, chlorantraniliprole, chlorfénapyr, chlorfluazuron, chlorpyrifos, chlorpyrifos-méthyle, chromafénozide, clofentézine, clothianidine, cyantraniliprole, cyclaniliprole, cycloprothrine, cycloxaprid, cyflumetofène, cyfluthrine, bêta-cyfluthrine, cyhalodiamide, cyhalothrine, gamma-cyhalothrine, lambda-cyhalothrine, cyperméthrine, alpha-cyperméthrine, zêta-cyperméthrine, cyromazine, deltaméthrine, diafenthiuron, diazinon, dichlorantraniliprole, dieldrine, diflubenzuron, diméfluthrine, diméhypo, diméthoate, dinotéfurane, diofénolan, émamectine, endosulfan, esfenvalérate, éthiprole, étofenprox, étoxazole, fenbutatin oxyde, fénitrothion, fénothiocarbe, fénoxycarbe, fenpropathrine, fenvalérate, fipronil, flométoquine, flonicamide, flubendiamide, flucythrinate, flufénérim, flufénoxuron, flufénoxystrobine, flufensulfone, fluorpyrame, flupiprole, flupyradifurone, fluvalinate, tau-fluvalinate, fonophos, formétanate, fosthiazate, halofénozide, heptafluthrine, hexaflumuron, hexythiazox, hydraméthylnon, imidaclopride, indoxacarbe, des savons insecticides, isofenphos, lufénuron, malathion, méperfluthrine, métaflumizone, métaldéhyde, méthamidophos, méthidathion, méthiodicarbe, méthomyle, méthoprène, méthoxychlore, métofluthrine, monocrotophos, monofluthrine, méthoxyfenozide, *N*-[1,1-diméthyl-2-(méthylthio)éthyl]-7-fluoro-2-(3-pyridinyl)-2H-indazole-4-carboxamide, *N*-[1,1-diméthyl-2-(méthylsulfinyl)éthyl]-7-fluoro-2-(3-pyridinyl)-2H-indazole-4-carboxamide, *N*-[1,1-diméthyl-2-(méthylsulfonyl)éthyl]-7-fluoro-2-(3-pyridinyl)-2*H-*indazole-4-carboxamide, N-(1-méthylcyclopropyl)-2-(3-pyridinyl)-2H-indazole-4-carboxamide, et *N*-[1-(difluorométhyl)cyclopropyl]-2-(3-pyridinyl)-2*H-*indazole-4-carboxamide, nitenpyrame, nithiazine, novaluron, noviflumuron, oxamyle, parathion, parathion-méthyle, perméthrine, phorate, phosalone, phosmet, phosphamidon, pirimicarbe, profenofos, profluthrine, propargite, protrifenbute, pyflubumide, pymétrozine, pyrafluprole, pyréthrine, pyridabène, pyridalyle, pyrifluquinazon, pyriminostrobine, pyriprole, pyriproxyfène, roténone, ryanodine, silafluofène, spinétorame, spinosad, spirodiclofène, spiromesifène, spirotétramate, sulprofos, sulfoxaflor, tébufénozide, tébufenpyrad, téflubenzuron, téfluthrine, terbufos, tétrachlorantraniliprole, tétrachlorvinphos, tétraméthrine, tétraméthylfluthrine, thiaclopride, thiaméthoxame, thiodicarbe, thiosultap-sodium, tolfenpyrade, tralométhrine, triazamate, trichlorfon, triflumuron, toutes les souches de *Bacillus thuringiensis,* des bactéries entomopathogènes, toutes les souches de virus de *Nucleo polyhedrosis,* des virus entomopathogènes et des champignons entomopathogènes.

10. Composition selon la revendication 9, l'au moins un composé ou agent biologiquement actif supplémentaire étant choisi dans le groupe constitué par abamectine, acétamipride, acrinathrine, afidopyropène, amitraze, avermectine, azadirachtine, benfuracarbe, bensultap, bifenthrine, 3-bromo-1-(3-chloro-2-pyridinyl)-N-[4-cyano-2-méthyl-6-[(méthylamino)carbonyl]phényl]-1H-pyrazole-5-carboxamide, buprofézine, carbaryle, cartap, chlorantraniliprole, chlorfénapyr, chlorpyrifos, clothianidine, cyantraniliprole, cyclaniliprole, cycloprothrine, cyfluthrine, bêta-cyfluthrine, cyhalothrine, lambda-cyhalothrine, gamma-cyhalothrine, cyperméthrine, alpha-cyperméthrine, zêta-cyperméthrine, cyromazine, deltaméthrine, dieldrine, dinotéfurane, diofénolan, émamectine, endosulfan, esfenvalérate, éthiprole, étofenprox, étoxazole, fénitrothion, fénothiocarbe, fénoxycarbe, fenvalérate, fipronil, flométoquine, flonicamide, flubendiamide, flufénoxuron, flufenoxystrobine, flufensulfone, flupiprole, flupyradifurone, fluvalinate, formétanate, fosthiazate, heptafluthrine, hexaflumuron, hydraméthylnon, imidaclopride, indoxacarbe, lufenuron, méperfluthrine, métaflumizone, méthiodicarbe, méthomyle, méthoprène, méthoxyfenozide, métofluthrine, monofluthrine, nitenpyrame, nithiazine, novaluron, oxamyle, pyflubumide, pymétrozine, pyréthrine, pyridabène, pyridalyle, pyriminostrobine, pyriproxyfène, ryanodine, spinétorame, spinosad, spirodiclofène, spiromésifène, spirotétramate, sulfoxaflor, tébufénozide, tétraméthrine, thiaclopride, thiaméthoxame, thiodicarbe, thiosultap-sodium, tralométhrine, tétraméthylfluthrine, triazamate, triflumuron, toutes les souches de *Bacillus thuringiensis* et toutes les souches de virus de *Nucleo polyhedrosis.*

11. Composition selon l'une quelconque des revendications 8 à 10 comprenant en outre un engrais liquide, telle que dans laquelle ledit engrais liquide est à base aqueuse.

12. Procédé pour la lutte contre un organisme nuisible invertébré comprenant la mise en contact de l'organisme nuisible invertébré ou de son environnement avec une quantité biologiquement efficace d'un composé ou d'une composition selon l'une quelconque des revendications 1 à 11, à la condition que le procédé ne soit pas un procédé de traitement du corps humain ou animal par thérapie.

13. Procédé selon la revendication 12, le composé, la composition ou la formulation étant distribué(e) par un système d'irrigation goutte-à-goutte, un sillon pendant la plantation, un pulvérisateur portable à la main, un pulvérisateur dorsal, un pulvérisateur à rampe, un pulvérisateur au sol, une application aérienne ou un véhicule aérien sans pilote.

14. Procédé selon la revendication 12 comprenant une pulvérisation de ladite composition ou formulation à un volume ultra-faible.

15. Graine traitée comprenant le composé, la composition ou la formulation selon l'une quelconque des revendications 1 à 11 en une quantité allant d'environ 0,0001 à 1 % en poids de la graine avant traitement.
